# EUROPEAN PATENT APPLICATION

(11) **EP 4 646 990 A1**
(43) Date of publication of application: **12.11.2025**
(21) Application number: 23914598.0
(22) Date of filing: 29.12.2023
(51) Int. Cl.: A47L 11/40

(54) **SENSOR MOUNTING STRUCTURE AND CLEANING DEVICE**

(30) Priority: 03.01.2023 CN 202310004893; 03.01.2023 CN 202310004803; 03.01.2023 CN 202310004753; 03.01.2023 CN 202310004890; 03.01.2023 CN 202310004898; 03.01.2023 CN 202310004891
(71) Applicant: Beijing Roborock Technology Co., Ltd., Beijing 102206 (CN)
(72) Inventor: YU, Luping, Beijing 102206 (CN); ZHAO, Degang, Beijing 102206 (CN); WANG, Xiaoliang, Beijing 102206 (CN)
(74) Representative: Cabinet Beau de Loménie
(86) International application number: PCT/CN2023/143125
(87) International publication number: WO 2024/146466

(57) **Abstract**

A sensor mounting structure and a cleaning device. The cleaning device comprises a main body (1) and a liquid storage tank (2), the liquid storage tank (2) being used for storing a cleaning liquid, the liquid storage tank (2) comprising a bottom case (9011), and the bottom case (9011) and the main body (1) being integrally formed; and a wet cleaning structure (927), the liquid storage tank (2) being used for providing the cleaning liquid for the wet cleaning structure (927).

## Description

### CROSS-REFERENCE TO RELATED APPLICATIONS

This application is based on and claims the priority of Chinese patent application No. 202310004898.9, filed on January 3, 2023, Chinese patent application No. 202310004890.2, filed on January 3, 2023, Chinese patent application No. 202310004753.9, filed on January 3, 2023, Chinese patent application No. 202310004803.3, filed on January 3, 2023, Chinese patent application No. 202310004891.7, filed on January 3, 2023, and Chinese patent application No. 202310004893.6, filed on January 3, 2023, the entire contents of which are hereby incorporated by reference.

### TECHNICAL FIELD

The present disclosure relates to household appliances, and in particular to a sensor mounting structure and a cleaning device.

### BACKGROUND ART

With the development of information technology, innovations in robots are changing with each passing day, and many robots have gradually become prevalent in our daily lives. Among them, sweeping robots, as a type of smart home appliance, can automatically complete floor sweeping and mopping in the room through artificial intelligence algorithms. However, as user's requirements increase, higher requirements are placed on robots during their automatic mopping process.

### SUMMARY OF THE INVENTION

The present invention provides a sensor mounting structure and a cleaning device.

According to one aspect of the present disclosure, a sensor mounting structure is provided, including:
a main body; and
a fixing cover, an accommodation cavity being provided between the fixing cover and the main body and configured to accommodate a sensor;
wherein the fixing cover has a first end and a second end opposite to each other, one of the first end and the main body is provided with a fixing portion, and the other of the first end and the main body is provided with a fixing matching portion matching with the fixing portion, and the second end of the fixing cover is provided with a mounting portion, and the mounting portion is connected to the main body via a fastener.

In some embodiments of the present disclosure, the fixing portion is an extension arm, the fixing matching portion is an insertion hole, and the extension arm is at least partially inserted into the insertion hole.

In some embodiments of the present disclosure, the extension arm is snap-connected to the insertion hole.

In some embodiments of the present disclosure, a limiting portion is provided on an outer side of the insertion hole and configured to limit the extension arm such that the extension arm passes through the insertion hole.

In some embodiments of the present disclosure, the limiting portion is of a U-shaped structure, and an open end of the U-shaped structure is disposed toward the extension arm.

In some embodiments of the present disclosure, the limiting portion is disposed on a bearing portion, and the bearing portion is configured to bear at least a portion of the extension arm.

In some embodiments of the present disclosure, upper and lower sides of the extension arm respectively abut against an inner wall of the insertion hole and the bearing portion; or
the upper side of the extension arm abuts against the inner wall of the insertion hole.

In some embodiments of the present disclosure, one of the main body and the fixing cover is provided with a limiting protrusion, and the other of the main body and the fixing cover is provided with a limiting groove, and the limiting groove is configured to limit the limiting protrusion.

In some embodiments of the present disclosure, the fastener is a bolt.

In some embodiments of the present disclosure, one of the mounting portion and the main body is provided with a positioning column, and the other of the mounting portion and the main body is provided with a positioning hole, and the positioning column passes through the positioning hole.

According to another aspect of the present disclosure, an embodiment of the present disclosure provides a cleaning device, including a sensor and the above-mentioned sensor mounting structure, the sensor mounting structure being configured to mount the sensor.

In some embodiments of the present disclosure, the sensor is a carpet sensor.

In some embodiments of the present disclosure, the cleaning device further includes:
a water storage tank configured to store cleaning liquid;
an overflow structure disposed on the water storage tank;
an enclosing structure at least partially disposed around the overflow structure and configured to retain and guide the cleaning liquid flowing out of the overflow structure; and
a wet cleaning structure, the water storage tank being configured to provide the cleaning liquid for the wet cleaning structure.

In some embodiments of the present disclosure, the cleaning device includes a liquid storage tank for the cleaning device, the liquid storage tank including:
a tank body configured to store cleaning liquid;
a liquid discharging structure disposed in the tank body and including a liquid discharging bevel structure and a liquid discharging pipeline, a first end of the liquid discharging pipeline being connected to a bottom end of the liquid discharging bevel structure, and a second end of the liquid discharging pipeline being provided with a liquid discharging port;
wherein the cleaning liquid in the tank body is configured to be guided to the first end of the liquid discharging pipeline through the liquid discharging bevel structure, and discharged to outside of the tank body through the liquid discharging port.

In some embodiments of the present disclosure, the cleaning device includes a cleaning device water tank, the water tank including:
a tank body;
a liquid inlet pipe disposed on the tank body, a liquid inlet being disposed at a first end of the liquid inlet pipe;
a water inlet valve disposed inside a second end of the liquid inlet pipe, the liquid inlet pipe being capable of communicating with an inner cavity of the tank body through the water inlet valve; and
a supporting structure disposed in the liquid inlet pipe and configured to support one side of the water inlet valve.

In some embodiments of the present disclosure, the cleaning device includes a liquid level detection structure for the cleaning device water tank, the liquid level detection structure including:
a limiting structure disposed in the water tank;
a floating assembly disposed in the limiting structure and configured to move as a liquid level in the water tank changes, the limiting structure being configured to limit the floating assembly; and
a sensing member disposed on the water tank and configured to generate a sensing signal in response to the movement of the floating assembly;
wherein a reinforcement structure is disposed on a side of the limiting structure away from the floating assembly.

In some embodiments of the present disclosure, the cleaning device further includes:
a main body;
a liquid storage tank configured to store cleaning liquid and including a bottom shell, the bottom shell and the main body being of an integrally formed structure; and
a wet cleaning structure configured to store liquid for the wet cleaning structure.

### BRIEF DESCRIPTION OF THE DRAWINGS

In order to better understand the present disclosure, reference may be made to the embodiments shown in the following drawings. The components in the drawings are not necessarily to scale, and related elements may be omitted in order to emphasize and clearly illustrate the technical features of the present disclosure. In addition, related elements or components may have different settings as known in the art. In addition, in the drawings, the same reference numeral represents the same or similar components in each drawing. In the drawings:
FIG. 1 is a schematic structural diagram of a cleaning device in the related art;
FIG. 2 is a schematic structural diagram of a cleaning device according to an embodiment of the present disclosure;
FIG. 3 is a schematic structural diagram of a cleaning device with an upper cover opened according to an embodiment of the present disclosure;
FIG. 4 is a schematic structural diagram of a cleaning device with an upper cover opened according to an embodiment of the present disclosure;
FIG. 5 is a schematic structural diagram of an upper cover of a cleaning device according to an embodiment of the present disclosure;
FIG. 6 is a schematic structural diagram showing an ultrasonic protrusion on an upper cover of a cleaning device according to an embodiment of the present disclosure;
FIG. 7 is a schematic structural diagram of a bottom shell of a cleaning device according to an embodiment of the present disclosure;
FIG. 8 is a schematic structural diagram showing an ultrasonic surface on a bottom shell of a cleaning device according to an embodiment of the present disclosure;
FIG. 9 is a schematic structural diagram showing an ultrasonic surface on a protective structure of a cleaning device according to an embodiment of the present disclosure;
FIG. 10 is a schematic structural diagram showing an ultrasonic surface on a protective structure of a cleaning device according to an embodiment of the present disclosure;

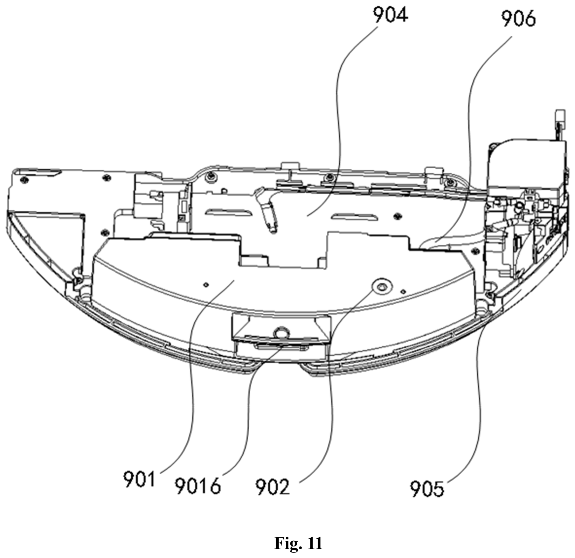

FIG. 11 is a schematic structural diagram of a cleaning device according to an embodiment of the present disclosure;
FIG. 12 is a schematic structural diagram of a cleaning device according to an embodiment of the present disclosure;
FIG. 13 is a schematic exploded structural diagram of a cleaning device according to an embodiment of the present disclosure;
FIG. 14 is a schematic structural diagram showing an enclosing structure and water trough of a cleaning device according to an embodiment of the present disclosure;
FIG. 15 is a schematic structural diagram showing an enclosing structure and water trough of a cleaning device according to an embodiment of the present disclosure;
FIG. 16 is a partial cross-sectional view of a cleaning device according to an embodiment of the present disclosure;
FIG. 17 is a schematic structural diagram of a cleaning device according to an embodiment of the present disclosure;
FIG. 18 is a schematic structural diagram of a cleaning device according to an embodiment of the present disclosure;
FIG. 19 is a schematic structural diagram of a cleaning device before a cover plate is installed according to an embodiment of the present disclosure;
FIG. 20 is a schematic structural diagram of a cleaning device after a cover plate is installed according to an embodiment of the present disclosure;
FIG. 21 is a schematic diagram of a liquid storage tank for a cleaning device according to an embodiment of the present disclosure;
FIG. 22 is a schematic structural diagram of a liquid storage tank for a cleaning device with an upper cover opened according to an embodiment of the present disclosure;
FIG. 23 is a schematic structural diagram of a liquid storage tank for a cleaning device with an upper cover closed according to an embodiment of the present disclosure;
FIG. 24 is a schematic structural diagram of a liquid storage tank for a cleaning device with an upper cover closed according to an embodiment of the present disclosure;
FIG. 25 is an exploded schematic diagram of a liquid storage tank for a cleaning device according to an embodiment of the present disclosure;
FIG. 26 is a schematic structural diagram of a liquid storage tank for a cleaning device in an upright state according to an embodiment of the present disclosure;
FIG. 27 is a schematic structural diagram of a liquid storage tank for a cleaning device in an inverted state according to an embodiment of the present disclosure;
FIG. 28 is a cross-sectional view of a cleaning device water tank from one perspective according to an embodiment of the present disclosure;
FIG. 29 is a partial cross-sectional view of a sweeping robot in the related art;
FIG. 30 is a schematic diagram of the coordination of a water inlet valve, a supporting structure and a liquid inlet pipe in a cleaning device water tank according to an embodiment of the present disclosure;
FIG. 31 is a schematic structural diagram of a water inlet valve of a cleaning device water tank according to an embodiment of the present disclosure;
FIG. 32 is a schematic structural diagram showing a supporting structure and a liquid inlet pipe of a cleaning device water tank according to an embodiment of the present disclosure;
FIG. 33 is a cross-sectional view of a cleaning device water tank from another perspective according to an embodiment of the present disclosure;
FIG. 34 is a schematic structural diagram showing a liquid inlet pipe of a cleaning device water tank according to an embodiment of the present disclosure;
FIG. 35 is a schematic structural diagram showing a liquid inlet pipe of a cleaning device water tank according to an embodiment of the present disclosure;
FIG. 36 is an exploded schematic diagram of a tank body of a cleaning device water tank according to an embodiment of the present disclosure;
FIG. 37 is an exploded schematic diagram of a tank body of a cleaning device water tank according to an embodiment of the present disclosure;
FIG. 38 is a schematic structural diagram showing a water outlet hole of a cleaning device water tank according to an embodiment of the present disclosure;
FIG. 39 is a schematic structural diagram of a liquid level detection structure of a cleaning device water tank according to an embodiment of the present disclosure;
FIG. 40 is a schematic structural diagram of a liquid level detection structure of a cleaning device water tank according to an embodiment of the present disclosure, with a sensing member hidden;
FIG. 41 is a structural schematic diagram of a liquid level detection structure of a cleaning device water tank according to an embodiment of the present disclosure, with a floating assembly hidden;
FIG. 42 is a partial enlarged schematic diagram of FIG. 41;
FIG. 43 is a schematic structural diagram of a floating assembly in a liquid level detection structure of a cleaning device water tank according to an embodiment of the present disclosure;
FIG. 44 is a schematic structural diagram of a sensor mounting structure according to an embodiment of the present disclosure;
FIG. 45 is a cross-sectional view of a sensor mounting structure according to an embodiment of the present disclosure;
FIG. 46 is a schematic structural diagram of the existing sensor mounting;
FIG. 47 is a schematic structural diagram of a fixing cover in a sensor mounting structure according to an embodiment of the present disclosure; and
FIG. 48 is a schematic structural diagram of a main body in a sensor mounting structure according to an embodiment of the present disclosure.

The following are the descriptions of the reference numerals:
1', main body; 2', liquid storage tank;
1. main body; 2. liquid storage tank; 3. protective structure; 4. wet cleaning structure; 5. liquid inlet pipe; 6. liquid outlet pipe assembly; 7. overflow structure;
21, bottom shell; 211, ultrasonic surface;
22, upper cover; 221, ultrasonic protrusion;
31, first rib plate; 32, second rib plate;
61, liquid outlet pipe; 62, liquid outlet connector.
901, water storage tank; 902, overflow structure; 903, enclosing structure; 904, wet cleaning structure; 905, main body; 906, water supply pipe; 907, guide pipe; 8, cover plate;
9011, bottom shell; 9012, upper cover; 9013, guide slope; 9014, limiting structure; 9015, water trough; 151, bevel; 152, reinforcing rib; 9016, water inlet;
9031, arc portion; 9032, linear portion;
9051, communicating hole;
81, mounting hole; 82, fixing hole;
911, tank body; 912, liquid discharging structure; 913, wet cleaning structure; 914, delivery pipeline;
9111, liquid discharging joint; 9112, bottom shell; 91121, water flow channel; 91122, liquid outlet pipe; 123, liquid outlet joint; 9113, upper cover; 131, liquid inlet pipe; 132, liquid inlet joint; 133, delivery joint;
9121, liquid discharging bevel structure; 91211, liquid discharging trough; 9122, liquid discharging pipeline;
92100, water inlet nozzle; 200, water inlet valve;
921, tank body; 922, liquid inlet pipe; 923, water inlet valve; 924, supporting structure; 925, fixing member; 926, liquid inlet pipe; 927, wet cleaning structure;
9211, bottom shell; 92111, water outlet hole; 9212, upper cover; 92121, joint;
9221, liquid inlet; 9222, first flow channel; 9223, second flow channel;
9231, central portion; 311, gap; 312, single bodies; 9232, fixing portion; 33, annular portion;
9251, third flow channel;
93100, water tank; 101, side wall; 102, bottom wall;
931, limiting structure; 932, floating assembly; 933, reinforcement structure; 934, sensing member;
9311, enclosure member; 93111, first enclosure member; 112, second enclosure member;
9312, water inlet; 9313, recessed portion; 9314, guide structure; 141, claw; 142, guide cavity;
9321, floating shell; 9322, permanent magnet;
9331, reinforcing rib;
100', sensor fixing cover; 200 ', main shell; 300 ', screws;
94100, sensor;
941, main body; 942, fixing cover; 943, accommodation cavity; 944, bolt;
10, first cavity; 9411, insertion hole; 9412, limiting portion; 9413, bearing portion; 941 4, limiting protrusion; 9415, positioning column; 9416, step;
20, second cavity; 9421, extension arm; 94211, transition portion; 212, snap-fit portion;
9422, limiting groove; 9423, mounting portion; 231, positioning hole; 232, first connection hole; 24, through hole.

### DETAILED DESCRIPTION

The technical solutions in the exemplary embodiments of the present disclosure will be described clearly and completely hereinafter in combination with the accompanying drawings in the exemplary embodiments of the present disclosure. The exemplary embodiments described herein are only for illustrative purposes and are not intended to limit the scope of protection of the present disclosure. Therefore, it should be understood that various modifications and changes can be made to the exemplary embodiments without departing from the scope of protection of the present disclosure.

In the description of the present disclosure, unless otherwise clearly specified and limited, the terms "first" and "second" are used for descriptive purposes only and shall not be understood as indicating or implying relative importance; the term "plurality" refers to two or more; the term "and/or" includes any and all combinations of one or more associated listed items. In particular, reference to "the/said" object or "an" object is also intended to indicate one of possible multiple such objects.

Unless otherwise specified or explained, the terms "connection", "fixing", etc. should be understood in a broad sense. For example, "connection" may be a fixed connection, a detachable connection, an integral connection, an electrical connection, or a signal connection; "connection" may be a direct connection or an indirect connection through an intermediate medium. For those skilled in the art, the specific meanings of the above terms in this disclosure can be understood according to specific circumstances.

Further, in the description of the present disclosure, it should be understood that the directional words such as "above", "below", "inside", "outside" and the like described in the exemplary embodiments of the present disclosure are described from the perspectives shown in the accompanying drawings and shall not be understood as limiting the exemplary embodiments of the present disclosure. It should also be understood that in the context, when it is mentioned that an element or feature is connected "above", "below", or "inside", "outside" the other element(s), it can not only be directly connected "above", "below", "inside", "outside" the other element(s), but also can be indirectly connected "above", "below", or "inside", "outside" the other element(s) through an intermediate element.

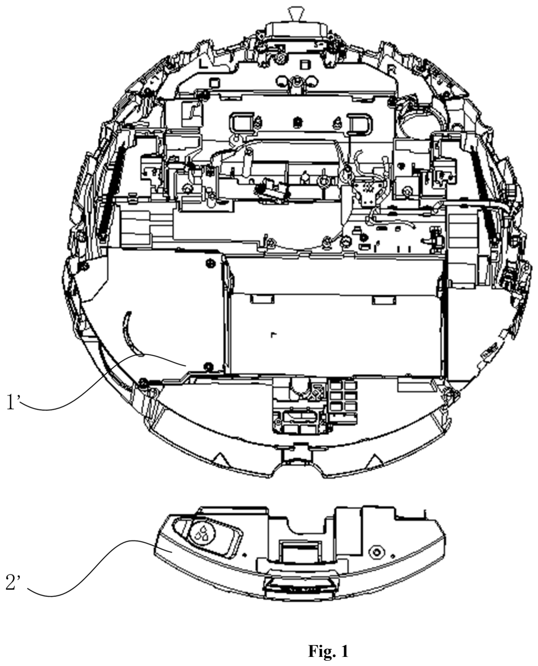

As shown in FIG. 1, in the related cleaning device, the liquid storage tank 2' is detachably connected to the main body 1'. When the liquid storage tank 2' is empty of cleaning liquid, the liquid storage tank 2' is removed from the main body 1' and then filled with cleaning liquid. The liquid storage tank 2' adopts an independent structure, which has a relatively high production cost and poor overall consistency, resulting in an unsightly appearance and a high accident rate, which affects the service life of the cleaning device.

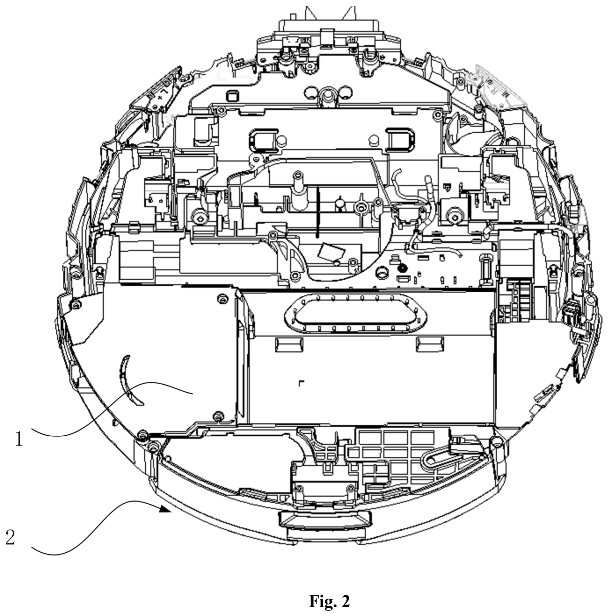

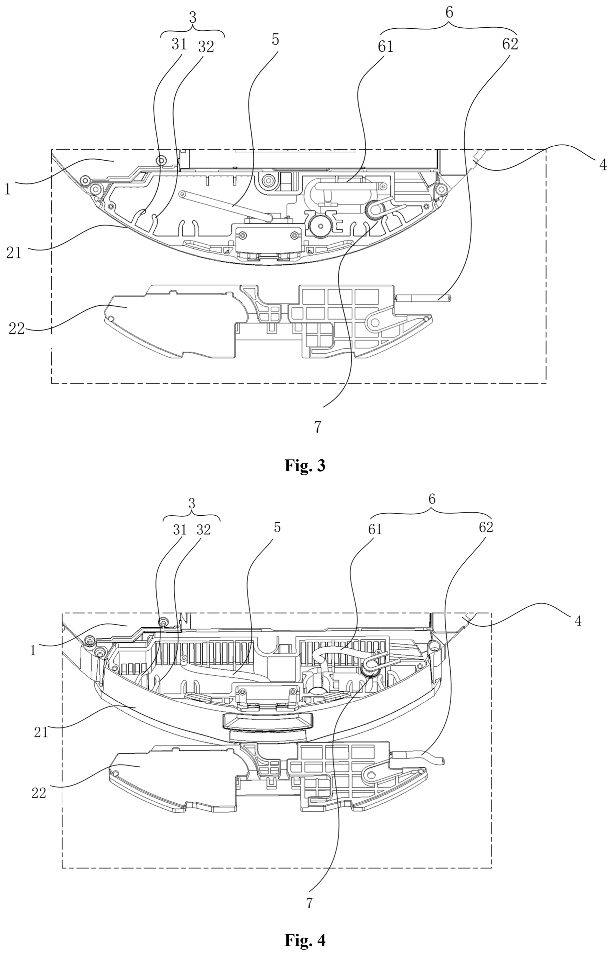

In order to solve this problem, the present embodiment provides a cleaning device, which specifically refers to cleaning devices such as a sweeping robot or a mopping machine, or other types of cleaning devices, such as a floor scrubber. As shown in FIGS. 2 and 3, the cleaning device includes a main body 1 and a liquid storage tank 2. The liquid storage tank 2 is configured to store cleaning liquid. The liquid storage tank 2 includes a bottom shell 21. The bottom shell 21 and the main body 1 are of an integrally formed structure. The cleaning liquid may be water, or water added with a detergent, or other liquids with cleaning ability.

In the cleaning device provided in this embodiment, the main body 1 plays a role of supporting the liquid storage tank 2. The liquid storage tank 2 is configured to store cleaning liquid and can provide the required cleaning liquid for a wet cleaning structure and other components in the cleaning device. The bottom shell 21 of the liquid storage tank 2 and the main body 1 are of an integrally formed structure, which is equivalent to integrating the bottom shell 21 of the liquid storage tank 2 and the main body 1. That is, the bottom shell 21 of the liquid storage tank 2 and the main body 1 are of an integral structure, with good overall consistency, low cost, beautiful appearance, and good reliability. Compared with the independent structure of the liquid storage tank 2' in the related art, the frequency of repeated disassembly and assembly of the liquid storage tank 2 is reduced, the probability of accidents and the number of maintenance times are reduced, and the service life is extended.

It should be particularly noted that the bottom shell 21 and the main body 1 may be formed by injection molding to achieve the purpose of integrally forming the bottom shell 21 and the main body 1, reducing the number of parts and the steps of part assembly, thereby reducing production costs.

The bottom shell 21 and the main body 1 of the liquid storage tank 2 provided in this embodiment are of an integrally formed structure. When the liquid storage tank 2 is short of cleaning liquid, the cleaning liquid may be added to the liquid storage tank 2 through a cleaning base station. A water storage bucket is provided in the cleaning base station, and is detachably connected to the cleaning base station. A user may remove the water storage bucket from the cleaning base station and fill it with cleaning liquid, and then put the water storage bucket into the cleaning base station. The water storage bucket provides the cleaning liquid required for wet cleaning for the liquid storage tank 2 of the cleaning device. In addition, the cleaning base station may also be directly connected to a tap water pipe to supply water to the above-mentioned water storage bucket through the tap water pipe.

In one embodiment, as shown in FIGS. 3-4, the liquid storage tank 2 further includes an upper cover 22. The bottom shell 21 is provided with an open end. The upper cover 22 covers the open end of the bottom shell 21.

The bottom shell 21 is of a shell structure, and a cavity inside the bottom shell 21 provides a storage space for the cleaning liquid. The upper cover 22 covers the open end of the bottom shell 21. The upper cover 22 plays a role of blocking the open end of the bottom shell 21 to prevent the cleaning liquid from leaking from the bottom shell 21.

It should be particularly noted that a sealed space is formed between the upper cover 22 and the bottom shell 21 to store cleaning liquid, and has a good sealing effect.

In one embodiment, the upper cover 22 is made of a transparent material.

After the bottom shell 21 and the upper cover 22 of the liquid storage tank 2 are assembled, whether the internal parts of the liquid storage tank 2 are missing or misassembled can be more conveniently checked through the upper cover 22 made of transparent material, which provides convenience for subsequent maintenance.

It can be understood that the bottom shell 21 and the main body 1 are integrally formed, but the bottom shell 21 and the upper cover 22 are not integrally formed. The reasons are: first, due to the material difference between the bottom shell 21 made of non-transparent material and the upper cover 22 made of transparent material, integral forming is difficult; second, if the bottom shell 21 and the upper cover 22 are integrally formed, then after the injection molding is completed, the upper cover 22 has blocked the open end of the bottom shell 21, and the components inside the liquid storage tank 2 cannot be installed, and other structures inside the bottom shell 21 cannot be inspected and repaired. However, after the bottom shell 21 provided in this embodiment is integrally formed with the main body 1, various components can be installed inside the bottom shell 21 through the open end of the bottom shell 21, and the internal parts of the bottom shell 21 can also be inspected. After the initial installation or inspection is completed, the upper cover 22 and the bottom shell 21 are connected.

It can be understood that the upper cover 22 is made of plastic material such that the material of the upper cover 22 can match that of the bottom shell 21 and the main body 1 formed by injection molding, thereby improving the connection stability between the bottom shell 21 and the upper cover 22.

In one embodiment, as shown in FIGS. 3-4, the cleaning device further includes a wet cleaning structure 4. The liquid storage tank 2 is configured to provide the cleaning liquid to the wet cleaning structure 4. Providing the cleaning liquid to the wet cleaning structure 4 through the liquid storage tank 2 can realize the function of supplying cleaning liquid to the wet cleaning structure 4, thereby achieving the purpose of wet mopping.

It should be noted that the wet cleaning structure 4 may be a mop structure or a roller brush structure. The mop structure or the roller brush structure has a water-absorbing material, such as cotton material, which can drive the mop structure to reciprocate or rotate, or drive the roller brush structure to roll, such that the ground to be cleaned can be wet cleaned.

In one embodiment, as shown in FIGS. 3-4, the cleaning device also includes a liquid inlet pipe 5 disposed in the liquid storage tank 2. The liquid storage tank 2 is provided with a water inlet. A first end of the liquid inlet pipe 5 is communicated with the water inlet, and a second end of the liquid inlet pipe 5 is configured to transport the cleaning liquid to an inner cavity of the liquid storage tank 2.

The liquid inlet pipe 5 serves to communicate the water inlet of the liquid storage tank 2 with the inner cavity of the liquid storage tank 2. The liquid inlet pipe 5 can transport the cleaning liquid from the water inlet of the liquid storage tank 2 to the inner cavity of the liquid storage tank 2.

In one embodiment, as shown in FIGS. 3-4, the cleaning device also includes a liquid outlet pipe assembly 6 disposed in the liquid storage tank 2. A first end of the liquid outlet pipe assembly 6 is communicated with the inner cavity of the liquid storage tank 2, and a second end of the liquid outlet pipe assembly 6 is communicated with the wet cleaning structure 4 and configured to transport the cleaning liquid from the liquid storage tank 2 to the wet cleaning structure 4.

The cleaning liquid in the liquid storage tank 2 is transported to the wet cleaning structure 4 through the liquid outlet pipe assembly 6. The liquid outlet pipe assembly 6 serves to communicate the liquid storage tank 2 with the wet cleaning structure 4, thereby realizing the function of transporting the cleaning liquid to the wet cleaning structure 4 to implement the wet mopping process of the wet cleaning structure 4.

It should be particularly noted that a liquid inlet joint is provided on a side of the upper cover 22 of the liquid storage tank 2 facing the bottom shell 21. The second end of the liquid inlet pipe 5 is connected to the liquid inlet joint, and a connecting through hole is provided on an outer peripheral wall of the liquid inlet joint. The connecting through hole acts as a shower, such that the cleaning liquid transported from the liquid inlet pipe 5 passes through the liquid inlet joint and is sprayed into the inner cavity of the liquid storage tank 2 through the connecting through hole.

It can be understood that the liquid inlet pipe 5 and the liquid inlet joint do not need to be completely sealed to facilitate the cleaning liquid to flow out of the connecting through hole.

In one embodiment, as shown in FIGS. 3-4, the liquid outlet pipe assembly 6 includes a liquid outlet pipe 61 and a liquid outlet joint 62. The liquid outlet pipe 61 is disposed in the liquid storage tank 2 and is communicated with the inner cavity of the liquid storage tank 2. A first end of the liquid outlet joint 62 is communicated with the liquid outlet pipe 61, and a second end of the liquid outlet joint 62 is communicated with the wet cleaning structure 4.

The liquid outlet pipe 61 is disposed in the liquid storage tank 2 and communicated with the inner cavity of the liquid storage tank 2. The liquid outlet pipe 61 can draw the cleaning liquid from the inner cavity of the liquid storage tank 2. The first end of the liquid outlet joint 62 is communicated with the liquid outlet pipe 61, and the second end of the liquid outlet joint 62 is communicated with the wet cleaning structure 4. The liquid outlet joint 62 plays a role of intermediate communication, such that the cleaning liquid flowing out of the liquid outlet pipe 61 is transported to the wet cleaning structure 4 through the liquid outlet joint 62, so as to achieve the purpose of transporting the cleaning liquid to the wet cleaning structure 4.

In one embodiment, the cleaning device further includes an overflow structure 7 disposed on the liquid storage tank 2 and configured to deal with overflow of the cleaning liquid in the liquid storage tank 2.

Since the internal space of the liquid storage tank 2 is limited, the overflow structure 7 is provided on the upper cover 22 of the liquid storage tank 2 in order to prevent the liquid storage tank 2 from being overfilled. If there is too much cleaning liquid in the liquid storage tank 2, the cleaning liquid in the liquid storage tank 2 may be discharged through the overflow structure 7, thereby avoiding the situation where the liquid storage tank 2 is damaged due to being overfilled with cleaning liquid, which can protect the liquid storage tank 2.

It should be noted that the overflow structure 7 may specifically be an overflow valve, which is generally a one-way valve that limits the flow direction of the cleaning liquid such that the cleaning liquid can only flow from the inside of the liquid storage tank 2 to the outside of the liquid storage tank 2.

It should be noted that the overflow structure 7 may be alternatively provided on the upper cover 22 of the liquid storage tank 2, such that the cleaning liquid can overflow and be discharged through the overflow structure 7 only when the liquid storage tank 2 is filled with the cleaning liquid.

In one embodiment, the bottom shell 21 and the upper cover 22 are connected by ultrasonic processing.

When the bottom shell 21 and the upper cover 22 are connected by ultrasonic technology, the following stages are required:
First, in a clamping stage, the bottom shell 21 and the upper cover 22 are each placed in a fixture respectively, and a welding head is adopted to press down and contact the upper cover 22, and then the welding head applies pressure to press the upper cover 22 tightly against the bottom shell 21.
Second, in a contact stage, the welding head oscillates vertically at a certain frequency. Since the plastics of the bottom shell 21 and the upper cover 22 have poor thermal conductivity and cannot dissipate heat in time, the mechanical oscillation energy is conducted and gathered in a contact area between the bottom shell 21 and the upper cover 22, which may also be called a welding surface. Since the contact area has a relatively large acoustic impedance as an interface, local high temperature will be generated, and the mechanical oscillation energy will generate heat energy through intense friction.
Third, in a melting stage, when the temperature of the welding surface reaches the melting point of the plastic, the plastic melts, causing the bottom shell 21 and the upper cover 22 to fuse into one, and then the oscillation stops.
Fourth, in a pressure holding stage, the welding head continues to apply pressure until the contact area between the bottom shell 21 and the upper cover 22 cools and solidifies, such that the bottom shell 21 and the upper cover 22 are solidified.
Fifth, in a blanking stage, after the contact area between the bottom shell 21 and the upper cover 22 solidifies, the welding head is reset and no longer applies pressure to the upper cover 22. At this time, the two plastic parts, i.e., the bottom shell 21 and the upper cover 22, are fused together, thereby completing the entire ultrasonic welding process.

By adopting this ultrasonic welding process, a strong molecular chain can be formed to achieve the purpose of welding, and the welding strength can be close to the strength of the raw materials of the bottom shell 21 and the upper cover 22 themselves.

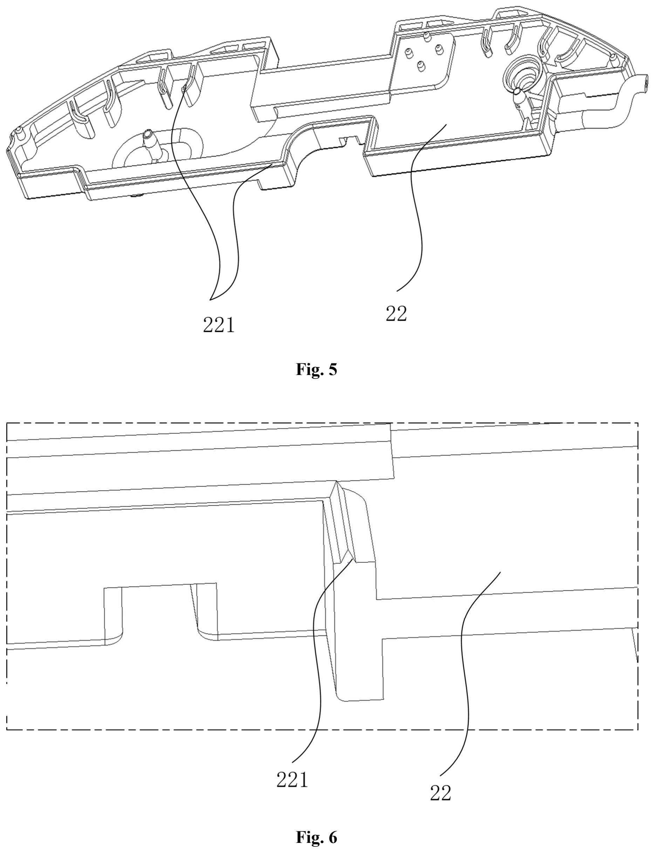

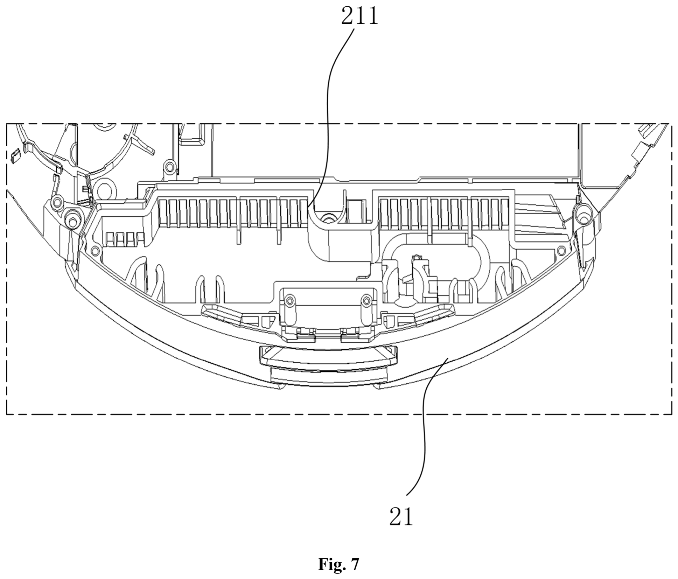

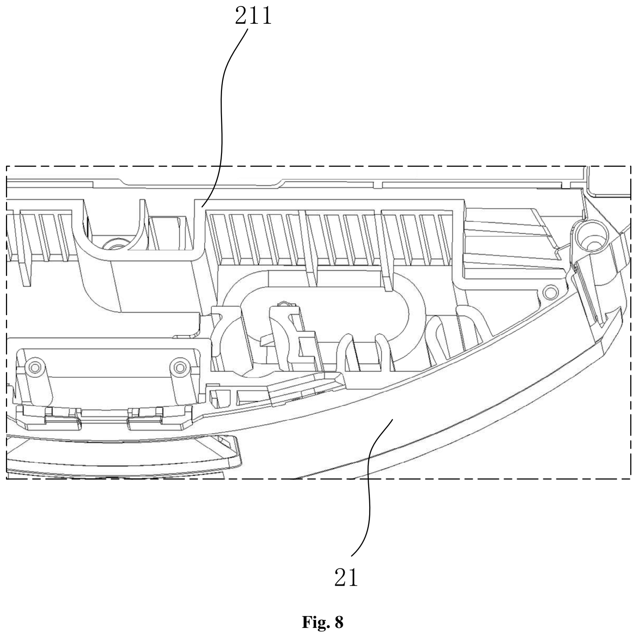

In one embodiment, as shown in FIGS. 5 to 8, one of the upper cover 22 and the bottom shell 21 is provided with an ultrasonic protrusion 221, and the other of the upper cover 22 and the bottom shell 21 is provided with an ultrasonic surface 211. The ultrasonic protrusion 221 is connected to the ultrasonic surface 211, such that the bottom shell 21 and the upper cover 22 are connected through an ultrasonic process.

It can be understood that end surfaces of the sides where the upper cover 22 and the bottom shell 21 face each other form the contact area between the bottom shell 21 and the upper cover 22. The ultrasonic protrusion 221 is protruding relative to the bottom shell 21 or the upper cover 22. The ultrasonic protrusion 221 may form a molten glue under the vibration pressure of the ultrasonic wave. The ultrasonic protrusion 221 provides the raw materials for welding the bottom shell 21 and the upper cover 22 in a molten state and provides a reserved position for the melting of the plastic. The ultrasonic surface 211 is of a planar structure with a relatively high flatness, which provides a good attachment position for the molten glue, such that the ultrasonic protrusion 221 and the ultrasonic surface 211 are tightly connected, thereby improving the reliability of the connection between the bottom shell 21 and the upper cover 22.

It should be particularly noted that the ultrasonic protrusion 221 may be provided on the side of the upper cover 22 facing the bottom shell 21, and the ultrasonic surface 211 may be provided on the side of the bottom shell 21 facing the upper cover 22. Alternatively, the ultrasonic surface 211 may be provided on the side of the upper cover 22 facing the bottom shell 21, and the ultrasonic protrusion 221 may be provided on the side of the bottom shell 21 facing the upper cover 22.

It can be understood that in some other embodiments, one of the end surfaces of the sides where the upper cover 22 and the bottom shell 21 are close to each other is provided with the ultrasonic protrusion 221, and the other one may also be provided with an ultrasonic groove. The ultrasonic protrusion 221 is at least partially disposed in the ultrasonic groove. The ultrasonic groove acts as a glue overflow groove, thereby reducing the glue overflow after the ultrasonic protrusion 221 produces molten glue.

Therefore, the present embodiment does not limit the specific forms in which the bottom shell 21 and the upper cover 22 are provided with the ultrasonic surface 211 and the ultrasonic protrusion 221, which may be adjusted according to actual production conditions.

In one embodiment, as shown in FIGS. 5-8, the ultrasonic protrusion 221 is of a closed ring structure, and the ultrasonic protrusion 221 is disposed along the circumference of the upper cover 22; the ultrasonic surface 211 is of a closed ring structure, and the ultrasonic surface 211 is disposed along the circumference of the bottom shell 21.

The ultrasonic protrusion 221 is disposed along the circumference of the upper cover 22, and the ultrasonic protrusion 221 is of a closed ring structure, such that the ultrasonic protrusion 221 is of a continuous structure of a whole circle. The ultrasonic surface 211 is disposed along the circumference of the bottom shell 21, and the ultrasonic surface 211 is of a closed ring structure, such that the ultrasonic surface 211 is of a continuous structure of a whole circle. With this provision, the fusion area between the ultrasonic protrusion 221 and the ultrasonic surface 211 is continuous, thereby improving the stability and reliability of the welding between the bottom shell 21 and the upper cover 22.

In one embodiment, a cross-section of the ultrasonic protrusion 221 is at least one of a triangle, an arc, and a polygon.

When the cross-section of the ultrasonic protrusion 221 is triangular, it may also be called a triangular ultrasonic line, which is equivalent to the volume of the ultrasonic protrusion 221 gradually decreasing in the direction where the upper cover 22 and the bottom shell 21 approach each other. That is, the ultrasonic protrusion 221 is of a pointed structure. During the welding process, the molten glue formed by the melting of the ultrasonic protrusion 221 covers the ultrasonic surface 211, such that the contact area between the bottom shell 21 and the upper cover 22 is continuous and has no welding boundary and boundary stress, and the welding interface gap is uniform, thereby improving the stability of the welding and effectively improving the welding efficiency.

By adopting this ultrasonic welding process, a triangular ultrasonic line is provided on the upper cover 22 of the liquid storage tank 2. When the ultrasonic operation is completed, the triangular ultrasonic line of the upper cover 22 is completely fused and bonded with the ultrasonic surface 211 of the main body 1, so as to completely connect the two independent components, i.e., the upper cover 22 of the liquid storage tank 2 and the main body 1, together, thereby achieving the purpose of an integrated liquid storage tank 2, while improving the overall consistency, and also improving the sealing of the liquid storage tank 2.

It can be understood that the cross-sectional shape of the ultrasonic protrusion 221 is not limited in the present embodiment, and the specific actual cross-sectional shape may be adjusted according to actual production conditions.

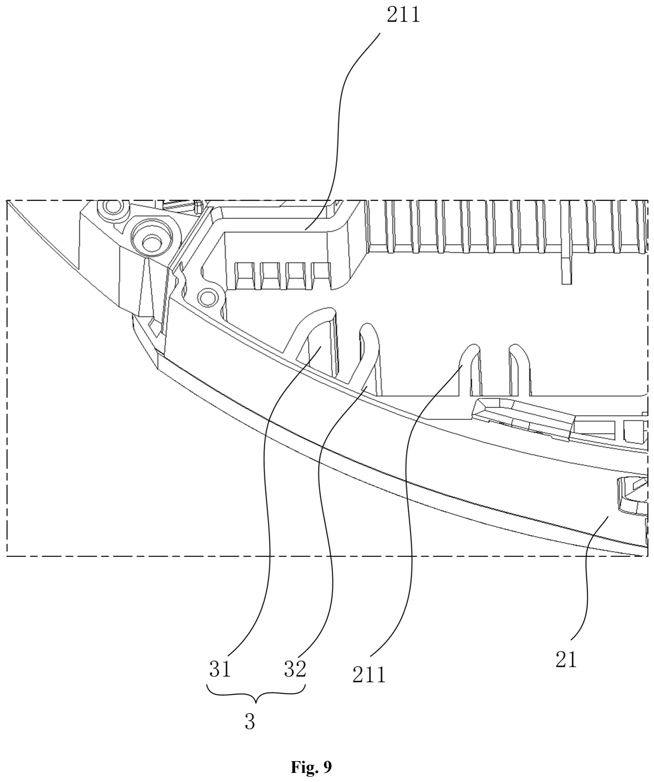

In one embodiment, as shown in FIG. 9, the bottom shell 21 includes a shell and a protective structure 3. The protective structure 3 is disposed in the shell to prevent the liquid storage tank 2 from impact and deformation.

The shell is of a hollow structure, and the structural strength of the shell may be slightly poor. For this reason, the protective structure 3 is disposed in the shell. The protective structure 3 plays a role of protecting the shell, enhancing the structural strength of the shell, and reducing the deformation of the entire liquid storage tank 2 due to impact, thereby improving the impact resistance of the liquid storage tank 2 having the integral structure.

It can be understood that the shell and the upper cover 22 may be connected by an ultrasonic process, and the protective structure 3 and the upper cover 22 may be connected by an ultrasonic process.

In one embodiment, the protective structure 3 includes a rib plate. A bottom of the rib plate is connected to a bottom wall of the shell, and a side wall of the rib plate is connected to a side wall of the shell.

Since the bottom wall of the shell and the side wall of the shell are connected to each other, the connection position between the bottom wall of the shell and the side wall of the shell may be weak. The bottom of the rib plate and the side wall of the rib plate are respectively connected to the bottom wall of the shell and the side wall of the shell, which is equivalent to the rib plate being disposed near the connection position between the bottom wall of the shell and the side wall of the shell, thereby enhancing the structural strength of the connection position.

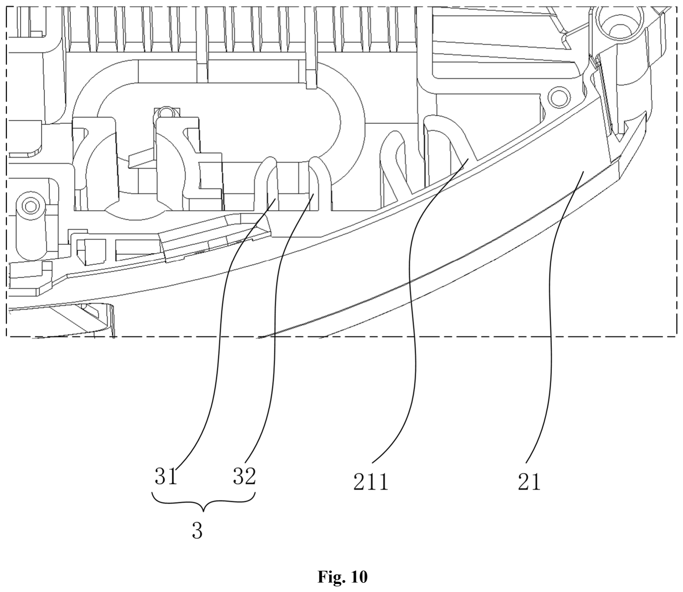

In one embodiment, as shown in FIGS. 9-10, the rib plate includes a first rib plate 31 and a second rib plate 32. The first rib plate 31 and the second rib plate 32 are bent toward sides opposite to each other.

The first rib plate 31 and the second rib plate 32 are connected to the bottom wall and the side wall of the shell. The first rib plate 31 and the second rib plate 32 play a role of reinforcing ribs to improve the structural strength of the entire bottom shell 21. The first rib plate 31 and the second rib plate 32 adopt a curved structure. Compared with a rib plate structure with a straight plate structure, the first rib plate 31 and the second rib plate 32 have a greater structural strength and are less likely to break and bend. The first rib plate 31 and the second rib plate 32 are bent toward sides opposite to each other. That is, the bending directions of the first rib plate 31 and the second rib plate 32 are not in the same direction, but are opposite to each other. The structural strengths of the first rib plate 31 and the second rib plate 32 with different bending directions complement each other. Even if the two sides of the protective structure 3 are impacted in opposite directions, it can still ensure good structural strength, further preventing the liquid storage tank 2 from being deformed after being impacted, thereby improving the impact resistance of the liquid storage tank 2 with the integral structure.

It can be understood that the number of the protective structures 3 may be multiple, and multiple protective structures 3 are disposed in the shell and along the side wall of the shell. For positions where the shell structure strength is relatively weak, the number of the protective structures 3 is appropriately increased. The specific number and arrangement positions of the protective structures 3 may be adjusted according to actual production conditions.

One embodiment of the present disclosure provides a cleaning device, which specifically refers to a cleaning device such as a sweeping robot or a mopping machine, or other types of cleaning devices, such as a floor scrubber, etc. The cleaning device has a mopping function or a sweeping function or both a sweeping and mopping function, and can automatically complete the cleaning and mopping of the room floor. With reference to FIG. 1, the cleaning device includes a water storage tank 901 and a wet cleaning structure 904. A water inlet 9016 is provided on the water storage tank 901. Cleaning liquid is provided to the water storage tank 901 through the water inlet 9016. The water storage tank 901 is configured to store the cleaning liquid, and the water storage tank 901 is configured to provide the cleaning liquid to the wet cleaning structure 904. The cleaning liquid may be water, water added with a detergent, or other liquids with cleaning ability.

In the cleaning device provided in this embodiment, the water storage tank 901 is configured to store the cleaning liquid, and the water storage tank 901 provides a storage space for the cleaning liquid. The water storage tank 901 provides the cleaning liquid to the wet cleaning structure 904, realizing the function of supplying the cleaning liquid to the wet cleaning structure 904, thereby achieving the purpose of wet mopping. The wet cleaning structure 904 may be a mop structure or a roller brush structure. The mop structure or the roller brush structure has a water-absorbing material, such as cotton material, etc., which can drive the mop structure to reciprocate or rotate, or drive the roller brush structure to roll, such that the ground to be cleaned can be wet cleaned.

The cleaning device also includes a water supply pipe 906 disposed between the water storage tank 901 and the wet cleaning structure 904. A first end of the water supply pipe 906 is communicated with the water inlet 9016 of the water storage tank 901, and a second end of the water supply pipe 906 is communicated with the wet cleaning structure 904, such that the cleaning liquid in the water storage tank 901 is transported to the wet cleaning structure 904 through the water supply pipe 906, so as to realize the process of supplying and transporting the cleaning liquid.

Since the internal space of the water storage tank 901 is limited, as shown in FIG. 1, the cleaning device further includes an overflow structure 902 disposed on the water storage tank 901, in order to prevent the water storage tank 901 from being overfilled with cleaning liquid. If there is too much cleaning liquid in the water storage tank 901, the cleaning liquid in the water storage tank 901 may be discharged through the overflow structure 902, thereby preventing the water storage tank 901 from being overfilled with the cleaning liquid and causing damage, thereby protecting the water storage tank 901.

It should be noted that the overflow structure 902 is specifically an overflow valve, which is generally a one-way valve that limits the flow direction of the cleaning liquid. That is, the cleaning liquid can only flow one-way from the inside of the water storage tank 901 to the outside of the water storage tank 901.

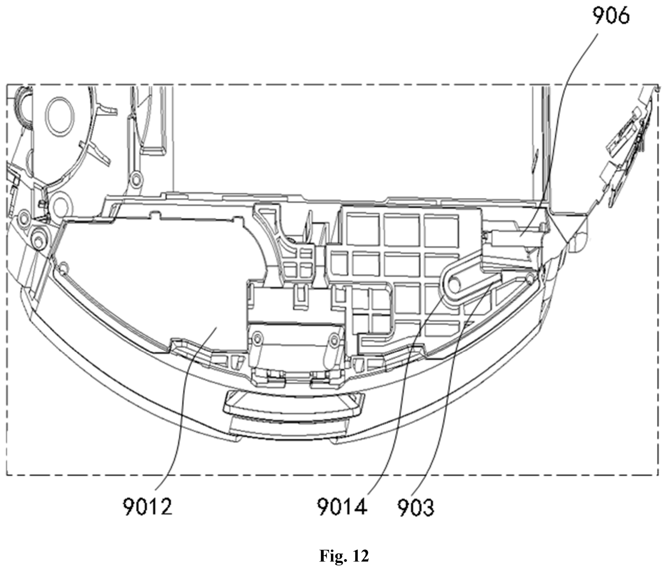

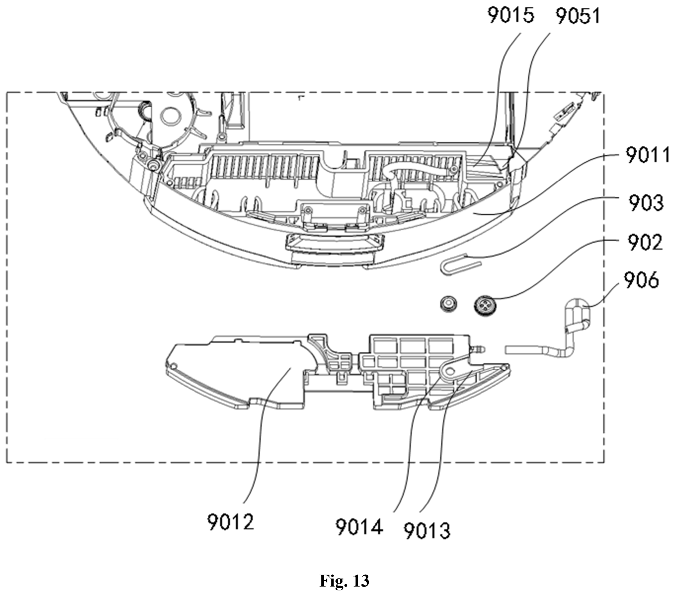

It can be understood that, as shown in FIGS. 12 and 13, the water storage tank 901 specifically includes a bottom shell 9011 and an upper cover 9012. One side of the bottom shell 9011 is provided with an open end. The upper cover 9012 covers the open end of the bottom shell 9011. The upper cover 9012 plays a role of blocking the open end of the bottom shell 9011. The bottom shell 9011 and the upper cover 9012 may be detachably connected or fixedly connected. It should be noted that the overflow structure 902 is provided on the upper cover 9012 of the water storage tank 901. When the water storage tank 901 is filled with the cleaning liquid, the overflow structure 902 realizes the discharge of the overflow liquid.

It should be noted that the water storage tank 901 is full of cleaning liquid when the water inlet 9016 of the water storage tank 901 is supplied with cleaning liquid by the base station and the maximum capacity limit of the water storage tank 901 is reached. At this time, the cleaning liquid will overflow from the overflow structure 902 to prevent the base station from continuously supplying cleaning liquid to the water storage tank 901, which causes the water storage tank 901 to be overfilled with cleaning liquid. Therefore, when the water storage tank 901 is just full of liquid, the cleaning liquid does not overflow from the overflow structure 902, but only when the hydraulic pressure in the water storage tank 901 is relatively high, the cleaning liquid will overflow from the overflow structure 902.

When the overflow structure 902 is actually working, the cleaning liquid overflowing from the overflow structure 902 will flow disorderly around the overflow structure 902, which is likely to damage other parts inside the cleaning device and affect the service life of the cleaning device. To this end, as shown in FIGS. 12 and 13, the cleaning device provided in this embodiment also includes an enclosing structure 903, which is at least partially disposed around the overflow structure 902 to retain and guide the cleaning liquid flowing out of the overflow structure 902.

In the cleaning device provided in this embodiment, the enclosing structure 903 is at least partially disposed around the overflow structure 902. The enclosing structure 903 serves to block and retain the cleaning liquid flowing out of the overflow structure 902, preventing the cleaning liquid from overflowing in all directions to the periphery of the overflow structure 902, thereby reducing the impact on other parts of the cleaning device, reducing the number and cost of repair and maintenance of the cleaning device, and extending the service life of the cleaning device. At the same time, the cleaning liquid overflowing from the overflow structure 902 is discharged under the drainage effect of the enclosing structure 903, and the enclosing structure 903 can guide the overflowing cleaning liquid such that the overflowing cleaning liquid may be discharged in an orderly manner.

It can be understood that, since the overflow structure 902 is disposed on the upper cover 9012 of the water storage tank 901, the enclosing structure 903 is also disposed on the upper cover 9012 of the water storage tank 901 to retain the overflowing cleaning liquid.

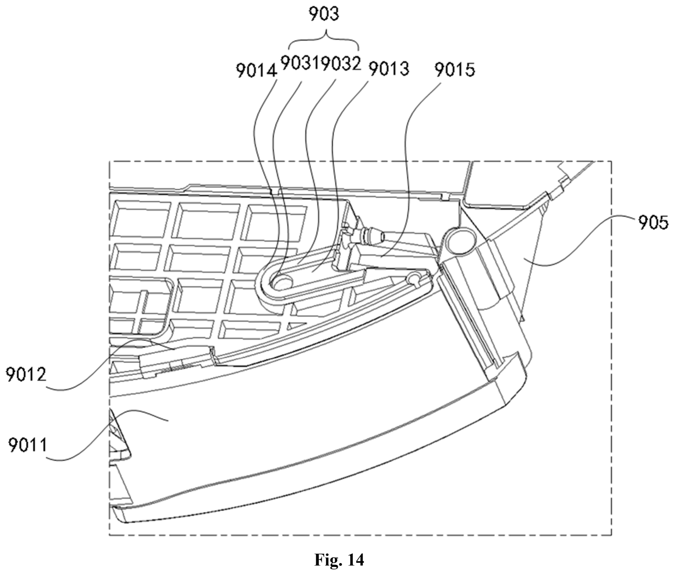

In one embodiment, as shown in FIG. 14, the enclosing structure 903 may be a semi-open structure. Specifically, the enclosing structure 903 is provided with an open end such that the cleaning liquid flowing out from the overflow structure 902 can flow out through the open end of the enclosing structure 903.

If a lot of cleaning liquid overflows out of the overflow structure 902, the overflowing cleaning liquid will still overflow from the enclosing structure 903 when the enclosing structure 903 cannot completely contain the overflowing cleaning liquid, and the overflow of the cleaning liquid may also be disordered. For this reason, the open end is provided in the enclosing structure 903, such that the enclosing structure 903 is not a completely closed structure, and the overflowing cleaning liquid can flow out through the open end of the enclosing structure 903, which plays a role of discharging the overflowing cleaning liquid. At this time, the open end of the enclosing structure 903 serves as a drain to achieve the function of draining the cleaning liquid.

It should be noted that the shape of the overflow structure 902 may be a cylindrical structure, and the enclosing structure 903 may be a U-shaped structure. The U-shaped structure includes an arc portion 9031 and two linear portions 9032. The arc portion 9031 is wrapped around the outside of the overflow structure 902. The arc portion 9031 plays a role of retaining the cleaning liquid. Two ends of the arc portion 9031 are correspondingly connected to the two linear portions 9032. While playing a role of retaining, the linear portion 9032 itself extends to drain and guide the overflowing cleaning liquid. It can be understood that the enclosing structure 903 of the U-shaped structure is equivalent to retaining three sides of the overflow structure 902, and the other side is open for discharging the overflowing cleaning liquid.

In one embodiment, a portion of the water storage tank 901 disposed inside the enclosing structure 903 is provided with a guide slope 9013, which is inclined in a direction away from the overflow structure 902 and is configured to divert the cleaning liquid flowing out of the overflow structure 902.

It can be understood that the top surface of the water storage tank 901 and the two linear portions 9032 of the enclosing structure 903 substantially form a groove structure, and the groove structure plays a role of guiding the overflowing cleaning liquid. That is, the part of the top surface of the upper cover 9012 disposed inside the enclosing structure 903 is equivalent to a groove bottom of the groove structure, and the sides of the two linear portions 9032 in the enclosing structure 903 that face each other are equivalent to side walls of the groove structure. The guide slope 9013 is provided at the part of the water storage tank 901 disposed inside the enclosing structure 903. That is, the groove bottom of the groove structure is substantially inclined. At the same time, since the guide slope 9013 is inclined in a direction away from the overflow structure 902, the cleaning liquid overflowing from the overflow structure 902 can flow to the open end of the enclosing structure 903 through the guide slope 9013 under the guidance of the guide slope 9013, thereby accelerating the speed of the cleaning liquid being discharged from the open end of the enclosing structure 903, and improving the timeliness and effectiveness of the liquid discharge.

In one embodiment, as shown in FIG. 14, the water storage tank 901 is provided with a limiting structure 9014 configured to limit the enclosing structure 903.

The limiting structure 9014 is provided in the water storage tank 901 and serves to limit the position of the enclosing structure 903, thereby avoiding a large displacement of the position of the enclosing structure 903 and ensuring the position stability of the enclosing structure 903.

Specifically, a reinforcing rib net is provided on the upper cover 9012 of the water storage tank 901. The reinforcing rib net includes a plurality of horizontal ribs and vertical ribs disposed in an interlaced manner. The reinforcing rib net plays a role of reinforcing the upper cover 9012. The reinforcing rib net around the overflow structure 902 is similar to a U-shaped structure to achieve effective avoidance of the overflow structure 902. At this time, the shape of the reinforcing rib net of the U-shaped structure matches that of the enclosing structure 903 of the U-shaped structure, such that the reinforcing rib net of the U-shaped structure can not only strengthen the structure of the upper cover 9012, but also limit the enclosing structure 903.

In one embodiment, a side of the enclosing structure 903 close to the overflow structure 902 is at least partially attached to the overflow structure 902, and a side of the enclosing structure 903 away from the overflow structure 902 is at least partially attached to the limiting structure 9014.

In other words, the two sides of the arc portion 9031 of the enclosing structure 903 are respectively attached to the overflow structure 902 and the limiting structure 9014, and at this point, the arc portion 9031 may intercept the overflowing cleaning liquid. At the same time, under the joint action of the overflow structure 902 and the limiting structure 9014, it is equivalent to sandwiching the arc portion 9031 between the overflow structure 902 and the limiting structure 9014, thereby improving the position stability of the arc portion 9031. An outer side surface of the linear portion 9032 of the enclosing structure 903 is attached to the limiting structure 9014, and an inner side surface of the linear portion 9032 of the enclosing structure 903 may guide the overflowing cleaning liquid.

It should be particularly noted that the enclosing structure 903 is higher than the limiting structure 9014, so as to improve the retaining effect of the enclosing structure 903 on the cleaning liquid.

In one embodiment, the enclosing structure 903 is made of sealing foam, such that the enclosing structure 903 not only serves as a barrier, but also has a sealing and waterproofing effect, thereby preventing overflowing water from leaking through a gap between the enclosing structure 903 and the water storage tank 901, and has good sealing performance.

In one embodiment, the cleaning device further includes a protective cover disposed on the top of the water storage tank 901. The enclosing structure 903 is sandwiched between the protective cover and the water storage tank 901.

The protective cover is specifically disposed above the upper cover 9012 in the water storage tank 901, and the protective cover plays a role of isolating and protecting the upper cover 9012. The enclosing structure 903 is sandwiched between the protective cover and the upper cover 9012 of the water storage tank 901, such that the two side surfaces of the enclosing structure 903 respectively abut against an inner wall of the protective cover and an outer wall of the upper cover 9012, and the enclosing structure 903 is clamped and fixed due to its own elasticity.

In one embodiment, the enclosing structure 903 is capable of guiding the cleaning liquid overflowing from the overflow structure 902 to the wet cleaning structure 904.

Since the wet cleaning structure 904 requires a large amount of cleaning liquid, the enclosing structure 903 may guide the cleaning liquid overflowing from the overflow structure 902 to the wet cleaning structure 904, such that the overflowing cleaning liquid is not only converted into liquid that has a negative impact, but also provides the required cleaning liquid for the wet cleaning structure 904, thereby playing a role in waste recycling. While reducing the waste of cleaning liquid, it can also reduce the damage of the overflowing cleaning liquid to other parts, thereby extending the service life of the cleaning device.

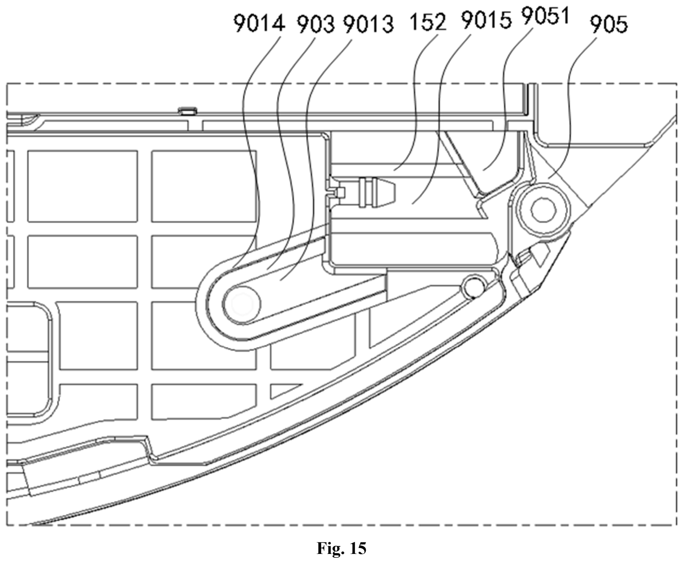

In one embodiment, as shown in FIGS. 14 and 15, the water storage tank 901 is provided with a water trough 9015. A first end of the water trough 9015 is configured to receive the cleaning liquid flowing out from the open end of the enclosing structure 903, and a second end of the water trough 9015 is configured to discharge the cleaning liquid. For example, the second end of the water trough 9015 may be communicated with the wet cleaning structure 904.

Specifically, the water trough 9015 is provided on the bottom shell 9011 of the water storage tank 901. The water trough 9015 is essentially of a through-trough structure. The first end of the water trough 9015 is configured to receive water flowing out from the open end of the enclosing structure 903, and the second end of the water trough 9015 is communicated with the wet cleaning structure 904. That is, a water inlet of the water trough 9015 is connected to the opening of the enclosing structure 903, such that the cleaning liquid flowing out from the open end of the enclosing structure 903 flows into the water trough 9015 through the water inlet of the water trough 9015, and then the cleaning liquid is discharged from a water outlet of the water trough 9015 to the wet cleaning structure 904, so as to realize the function of auxiliary water supply to the wet cleaning structure 904.

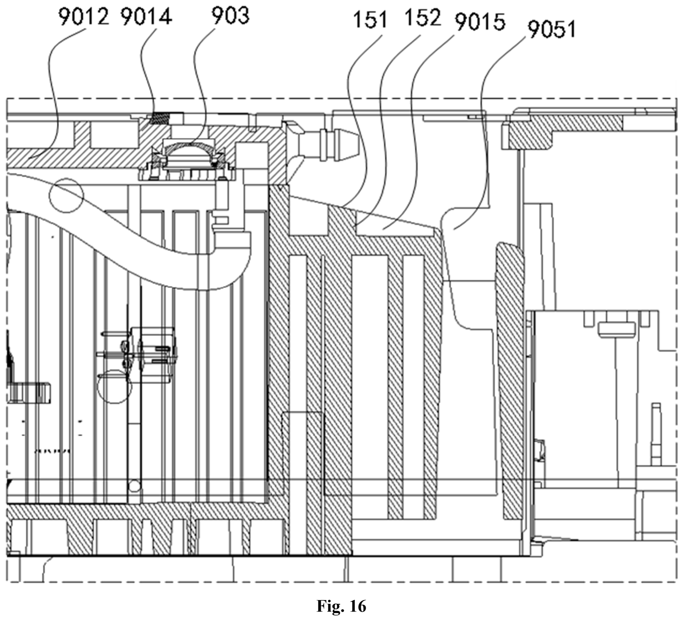

It should be noted that, as shown in FIGS. 15 and 16, the water trough 9015 is disposed at a portion of the bottom shell 9011 that is not provided with an open end. In other words, the water trough 9015 is disposed at an edge of the bottom shell 9011, which is equivalent to a notch of the bottom shell 9011, such that the water trough 9015 and the upper cover 9012 are disposed on the bottom shell 9011 without affecting each other.

In one embodiment, a bottom of the water trough 9015 is provided with a bevel 151 which is inclined in a direction away from the opening of the enclosing structure 903, such that the cleaning liquid flowing out from the opening end of the enclosing structure 903 is drained to the wet cleaning structure 904 through the bevel 151.

The bevel 151 is provided at the bottom of the water trough 9015, and the bevel 151 is inclined in a direction away from the opening of the enclosing structure 903, such that the cleaning liquid in the water trough 9015 flows to the wet cleaning structure 904 through the bevel 151 under the guidance of the bevel 151, thereby accelerating the discharge speed of the overflowing cleaning liquid from the water trough 9015 and improving the timeliness and effectiveness of drainage.

In one embodiment, the bottom of the water trough 9015 is provided with reinforcing ribs 152. The bottom of the water trough 9015 is provided with reinforcing ribs 152, which play a role of reinforcing the bottom structure of the water trough 9015, thereby improving the structural strength of the water trough 9015 and extending the service life.

If the bevel 151 is directly processed on the end surface of the bottom shell 9011 of the water storage tank 901, it is equivalent to cutting the end surface of the bottom shell 9011, which will reduce the structural strength of the bottom shell 9011 and there is a risk of affecting the realization of other functions of the water storage tank 901. Therefore, the reinforcing ribs 152 provided in this embodiment are protruding from the bottom of the water trough 9015, which not only does not affect the structural strength of the water storage tank 901, but also does not affect the realization of other functions of the water storage tank 901.

In one embodiment, there are multiple reinforcing ribs 152, and the multiple reinforcing ribs 152 are disposed in parallel and at intervals, so as to further strengthen the structure of the water storage tank 901.

It can be understood that a delivery channel may be formed between two adjacent reinforcing ribs 152, the delivery channel is configured to drain the cleaning liquid, and the delivery channel may divert the overflowing cleaning liquid.

In one embodiment, the cleaning device further includes a main body 905. The water storage tank 901 is disposed in the main body 905. The main body 905 is provided with a communicating hole 9051. The water trough 9015 is communicated with the wet cleaning structure 904 through the communicating hole 9051.

The water storage tank 901 is provided in the main body 905, and the main body 905 plays a role of supporting and accommodating the water storage tank 901. The water trough 9015 is communicated with the wet cleaning structure 904 through the communicating hole 9051 of the main body 905, and the communicating hole 9051 plays a role of intermediate communication, such that the cleaning liquid flowing out of the water outlet of the water trough 9015 is discharged to the wet cleaning structure 904 through the communicating hole 9051.

It should be particularly noted that the communicating hole 9051 is disposed in the vertical direction, such that the communicating hole 9051 directly falls into the wet cleaning structure 904 under the action of its own gravity.

It should be particularly noted that the communicating hole 9051 is as straight as possible to avoid the communicating hole 9051 having a curved structure from affecting the speed of discharging cleaning liquid.

The operation process of the cleaning device provided in this embodiment is as follows:
If there is too much cleaning liquid in the water storage tank 901 and the hydraulic pressure inside the water storage tank 901 is too high, the cleaning liquid in the water storage tank 901 may be discharged through the overflow structure 902, and the enclosing structure 903 blocks and retains the cleaning liquid overflowing from the overflow structure 902. Under the guidance of the guide slope 9013, the cleaning liquid overflowing from the overflow structure 902 flows to the open end of the enclosing structure 903 through the guide slope 9013, and the cleaning liquid flowing out from the open end of the enclosing structure 903 flows into the water trough 9015 through the water inlet of the water trough 9015. Under the guidance of the bevel 151, the cleaning liquid in the water trough 9015 flows to the wet cleaning structure 904 through the bevel 151 and the communicating hole 9051, so as to realize the function of auxiliary water supply to the wet cleaning structure 904.

The cleaning device provided in this embodiment adopts sealing and waterproof measures to effectively avoid the disorderly flow of overflow water from the overflow structure 902 of the water storage tank 901, and utilizes the method of blocking three sides of the enclosing structure 903 and opening one side to convert the excess cleaning liquid that is inevitably produced in the normal operation of the cleaning device into water that does not produce negative effects, thereby ensuring the normal service life of the cleaning device, reducing the number of repairs and maintenance of the cleaning device to a certain extent, and improving the user experience.

This embodiment is similar to the above embodiment, except that the communication path between the enclosing structure 903 and the wet cleaning structure 904 and the specific structure of the enclosing structure 903 are different.

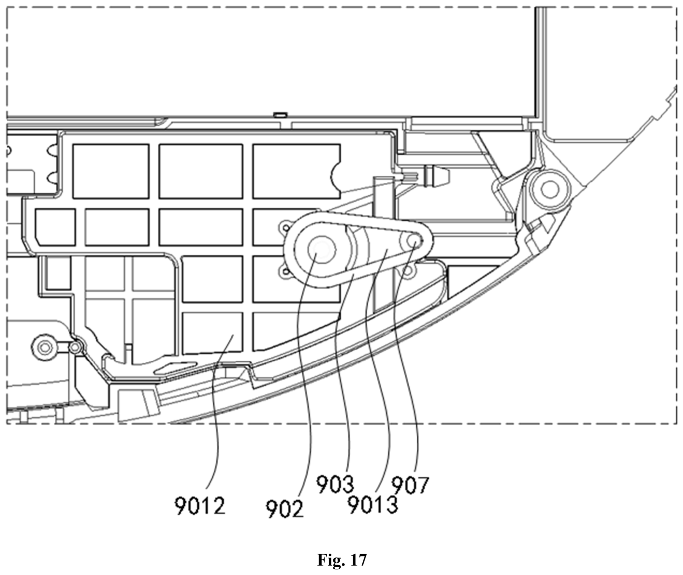

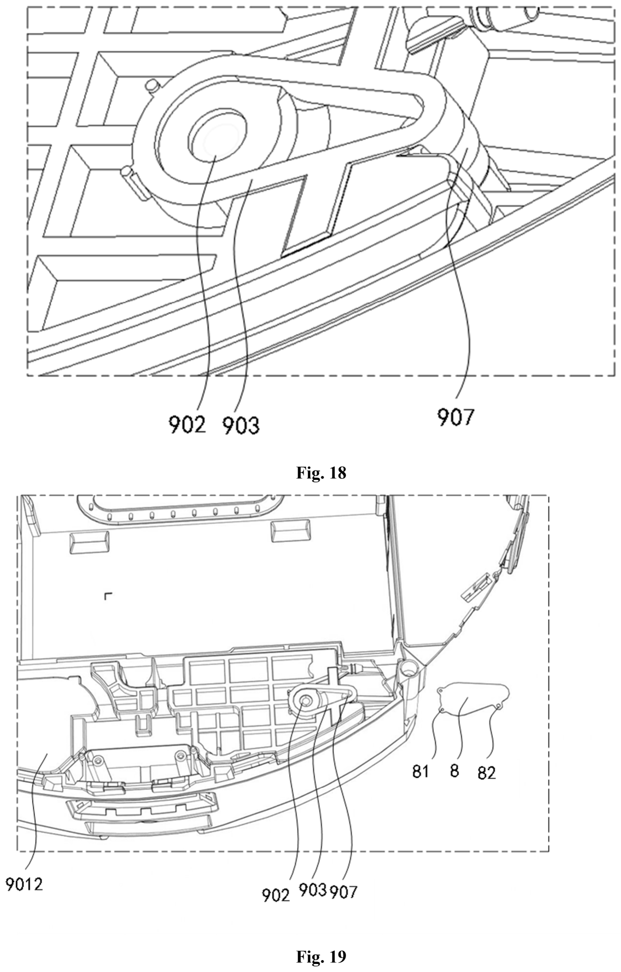

As shown in FIGS. 17 to 20, the cleaning device provided in this embodiment also includes a guide pipe 907. The enclosing structure 903 is a closed ring structure. The enclosing structure 903 is disposed around the overflow structure 902 and the guide pipe 907, such that the cleaning liquid flowing out of the overflow structure 902 flows out through the guide pipe 907.

The overflow structure 902, the enclosing structure 903 and the guide pipe 907 are disposed on the upper cover 9012 of the water storage tank 901, and the enclosing structure 903 is disposed around the overflow structure 902 and the guide pipe 907, such that the enclosing structure 903 surrounds the overflow structure 902 and the guide pipe 907. The cleaning liquid can also be directly guided into the guide pipe 907, while preventing the cleaning liquid flowing out of the overflow structure 902 from flowing freely.

It can be understood that if the flow rate of the cleaning liquid flowing out of the overflow structure 902 is relatively large, the height of the enclosing structure 903 of the closed structure needs to be higher than the height of the overflow structure 902, or the gap between the enclosing structure 903 of the closed structure and the overflow structure 902 is relatively large, so as to ensure that a certain amount of cleaning liquid can be stored.

In one embodiment, the guide pipe 907 may be communicated with the wet cleaning structure 904, such that the cleaning liquid flowing out from the overflow structure 902 flows to the wet cleaning structure 904 through the guide pipe 907. The guide pipe 907 plays a role of intermediate communication, directly transporting the cleaning liquid to the wet cleaning structure 904 in a directional manner.

The guide pipe 907 may be approximately of a vertical structure, and the cleaning liquid, under the action of its own gravity, directly falls into the wet cleaning structure 904 through the guide pipe 907, thereby preventing the guide pipe 907 with the curved structure from affecting the speed of discharging cleaning liquid.

In one embodiment, the cleaning device further includes a cover plate 8. An opening is provided on a side of the enclosing structure 903 away from the water storage tank 901. The cover plate 8 covers the enclosing structure 903 and blocks the opening.

A side of the enclosing structure 903 away from the upper cover 9012 has an opening. When the flow rate of the cleaning liquid flowing out of the overflow structure 902 is relatively large, it may overflow from the opening before being discharged from the guide pipe 907. To this end, the cover plate 8 covers the enclosing structure 903 and blocks the opening. The cover plate 8 plays a role of retaining and blocking, such that the cleaning liquid flowing out of the overflow structure 902 flows in a directional manner into the guide pipe 907 and is discharged to the wet cleaning structure 904.

The enclosing structure 903 is sleeved around the overflow structure 902 and the guide pipe 907. An end of the enclosing structure 903 facing the overflow structure 902 is a large end, and an end of the enclosing structure 903 away from the overflow structure 902 is a small end. The shape of the cover plate 8 is adapted to the shape of the enclosing structure 903. The cover plate 8 also has a large end and a small end. Of course, in other embodiments, the shapes of the enclosing structure 903 and the cover plate 8 may also be adaptively adjusted.

In one embodiment, an accommodating space is formed between the cover plate 8, the water storage tank 901 and the enclosing structure 903, and the accommodating space is configured to accommodate the cleaning liquid flowing out of the overflow structure 902 and prevent the cleaning liquid from overflowing.

Specifically, a top wall of the accommodating space is the cover plate 8, a bottom wall of the accommodating space is the upper cover 9012, and a side wall of the accommodating space is the enclosing structure 903. That is, under the joint action of the cover plate 8, the upper cover 9012 and the enclosing structure 903, a relatively closed space is formed. The accommodating space serves to temporarily store the cleaning liquid flowing out of the overflow structure 902, and can also prevent the overflow discharge of the cleaning liquid.

In one embodiment, the enclosing structure 903 and the upper cover 9012 of the water storage tank 901 are of an integrally formed structure, which reduces the number of part assembly steps and reduces the production cost.

In one embodiment, the cover plate 8 is bonded to the enclosing structure 903 by a bonding member.

The bonding member may be a double-sided adhesive or adhesive liquid, such that the cover plate 8 is fixed to the enclosing structure 903 by bonding, which has a simple process and low production cost.

In one embodiment, one of the enclosing structure 903 and the cover plate 8 is provided with a mounting column, and the other of the enclosing structure 903 and the cover plate 8 is provided with a mounting hole 81, in which the mounting column passes through the mounting hole 81.

The mounting column may be a positioning pin, a fixing pin or a bolt, and the mounting column is inserted into the mounting hole 81 to achieve fixation between the enclosing structure 903 and the cover plate 8.

It can be understood that after the cover plate 8 is bonded to the enclosing structure 903 by means of double-sided adhesive, the mounting column is then inserted into the mounting hole 81 to achieve a double fixing effect, thereby further improving the fixing effect.

In one embodiment, the cover plate 8 is provided with a fixing hole 82, and a fixing member is inserted into the fixing hole 82 and the upper cover 9012 for fixing the cover plate 8 and the water storage tank 901.

The fixing member may be a positioning pin, a fixing pin or a bolt. When the cover plate 8 and the water storage tank 901 are installed, the fixing member is inserted into the fixing hole 82 and the upper cover 9012. At this time, the first end of the cover plate 8 is fixed to the enclosing structure 903 by the mounting column, and the second end of the cover plate 8 is fixed to the upper cover 9012 by the fixing member, so as to improve the connection stability and fixing effect of the cover plate 8.

In one embodiment, the cover plate 8 may be made of a hard material, such as PET material, which has good structural strength.

The operation process of the cleaning device provided in this embodiment is as follows:
If there is too much cleaning liquid in the water storage tank 901 and the hydraulic pressure inside the water storage tank 901 is too high, the cleaning liquid in the water storage tank 901 may be discharged through the overflow structure 902. Under the guidance of the guide slope 9013, the cleaning liquid overflowing from the overflow structure 902 passes through the guide slope 9013 and flows along the inner wall of the enclosing structure 903 to the guide pipe 907. The enclosing structure 903 and the cover plate 8 serve to block and retain the cleaning liquid. Then the cleaning liquid flows through the guide pipe 907 to the wet cleaning structure 904 to realize the function of auxiliary water supply to the wet cleaning structure 904.

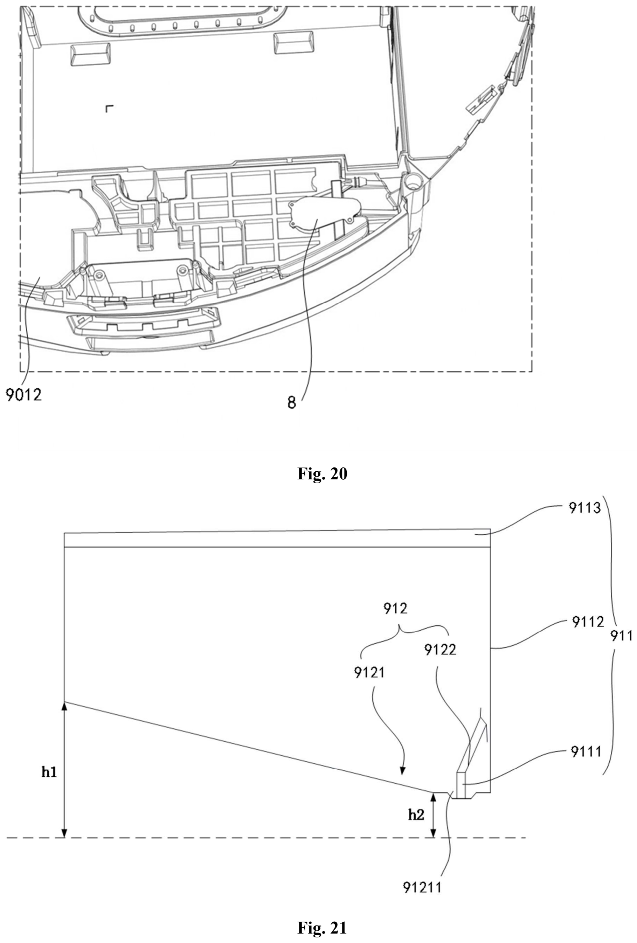

An embodiment of the present disclosure provides a liquid storage tank for cleaning devices, which is applicable to the cleaning devices. The cleaning device may be a cleaning device such as a cleaning robot or a mopping machine, or other types of cleaning device, such as a floor washing machine. As shown in FIG. 21, the liquid storage tank for the cleaning device includes a tank body 911 and a liquid discharging structure 912. The tank body 911 is used to store a cleaning liquid. The liquid discharging structure 912 is arranged in the tank body 911 and includes a liquid discharging bevel structure 9121 and a liquid discharging pipeline 9122. The first end of the liquid discharging pipeline 9122 is connected to the bottom end of the liquid discharging bevel structure 9121, and the second end of the liquid discharging pipeline 9122 is provided with a liquid discharging port. These components are configured such that the cleaning liquid in the tank body 911 is guided to the first end of the liquid discharging pipeline 9122 through the liquid discharging bevel structure 9121, and discharged out of the tank body 911 through the liquid discharging port of the liquid discharging pipeline 9122. The cleaning liquid may be water, water added with a detergent, or other liquids with cleaning ability.

It can be understood that the liquid discharging bevel structure 9121 includes a bevel and a bottom end that are connected to each other. The bevel is arranged inclined relative to a horizontal plane. The first end of the bevel away from the liquid discharging pipeline 9122 is the top end, and the second end of the bevel close to the liquid discharging pipeline 9122 is provided with the bottom end. The bottom end may be arranged parallel to the horizontal plane, and at this time, the bevel is inclined downward in a direction close to the liquid discharging pipeline 9122.

The liquid storage tank for cleaning devices according to this embodiment includes the tank body 911 for storing a cleaning liquid, the liquid discharging structure 912 is arranged in the tank body 911, and the cleaning liquid in the tank body 911 is discharged to the maximum extent by using the liquid discharging bevel structure 9121 of the liquid discharging structure 912. In actual use, the liquid discharging bevel structure 9121 is at least partially inclined relative to the horizontal plane, and the cleaning liquid in the tank body 911 flows from the liquid discharging bevel structure 9121 to the bottom end of the liquid discharging bevel structure 9121 under the action of its own gravity. The first end of the liquid discharging pipeline 9122 is connected to the bottom end of the liquid discharging bevel structure 9121, and the first end can directly receive the cleaning liquid at the bottom end of the liquid discharging bevel structure 9121, so as to facilitate the cleaning liquid to be discharged into the liquid discharging pipeline 9122. The second end of the liquid discharging pipeline 9122 is provided with the liquid discharging port used to discharge the cleaning liquid in the liquid discharging pipeline 9122 out of the tank body 911. In this way, with the cooperation between the liquid discharging bevel structure 9121 and the liquid discharging pipeline 9122, the cleaning liquid is discharged quickly and efficiently, which is conducive to the complete discharge of the cleaning liquid in the tank body 911.

In some embodiments, a water pump is connected to the second end of the liquid discharging pipeline 9122. Under the pumping action of the water pump, the cleaning liquid is sucked into the liquid discharging pipeline 9122 and then discharged out of the tank body 911 through the liquid discharging port.

In an embodiment, as shown in FIG. 21, the height of the first end of the liquid discharging bevel structure 9121 away from the liquid discharging pipeline 9122 is h1, and the height of the second end of the liquid discharging bevel structure 9121 close to the liquid discharging pipeline 9122 is h2; wherein h1 > h2.

It can be understood that, if the heights of both ends of the liquid discharging bevel structure 9121 are approximately the same and the liquid discharging bevel structure 9121 is approximately a horizontal structure, the cleaning liquid in the tank body 911 will flow dispersedly on the liquid discharging bevel structure 9121, making it difficult to empty and discharge completely the cleaning liquid in the tank body 911.

Therefore, according to this embodiment, the height h1 of the first end of the liquid discharging bevel structure 9121 away from the liquid discharging pipeline 9122 is greater than the height h2 of the second end of the liquid discharging bevel structure 9121 close to the liquid discharging pipeline 9122.

The bevel of the liquid discharging bevel structure 9121 acts as a slide. Under the liquid leading effect of the bevel, the cleaning liquid in the tank body 911 can flow from the top end at a relatively higher height to the bottom end at a relatively lower height, such that the cleaning liquid in the tank body 911 can eventually be gathered at the bottom end of the liquid discharging bevel structure 9121, thereby realizing the function of gathering the cleaning liquid in the tank body 911 in the direction close to the liquid discharging pipeline 9122 to discharge the cleaning liquid in the tank body 911 smoothly and completely.

In an embodiment, h1-h2 > 1 mm.

If h1-h2 ≤ 1mm, it means that the height difference between the first end of the liquid discharging bevel structure 9121 away from the liquid discharging pipeline 9122 and the second end of the liquid discharging bevel structure 9121 close to the liquid discharging pipeline 9122 is relatively small. In this case, the heights of the two ends of the liquid discharging bevel structure 9121 may be regarded as approximately the same, thus it is difficult to achieve the liquid leading effect on the cleaning liquid in the tank body 911. At the same time, the cleaning liquid in the tank body 911 will be dispersed and spread on the liquid discharging bevel structure 9121, making it difficult to empty and discharge completely the cleaning liquid in the tank body 911. To this end, according to this embodiment, the height difference between the first end of the liquid discharging bevel structure 9121 away from the liquid discharging pipeline 9122 and the second end of the liquid discharging bevel structure 9121 close to the liquid discharging pipeline 9122 is greater than 1 mm, such that there is an obvious difference in height between the two ends of the liquid discharging bevel structure 9121, so as to guide the cleaning liquid in the tank body 911. At the same time, the cleaning liquid in the tank body 911 will gather at the second end of the liquid discharging bevel structure 9121 close to the liquid discharging pipeline 9122, such that the cleaning liquid in the tank body 911 can be drained as much as possible.

In an embodiment, as shown in FIG. 21, the liquid discharging bevel structure 9121 is disposed on the inner wall of the tank body 911.

The liquid discharging bevel structure 9121 and the tank body 911 may be configured in a split structure. Alternatively, the inner wall of the top wall or the bottom wall of the tank body 911 itself may be inclined, and the inclined inner wall then forms the liquid discharging bevel structure 9121. The leading of the cleaning liquid in the tank body 911 can be achieved without adding an additional inclined guide plate in the tank body 911, thereby reducing the number of parts, and saving production and processing costs.

In an embodiment, a liquid discharging groove 91211 is further provided at the bottom end of the liquid discharging bevel structure 9121, and the first end of the liquid discharging pipeline 9122 is connected to the bottom end of the liquid discharging bevel structure 9121 through the liquid discharging groove 91211. The liquid discharging pipeline 9122 has a liquid discharging hole communicated with the liquid discharging groove 91211, such that the cleaning liquid in the liquid discharging groove 91211 can enter the liquid discharging pipeline 9122 through the liquid discharging hole and then be discharged out of the tank body 911.

Specifically, the liquid discharging groove 91211 is provided at approximately the middle of the bottom end of the liquid discharging bevel structure 9121, and the first end of the liquid discharging pipeline 9122 is connected to the bottom end of the liquid discharging bevel structure 9121 through the liquid discharging groove 91211. The liquid discharging groove 91211 plays the role of intermediate connection, thereby improving the reliable connection between the liquid discharging pipeline 9122 and the bottom end of the liquid discharging bevel structure 9121. The liquid discharging pipeline 9122 is communicated with to the liquid discharging groove 91211, which is equivalent to setting the position of the liquid discharging pipeline 9122 for sucking the cleaning liquid within the liquid discharging groove 91211 with a groove structure. After the cleaning liquid in the tank body 911 is guided to the liquid discharging groove 91211 through the bevel of the liquid discharging bevel structure 9121, the liquid discharging groove 91211 provides a certain accommodation space for the cleaning liquid, which is convenient for the liquid discharging pipeline 9122 to suck and discharge the cleaning liquid.

In an embodiment, the bottom height of the liquid discharging groove 91211 is lower than the height of the bottom end of the liquid discharging bevel structure 9121, and the cleaning liquid in the tank body 911 may be guided into the liquid discharging groove 91211 through the liquid discharging bevel structure 9121.

Since the bottom height of the liquid discharging groove 91211 is lower than the height of the bottom end of the liquid discharging bevel structure 9121, the bottom of the liquid discharging groove 91211 is actually at the lowest position, and the liquid discharging groove 91211 further plays a role of liquid leading and confluence. Even when the total amount of cleaning liquid in the tank body 911 is relatively small, the cleaning liquid in the tank body 911 can flow to the bottom of the liquid discharging groove 91211 to the greatest extent, such that the liquid discharging pipeline 9122 can suck and discharge the cleaning liquid.

In some embodiments, the side walls of the liquid discharging groove 91211 are inclined relative to the bottom wall of the liquid discharging groove 91211, such that the side walls of the liquid discharging groove 91211 can also play a role in liquid leading and guidance.

In some embodiments, the bottom end of the liquid discharging bevel structure 9121 and the liquid discharging groove 91211 are integrally formed, such that there is no gap between the bottom end of the liquid discharging bevel structure 9121 and the liquid discharging groove 91211, thereby reducing the risk of leakage. Specifically, in actual processing and production, the inner wall of the tank body 911 may be processed into the liquid discharging bevel structure 9121 that is at least partially inclined relative to a horizontal plane, and a groove is processed at the position of the inner wall of the tank body 911 corresponding to the liquid discharging pipeline 9122 to form the liquid discharging groove 91211.

In an embodiment, as shown in FIG. 21, a liquid discharging joint 11 is disposed at the bottom of the liquid discharging groove 91211. The liquid discharging joint 11 is connected to the first end of the liquid discharging pipeline 9122, and a communication hole is disposed on the outer peripheral wall of the liquid discharging joint 11.

By providing the liquid discharging joint 11 at the bottom of the groove for discharging the cleaning liquid and connecting the liquid discharging joint 11 to the first end of the liquid discharging pipeline 9122, the liquid discharging joint 11 serves to fix the liquid discharging pipeline 9122 to the liquid discharging groove 91211, thereby preventing the liquid discharging pipeline 9122 from being displaced due to violent shaking, and improving the position stability of the liquid discharging pipeline 9122. A communication hole is provided on the outer peripheral wall of the liquid discharging joint 11 to serve as intermediate communication, such that the cleaning liquid in the liquid discharging groove 91211 flows into the liquid discharging pipeline 9122 through the communication hole, thereby realizing the cleaning liquid delivery and discharge process.

In this way, it can also achieve the communication effect between the liquid discharging groove 91211 and the liquid discharging pipeline 9122, while improving the connection reliability between the liquid discharging pipeline 9122 and the liquid discharging joint 11, thereby improving the connection stability of the liquid discharging pipeline 9122 to achieve the purpose of discharge of the cleaning liquid.

In some embodiments, the liquid discharging joint 11 may be disposed approximately in the middle of the liquid discharging groove 91211, that is, the liquid discharging groove 91211 is disposed around the liquid discharging joint 11. At this time, the side wall of the side of the liquid discharging groove 91211 close to the liquid discharging bevel structure 9121 guides the cleaning liquid on the liquid discharging bevel structure 9121 to the liquid discharging groove 91211, and the side wall of the side of the liquid discharging groove 91211 away from the liquid discharging bevel structure 9121 acts as a baffle to reduce the overflow of the cleaning liquid from the liquid discharging groove 91211.

In some other embodiments, if the liquid discharging groove 91211 is not provided at the bottom end of the liquid discharging bevel structure 9121, the bottom end of the liquid discharging bevel structure 9121 is configured as a relatively small plane on which the liquid discharging pipeline 9122 is provided, and this plane is connected to the bevel, so as to also solve the problem of partial guidance for liquid discharge.

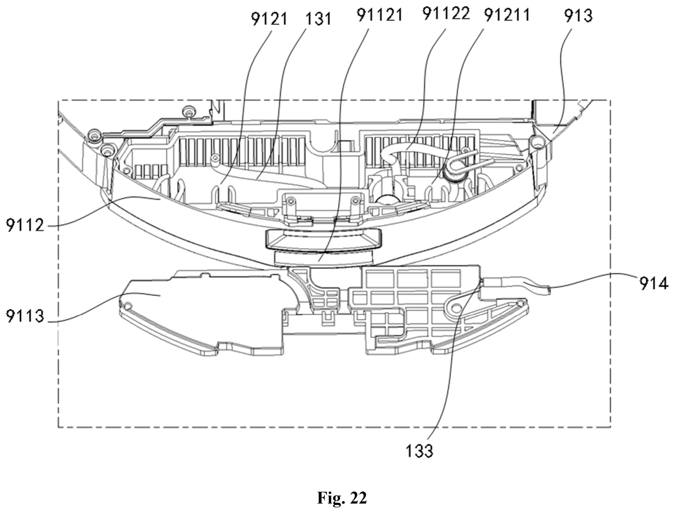

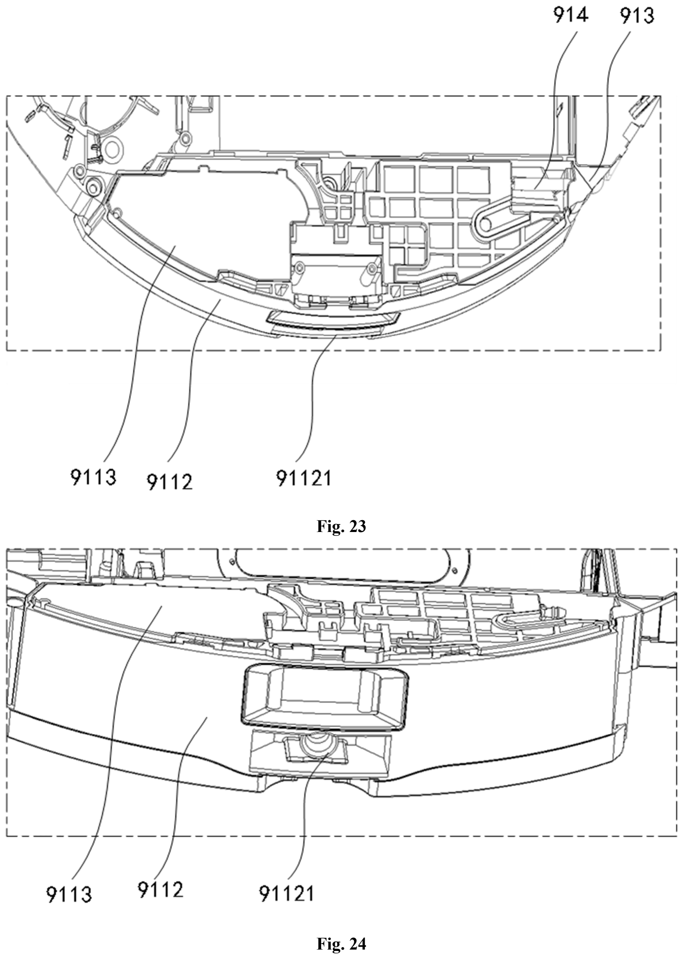

In an embodiment, as shown in FIGS. 22-24, the tank body 911 includes a bottom shell 9112 and an upper cover 9113, the bottom shell 9112 is provided with a water flow channel 91121 and an opening end on which the upper cover 9113 is covered.

The bottom shell 9112 has a shell structure, and a cavity inside the bottom shell 9112 provides the accommodation space for the cleaning liquid. The upper cover 9113 is covered on the opening end of the bottom shell 9112, such that the upper cover 9113 plays a role of sealing the opening end of the bottom shell 9112 to prevent the cleaning liquid from leaking from the bottom shell 9112.

In some embodiments, the bottom shell 9112 and the upper cover 9113 may be integrally formed, and the bottom shell 9112 and the upper cover 9113 may be connected by an ultrasonic process. This embodiment does not limit the method for fixing the bottom shell 9112 and the upper cover 9113, as long as the sealing effect between the bottom shell 9112 and the upper cover 9113 can be guaranteed to prevent leakage.

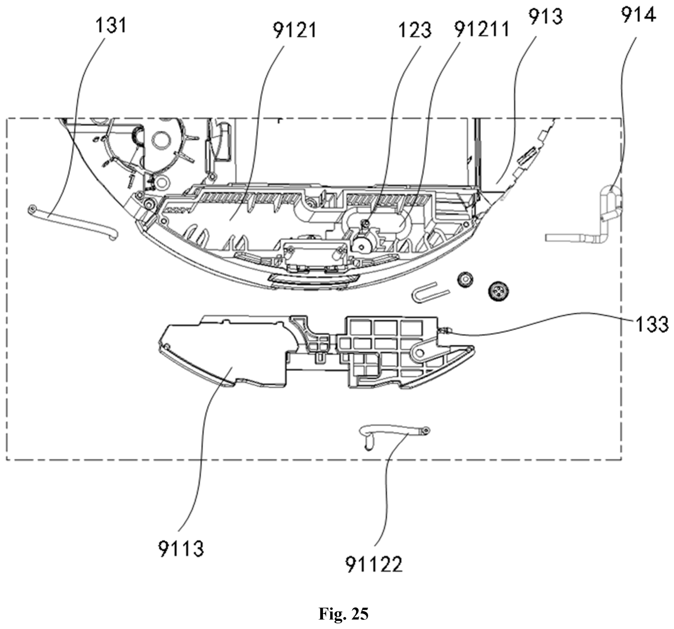

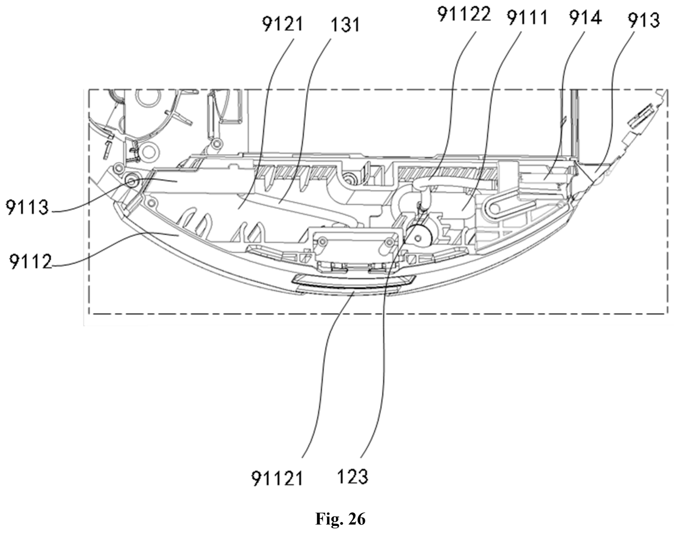

During normal use of the cleaning device, the effectiveness of the liquid storage tank directly affects the efficiency of providing cleaning liquid. If the liquid storage tank has a low liquid supply efficiency, the effect of mopping the floor will be directly affected. To this end, as shown in FIGS. 25 and 26, the liquid discharging bevel structure 9121 according to this embodiment is arranged on the bottom wall of the bottom shell 9112, and the cleaning liquid in the tank body 911 is discharged through the liquid discharging pipeline 9122 connected to the bottom wall of the bottom shell 9112 when the tank body 911 is in an upright state. The normal use refers to that the cleaning device performs a cleaning task.

It can be understood that when the cleaning device is in normal use, the tank body 911 is used to provide cleaning liquid for other structures, such as a wet cleaning structure 913. At this time, the tank body 911 is in an upright state, that is, the bottom shell 9112 is located below the upper cover 9113, and the upper cover 9113 is located above the bottom shell 9112. The cavity inside the bottom shell 9112 provides an accommodation space for the liquid discharging pipeline 9122. The bottom shell 9112 not only connects the liquid discharging pipeline 9122, but also discharges the cleaning liquid in the bottom shell 9112 through the liquid discharging pipeline 9122 for use by the wet cleaning structure 913 and other structures.

As shown in FIGS. 25 and 26, a liquid outlet pipe 91122 is provided in the bottom shell 9112, and a liquid outlet joint 123 is provided at the bottom of the liquid discharging groove 91211. When the tank body 911 is in an upright state, the liquid outlet pipe 91122 is the above-mentioned liquid discharging pipeline 9122, and the liquid outlet joint 123 is the above-mentioned liquid discharging joint 11. A communication hole is provided in the liquid outlet joint 123.

Specifically, the liquid discharging bevel structure 9121 is provided on the bottom wall of the bottom shell 9112, and the height difference between the first end of the liquid discharging bevel structure 9121 away from the liquid outlet pipe 91122 and the second end of the liquid discharging bevel structure 9121 close to the liquid outlet pipe 91122 is greater than 1 mm, such that there is a significant difference in height between the two ends of the liquid discharging bevel structure 9121, which plays a role of leading the cleaning liquid in the tank body 911. The liquid discharging groove 91211 is provided at the bottom end of the liquid discharging bevel structure 9121, and the bottom height of the liquid discharging groove 91211 is lower than the height of the bottom end of the liquid discharging bevel structure 9121. At this time, the bottom of the liquid discharging groove 91211 is the lowest position of the tank body 911 in the upright state, such that the cleaning liquid in the tank body 911 is guided to the liquid discharging groove 91211 through the liquid discharging bevel structure 9121, and the liquid discharging groove 91211 realizes the confluence of the cleaning liquid.

At this time, the liquid outlet joint 123 is at the position where the liquid outlet pipe 91122 sucks the cleaning liquid, such that the cleaning liquid in the liquid discharging groove 91211 enters the liquid outlet joint 123 through the communication hole of the liquid outlet joint 123, and is discharged through the liquid outlet pipe 91122, thereby realizing that the cleaning liquid in the tank body 911 can be discharged to the maximum extent when the tank body 911 is in an upright state during normal use.

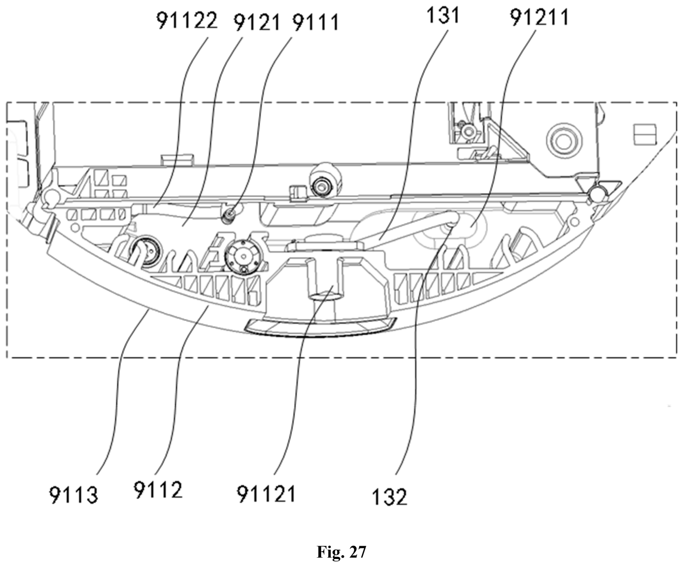

When the cleaning device fails and needs to be repaired and maintained, if the cleaning liquid remaining in the liquid storage tank cannot be completely discharged, it will cause certain difficulties in returning to the factory for repair. To this end, as shown in FIGS. 25 and 27, the liquid discharging bevel structure 9121 according to this embodiment is arranged on the inner wall of the upper cover 9113, and the second end of the liquid discharging pipeline 9122 is communicated with the water flow channel 91121. The cleaning liquid in the tank body 911 is discharged through the liquid discharging pipeline 9122 and the water flow channel 91121 when the tank body 911 is in an inverted state.

It can be understood that when the cleaning device is returned to the factory for repair, the cleaning liquid remaining in the tank body 911 needs to be discharged. At this time, the tank body 911 may be in an inverted state, that is, the upper cover 9113 is located below the bottom shell 9112, and the bottom shell 9112 is located above the upper cover 9113. The cavity formed by enclosure of the upper cover 9113 and the bottom shell 9112 provides an accommodation space for the liquid discharging pipeline 9122. The upper cover 9113 is connected to the liquid discharging pipeline 9122, and the cleaning liquid remaining in the tank body 911 may be discharged through the liquid discharging pipeline 9122.

The upper cover 9113 is connected to a liquid inlet pipe 131, and a liquid inlet joint 132 is provided at the bottom of the liquid discharging groove 91211. When the tank body 911 is in an inverted state, the liquid inlet pipe 131 is the above-mentioned liquid discharging pipeline 9122, and the liquid inlet joint 132 is the above-mentioned liquid discharging joint 11. A communication hole is provided in the liquid inlet joint 132.

Specifically, a liquid discharging bevel structure 9121 is provided on the inner wall of the upper cover 9113. When the tank body 911 is in an inverted state, the height difference between the first end of the liquid discharging bevel structure 9121 away from the liquid inlet pipe 131 and the second end of the liquid discharging bevel structure 9121 close to the liquid inlet pipe 131 is greater than 1 mm, such that there is a significant difference in height between the two ends of the liquid discharging bevel structure 9121, which plays a role of leading the cleaning liquid in the tank body 911. The liquid discharging groove 91211 is provided at the bottom end of the liquid discharging bevel structure 9121, and the bottom height of the liquid discharging groove 91211 is lower than the height of the bottom end of the liquid discharging bevel structure 9121. At this time, the bottom of the liquid discharging groove 91211 is the lowest position when the tank body 911 is in an inverted state, such that the cleaning liquid in the tank body 911 is guided to the liquid discharging groove 91211 through the liquid discharging bevel structure 9121, and the liquid discharging groove 91211 realizes the confluence of the cleaning liquid.

At this time, the liquid inlet joint 132 is at the position where the liquid inlet pipe 131 sucks the cleaning liquid, such that the cleaning liquid in the liquid discharging groove 91211 flows into the liquid inlet joint 132 through the communication hole of the liquid inlet joint 132, is discharged through the liquid inlet pipe 131, and is finally sucked out and discharged through the water flow channel 91121, thereby realizing that the cleaning liquid remaining in the tank body 911 can be discharged to the maximum extent when the tank body 911 is in an inverted state.

In an embodiment, as shown in FIGS. 25-27, the bottom wall of the bottom shell 9112 and the inner wall of the upper cover 9113 are both provided with the liquid discharging bevel structures 9121, and the cleaning liquid in the tank body 911 is discharged through the liquid discharging pipeline 9122 connected to the bottom wall of the bottom shell 9112 when the tank body 911 is in an upright state, while the cleaning liquid in the tank body 911 is discharged through the liquid discharging pipeline 9122 connected to the inner wall of the upper cover 9113 and through the water flow channel 91121 when the tank body 911 is in an inverted state.

With this arrangement, the tank body 911 can be emptied of cleaning liquid when it is in an upright state for normal use or when it is in an inverted state for repair. The liquid discharging bevel structures 9121 are provided on both the bottom wall of the bottom shell 9112 and the inner wall of the upper cover 9113, such that the liquid discharging bevel structures 9121 are utilized to discharge the cleaning liquid remaining in the tank body 911 to the maximum extent, thereby improving the efficiency of discharge of the cleaning liquid and facilitating after-sales maintenance.

In some embodiments, when the cleaning device is in a working state for performing a cleaning task, the cleaning device is in an upright state, and the cleaning base station provides cleaning liquid to the liquid storage tank through the water flow channel 91121 of the bottom shell 9112. The cleaning liquid flowing in through the water flow channel 91121 enters the liquid inlet pipe 131 and flows into the tank body 911 through the communication hole provided in the liquid inlet joint 132. At this time, the cleaning liquid falls from the upper cover 9113 to the bottom shell 9112, and the communication hole of the liquid inlet joint 132 serves to allow the liquid in the liquid inlet pipe 131 to flow into the water tank 1, such that the wet cleaning structure 913 can implement a mopping process.

In some embodiments, when the cleaning device is in a maintenance state, the cleaning device may be placed in an inverted state, with the upper cover 9113 located below the bottom shell 9112, and the cleaning liquid in the tank body 911 will be leaded to the liquid discharging groove 91211 through the liquid discharging bevel structure 9121 provided on the inner wall of the upper cover 9113. Under the suction action of external force, the cleaning liquid enters the liquid inlet pipe 131 through the communication hole of the liquid inlet joint 132, and is then sucked out of the tank body 911 through the water flow channel 91121.

It should be noted that, the reason of placing the cleaning device, i.e., the tank body 911, in an inverted state to discharge the cleaning liquid is mainly that, the speed of discharging cleaning liquid of the tank body 911 placed in an upright state in a manner of performing a cleaning task is slower, while placing the tank body 911 in an inverted state to discharge the cleaning liquid out of the tank body 911 through the water flow channel 91121 can fully utilize the large suction characteristics of the cleaning base station to speed up the discharge of the cleaning liquid. It can be understood that, it does not mean that the tank body 911 can only be repaired in an inverted state, but means that the tank body 911 in an inverted state is more conducive to the discharge of the cleaning liquid.

It can be understood that the cleaning liquid flow paths and achieved main functions of the liquid inlet pipe 131 and the communication hole are different when the tank body 911 is in the upright state and the inverted state. When the tank body 911 is in the upright state during normal use, the liquid inlet pipe 131 and the communication hole mainly play the role of filling the cleaning liquid supplied by the cleaning base station into the tank body 911, while when the tank body 911 is in the inverted state, the liquid inlet pipe 131 and the communication hole mainly play the role of discharging the cleaning liquid in the tank body 911 out of the tank body 911, which essentially plays the role of liquid discharge.

This embodiment further provides a cleaning device, which includes a liquid storage tank for the cleaning device. The cleaning device is specifically a cleaning robot with a wet cleaning function.

The cleaning device according to this embodiment includes the tank body 911 for storing cleaning liquid, the liquid discharging structure 912 is arranged in the tank body 911, and the cleaning liquid in the tank body 911 is discharged to the maximum extent by using the liquid discharging bevel structure 9121 of the liquid discharging structure 912. In actual use, the liquid discharging bevel structure 9121 is at least partially inclined relative to the horizontal plane, and the cleaning liquid in the tank body 911 flows from the liquid discharging bevel structure 9121 to the bottom end of the liquid discharging bevel structure 9121 under the action of its own gravity. The first end of the liquid discharging pipeline 9122 is connected to the bottom end of the liquid discharging bevel structure 9121, and the first end can directly receive the cleaning liquid at the bottom end of the liquid discharging bevel structure 9121, so as to facilitate the cleaning liquid to be discharged into the liquid discharging pipeline 9122; and the second end of the liquid discharging pipeline 9122 is provided with the liquid discharging port used to discharge the cleaning liquid in the liquid discharging pipeline 9122 out of the tank body 911. In this way, with the cooperation between the liquid discharging bevel structure 9121 and the liquid discharging pipeline 9122, the cleaning liquid is discharged quickly and efficiently, which is conducive to the complete discharge of the cleaning liquid in the tank body 911.

In an embodiment, as shown in FIG. 25 to FIG. 27, the cleaning device further includes the wet cleaning structure 913, and the liquid storage tank is used to provide cleaning liquid to the wet cleaning structure 913.

The wet cleaning structure 913 may be a mop structure or a rolling brush structure, and the mop structure or the rolling brush structure has a water-absorbing material, such as cotton material. The mop structure can be driven to reciprocate or rotate, or the rolling brush structure can be driven to roll, such that the floor to be cleaned can be subjected to wet cleaning. The liquid storage tank can provide cleaning liquid to the wet cleaning structure 913, such that the wet cleaning structure 913 can realize the function of wet mopping.

In an embodiment, the cleaning device further includes a delivery pipeline 914, a first end of the delivery pipeline 914 is communicated with the liquid discharging port of the liquid discharging pipeline 9122, and a second end of the delivery pipeline 914 is connected to the wet cleaning structure 913.

By communicating the first end of the delivery pipeline 914 with the liquid discharging port of the liquid discharging pipeline 9122 and connecting the second end of the delivery pipeline 914 to the wet cleaning structure 913, the delivery pipeline 914 serves as middle communication between the liquid discharging pipeline 9122 and the wet cleaning structure 913, such that the cleaning liquid flowing out of the liquid discharging pipeline 9122 flows to the wet cleaning structure 913 through the delivery pipeline 914, thereby completing the process of outputting the cleaning liquid in the tank body 911 to achieve the purpose of supplying cleaning liquid to the wet cleaning structure 913.

In some embodiments, a delivery joint 133 is provided on one side of the upper cover 9113 facing the bottom shell 9112, a first end of the delivery joint 133 is connected to the liquid discharging pipeline 9122 (i.e., the liquid outlet pipe 91122), and a second end of the delivery joint 133 is connected to the delivery pipeline 914. The delivery joint 133 achieves communication between the delivery pipeline 914 and the liquid discharging pipeline 9122 while ensuring that the relative positions of the delivery pipeline 914 and the liquid discharging pipeline 9122 are fixed.

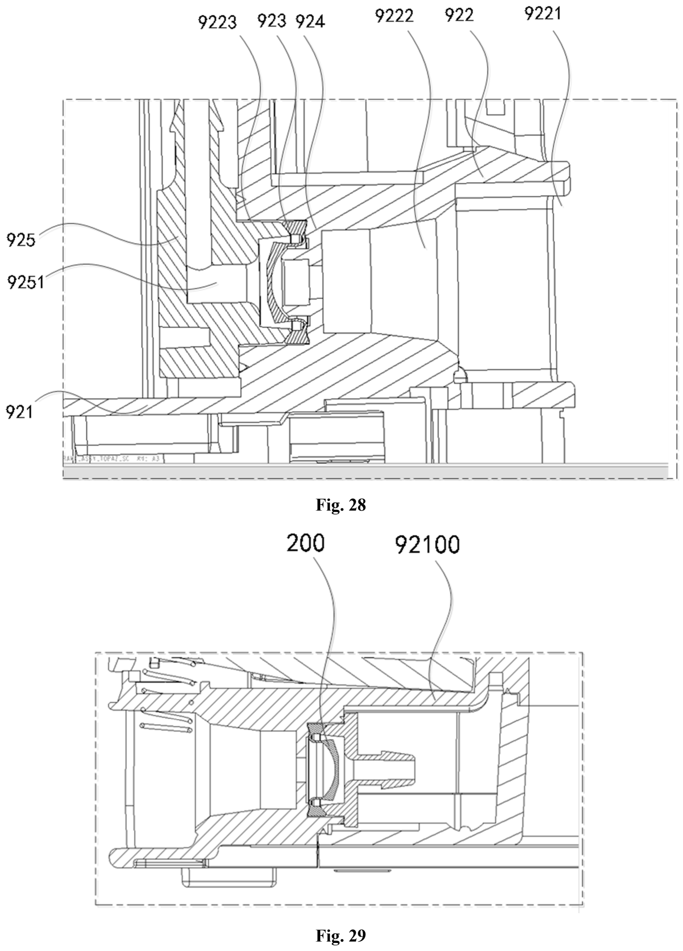

This embodiment provides a water tank of the cleaning device, which is applicable to cleaning devices. The cleaning device specifically refers to a cleaning device such as a cleaning robot or a mopping machine, or other types of cleaning device, such as a floor washing machine. As shown in FIG. 28, the water tank of the cleaning device includes a tank body 921, a liquid inlet pipe 922 and a water inlet valve 923. The liquid inlet pipe 922 is disposed on the tank body 921, a liquid inlet 9221 is disposed at the first end of the liquid inlet pipe 922, the water inlet valve 923 is disposed inside the second end of the liquid inlet pipe 922, and the liquid inlet pipe 922 can be communicated with the inner cavity of the tank body 921 through the water inlet valve 923. Specifically, by opening and closing the water inlet valve 923, the liquid inlet pipe 922 can be selectively communicated with the inner cavity of the tank body 921, and the inner cavity of the tank body 921 is used to store cleaning liquid, which may be water, or water added with a detergent, or other liquids with cleaning ability.

The water tank of the cleaning device according to this embodiment has the liquid inlet pipe 922 arranged in the tank body 921. The tank body 921 plays a role of supporting the liquid inlet pipe 922 as a whole. The liquid inlet 9221 is arranged at the first end of the liquid inlet pipe 922, and the water inlet valve 923 is arranged at the second end of the liquid inlet pipe 922, such that external cleaning liquid may enter the liquid inlet pipe 922 through the liquid inlet 9221. The liquid inlet pipe 922 can be communicated with the inner cavity of the tank body 921 through the water inlet valve 923 used to open and close the liquid inlet pipe 922. When cleaning liquid needs to be added to the tank body 921, the water inlet valve 923 is opened, and the cleaning liquid flowing in through the liquid inlet 9221 flows into the inner cavity of the tank body 921 through the liquid inlet pipe 922 and the water inlet valve 923. The tank body 921 provides an accommodation space for the cleaning liquid to provide the required water for cleaning structures such as the mop. When it is necessary to stop adding cleaning liquid to the tank body 921, the water inlet valve 923 is closed to play a role of cutting off the liquid inlet pipe 922, thereby reducing the risk of the cleaning liquid in the tank body 921 flowing back to the outside of the tank body 921 to a certain extent.

It should be noted that the cleaning base station is provided outside the water tank of the cleaning device, and the cleaning base station provides cleaning liquid to the inner cavity of the tank body 921 through the liquid inlet 9221. A water storage bucket is provided inside the cleaning base station, and the water storage bucket is detachably connected to the cleaning base station. The user can remove the water storage bucket from the cleaning base station and fill the water storage bucket with cleaning liquid, and then put the water storage bucket into the cleaning base station. The water storage bucket provides the cleaning device water tank with the cleaning liquid required for mopping the floor. In addition, the cleaning base station may also be directly connected to a tap water pipe to supply water to the above-mentioned water storage bucket through the tap water pipe.

In the related art, a water inlet nozzle 92100 is equipped with a water inlet valve 200 (as shown in FIG. 29). When the water tank is filled with water, the water pressure in the water tank is relatively high. If the material of the water inlet valve 200 is relatively soft, the water inlet valve 200 will deform in the direction away from the water tank, thereby causing water leakage in the water tank.

In order to solve this problem, as shown in FIG. 28, the water tank of the cleaning device according to this embodiment further includes a support structure 924 disposed in the liquid inlet pipe 922 for supporting the water inlet valve 923.

In the water tank of the cleaning device according to this embodiment, the support structure 924 is disposed in the liquid inlet pipe 922 which provides an installation position for the support structure 924. The support structure 924 is used to support the water inlet valve 923 and is used to resist the deformation force of the water inlet valve 923 toward the outside of the tank body 921, so as to prevent the water inlet valve 923 from generating an amount of deformation in a direction away from the tank body 921, thereby reducing the risk of liquid leakage from the tank body 921 for a better user's experience.

In an embodiment, the water inlet valve 923 is a one-way valve, which allows the cleaning liquid flowing in through the liquid inlet 9221 to flow into the inner cavity of the tank body 921 in a one-way manner.

The water inlet valve 923 may be a one-way valve, which serves to limit the flow direction of the cleaning liquid. The cleaning liquid flowing out of the cleaning base station flows into the inner cavity of the tank body 921 through the water inlet valve 923, while the cleaning liquid in the inner cavity of the tank body 921 cannot flow back into the cleaning base station through the water inlet valve 923, which prevents the cleaning liquid in the water tank of the cleaning device from flowing back to the outside of the water tank to a certain extent, thereby reducing the risk of liquid leakage in the water tank of the cleaning device.

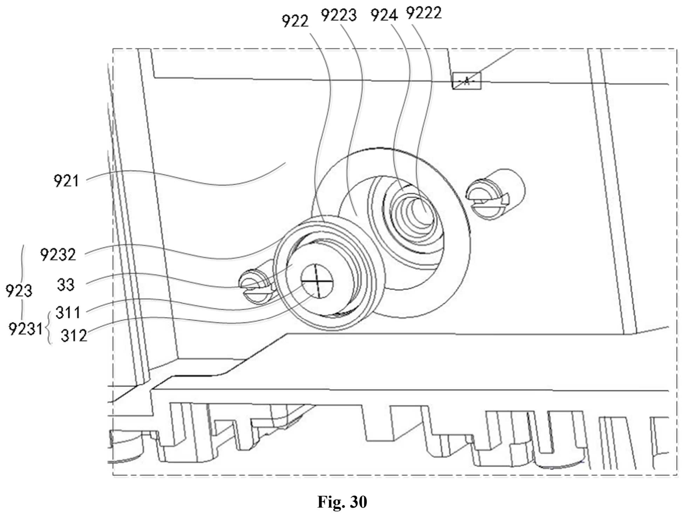

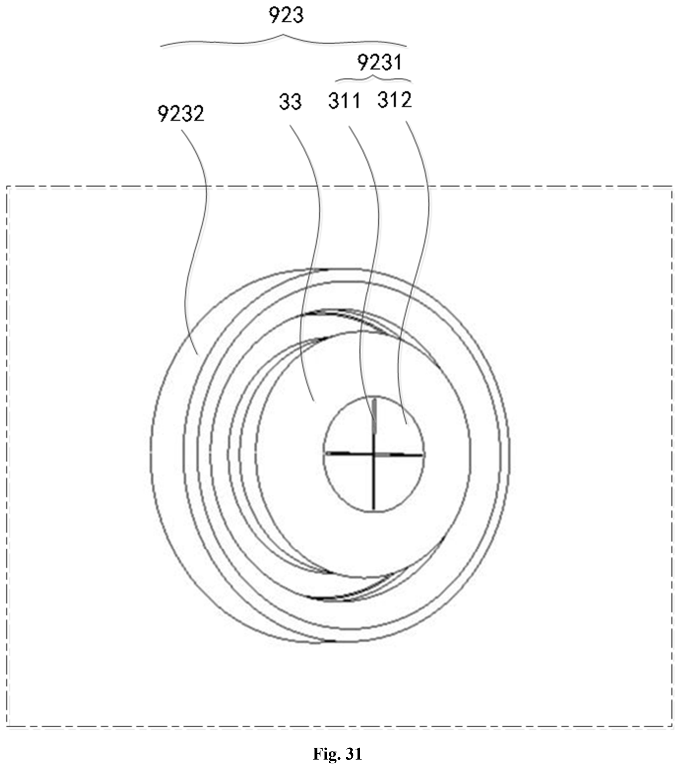

In an embodiment, as shown in FIG. 30 to FIG. 31, the shape of the water inlet valve 923 is similar to a cylindrical structure. The water inlet valve 923 specifically includes a central portion 9231, a fixing portion 9232 and an annular portion 33. The central portion 9231 is provided with a valve port, and the valve port is configured to be selectively opened and closed. The fixing portion 9232 is arranged around the central portion 9231, and the annular portion 33 is arranged between the central portion 9231 and the fixing portion 9232.

The fixing portion 9232 is arranged in the liquid inlet pipe 922 and is arranged around the central portion 9231. The fixing portion 9232 not only plays a role of protecting the central portion 9231, but also plays a role of installing and fixing the water inlet valve 923. The central portion 9231 is provided with a valve port, which is configured to be selectively opened and closed and can open or close the flow of the cleaning liquid in the liquid inlet pipe 922. The annular portion 33 is arranged between the central portion 9231 and the fixing portion 9232 and plays a role of intermediate connection between the central portion 9231 and the fixing portion 9232 to form an integral structure.

Specifically, the structure of the central portion 9231 is similar to a disc structure, and a gap 311 is provided in the central portion 9231. The gap 311 has a cross-shaped structure to separate the central portion 9231 into four single bodies 312. When no cleaning liquid passes through the gap 311, the structure of the central portion 9231 is similar to a closed structure, which means that the valve port is in a closed state. When the cleaning liquid of the external cleaning base station flows into the water inlet valve 923 through the liquid inlet pipe 922, there is a pressure difference between both ends of the liquid inlet pipe 922, and the cleaning liquid can break through the gap 311, such that the four single bodies 312 expand to form a through hole for the cleaning liquid to pass through. At this time, the annular portion 33 plays a role of connecting the single bodies 312, and the valve port is in an open state, such that the cleaning liquid in the cleaning base station can be delivered to the inner cavity of the tank body 921 through the valve port.

It can be understood that since the gap 311 has a cross-shaped structure, the water inlet valve 923 may also be called a cross valve. The present embodiment does not limit the shape structure of the gap 311, and the gap 311 may also in a shape of the Chinese character " 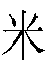 ", etc., such structure is within the protection scope of the present embodiment, as long as it can be achieved that a through hole can be formed between the single bodies 312 when water with a large pressure impacts the gap 311.

It should be noted that since the cleaning liquid flowing out of the cleaning base station has a large water pressure, it can impact the gap 311 to expand the four single bodies 312. However, the cleaning liquid in the tank body 921 generally does not have a large pressure, and the cleaning liquid in the tank body 921 cannot pass through the gap 311 to expand the single bodies 312. At the same time, the water inlet valve 923 is generally made of silicone material. When no cleaning liquid passes through the water inlet valve 923, the tightness between the four single bodies 312 is relatively good, which reduces the risk of liquid leakage to a certain extent.

It can be understood that when the tank body 921 is filled with cleaning liquid, the water pressure in the tank body 921 is relatively large. Since the water inlet valve 923 is made of silicone material which is relatively soft, the annular portion 33 of the water inlet valve 923 is sunken in the direction away from the tank body 921 and deformed, under the action of the large water pressure in the tank body 921. That is, the annular portion 33 is turned outward. As the annular portion 33 is turned outward, the single bodies 312 are caused to move in the direction away from the tank body 921, resulting in the four single bodies 312 no longer being tightly fitted but forming a through hole for the cleaning liquid to pass through. In this case, a small amount of cleaning liquid in the tank body 921 will flow reversely out of the tank body 921 through the water inlet valve 923.

In order to solve this problem, as shown in FIGS. 28 and 30, the support structure 924 supports the side of the annular portion 33 facing the liquid inlet 9221.

When the annular portion 33 has a tendency to turn outward, the support structure 924 is used to resist the force of the annular portion 33 turning outward since the support structure 924 supports the side of the annular portion 33 toward the liquid inlet 9221, which is equivalent to that the support structure 924 occupies the space for the annular portion 33 to turn outward and supports the annular portion 33, thereby reducing the risk of the annular portion 33 turning outward, avoiding liquid leakage of the tank body 921, so as to achieve the purpose of improving the user experience.

It can be understood that since the shape of the water inlet valve 923 is similar to a cylindrical structure, the central portion 9231 has an axial direction and a radial direction. The central portion 9231 and the support structure 924 are coaxially arranged, and the support structure 924 can also limit the outer side of the annular portion 33 along the axial direction of the central portion 9231.

In an embodiment, as shown in FIG. 28 and FIG. 30, along the radial direction of the central portion 9231, the inner wall of the liquid inlet pipe 922 and the support structure 924 are clamped on two sides of the fixing portion 9232, respectively.

It can be understood that the support structure 924 is located on the inner side of the water inlet valve 923, and is clamped on two sides of the fixing portion 9232 by using the inner wall of the liquid inlet pipe 922 and the support structure 924. That is, a clamping structure is added at the position of the water inlet valve 923. Under the joint clamping action of the liquid inlet pipe 922 and the support structure 924, the position stability of the water inlet valve 923 along the radial direction of the central portion 9231 is improved.

In an embodiment, as shown in FIG. 28 and FIG. 30, a first flow channel 9222 is arranged in the liquid inlet pipe 922, the liquid inlet 9221 is disposed at a first end of the first flow channel 9222, and the water inlet valve 923 and the support structure 924 are disposed at a second end of the first flow channel 9222.

The first flow channel 9222 provides a transport path for the cleaning liquid flowing out of the cleaning base station, such that the cleaning liquid enters the first flow channel 9222 through the liquid inlet 9221, and is transferred to the water inlet valve 923 under the function of transport and guidance of the first flow channel 9222. Specifically, the structure of the first flow channel 9222 is similar to a conical structure, the large end of the conical structure is arranged toward the cleaning base station to facilitate connection with the water injection port of the cleaning base station, and the small end of the conical structure is arranged toward the water inlet valve 923 and corresponds to the size of the water inlet valve 923.

In an embodiment, a second flow channel 9223 is further provided outside the second end of the liquid inlet pipe 922, a first end of the second flow channel 9223 is communicated with the first flow channel 9222, and a second end of the second flow channel 9223 is communicated with the inner cavity of the tank body 921. The water inlet valve 923 and the support structure 924 are provided at a position where the first flow channel 9222 and the second flow channel 9223 are connected, and the support structure 924 is provided between the water inlet valve 923 and the first flow channel 9222.

It can be understood that the side of the liquid inlet pipe 922 corresponding to the first flow channel 9222 and close to the water inlet valve 923 provides an arrangement position for the support structure 924. By arranging the support structure 924 on the end face of the liquid inlet pipe 922 corresponding to the first flow channel 9222, the water inlet valve 923 can be supported. The arrangement structure is simple and the production cost is relatively low.

It can be understood that the support structure 924 is specifically a reinforcing rib, which plays a role of reinforcing the structure of the liquid inlet pipe 922, such that its structural strength is relatively good.

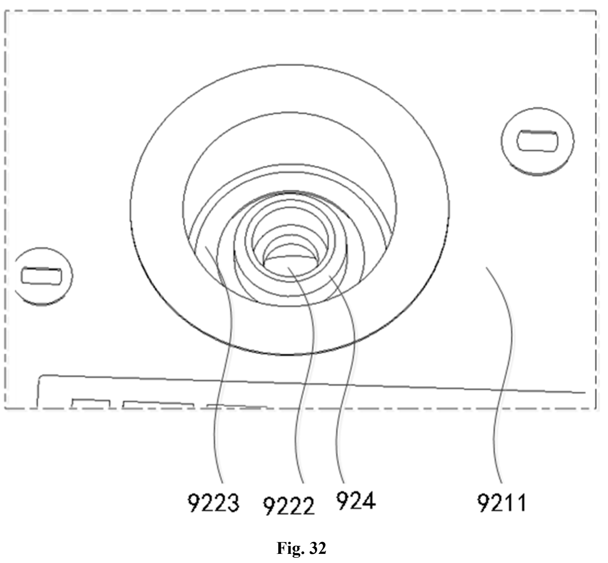

In an embodiment, as shown in FIGS. 28, 30 and 32, the support structure 924 is a support ring, a first end of the support ring is disposed opposite to and communicated with the first flow channel 9222, and a second end of the support ring abuts against the annular portion 33.

The central hole of the support ring is butted against the small end of the first flow channel 9222, such that the cleaning liquid flowing in through the first flow channel 9222 flows into the water inlet valve 923 through the support ring. The support ring plays a role of intermediate communication between the first flow channel 9222 and the water inlet valve 923, and does not hinder the cleaning liquid from flowing into the water inlet valve 923. In addition, since the support ring has an annular structure, which is a continuous structure, when the support ring and the annular portion 33 are arranged in contact with each other, the support ring can achieve a supporting effect on the annular portion 33. Therefore, the support ring has the dual functions of circulation of the cleaning liquid and supporting the water inlet valve 923.

In some other embodiments, the support structure 924 is at least one support protrusion. The support protrusion abuts against the annular portion 33, and two adjacent support protrusions are arranged at an interval at the second end of the first flow channel 9222.

The number of support protrusions may be one or more. If the number of support protrusions is one, the support of the annular portion 33 is achieved by arranging the support protrusion alone, and the support protrusion actually acts as a support rod; and if the number of support protrusions is more than one, the support protrusions are arranged at intervals along the circumferential direction of the water inlet valve 923 evenly. Compared with the support ring with a continuous structure, the support structure 924 has a discontinuous intermittent structure at this time, which saves raw materials and has a relatively low production cost.

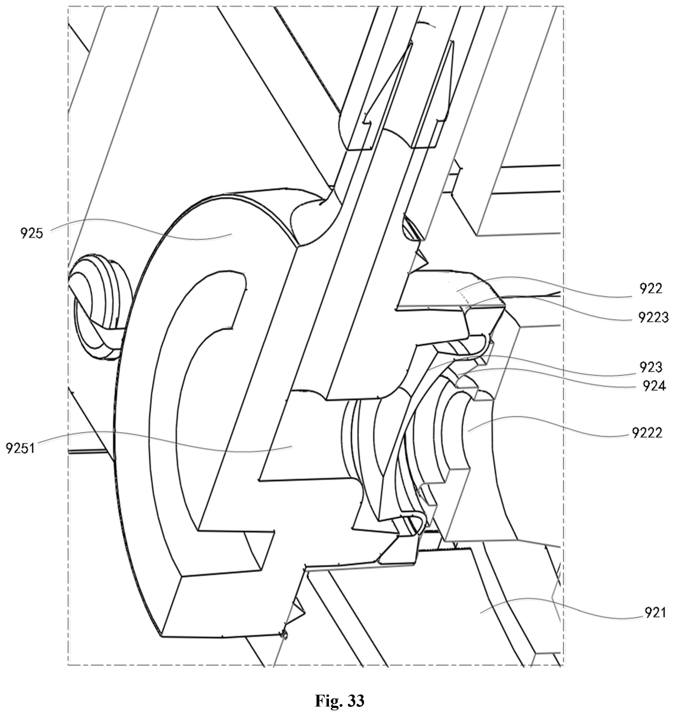

In an embodiment, as shown in FIG. 28 and FIG. 33, the water tank of the cleaning device further includes a fixing member 925, which is connected to the tank body 921 and disposed on a side of the water inlet valve 923 away from the support structure 924 for fixing the water inlet valve 923.

The fixing member 925 may also be called a fixing cover. The fixing member 925 is arranged on the inner wall of the tank body 921, and the tank body 921 provides an installation position for the fixing member 925. The fixing member 925 plays a role of limiting and fixing the water inlet valve 923 to prevent the water inlet valve 923 from falling out of the liquid inlet pipe 922.

It should be noted that the fixing member 925 is provided with a first communication hole, the tank body 921 is provided with a second communication hole corresponding to the first communication hole, and bolts are passed through the first communication hole and the second communication hole to fix the fixing member 925 in the tank body 921. Of course, in some other implementations, the fixing member 925 and the tank body 921 may also be connected by a connection method such as snap-fitting.

In an embodiment, at least part of the fixing member 925 is disposed in the second flow channel 9223, and a third flow channel 9251 is disposed in the fixing member 925. A first end of the third flow channel 9251 is communicated with the second flow channel 9223, and the second end of the third flow channel 9251 is communicated with the inner cavity of the tank body 921, such that the third flow channel 9251 plays a role of intermediate communication. The liquid inlet pipe 922 is communicated with the inner cavity of the tank body 921 through the third flow channel 9251 of the fixing member 925. The fixing member 925 further plays a role of intermediate communication, such that the cleaning liquid flowing out of the liquid inlet pipe 922 flows to the inner cavity of the tank body 921 through the third flow channel 9251 of the fixing member 925.

It can be understood that, along the axial direction of the central portion 9231 of the water inlet valve 923, the first end of the fixing portion 9232 of the water inlet valve 923 abuts against the end surface of the liquid inlet pipe 922 corresponding to the first flow channel 922, and the second end of the water inlet valve 923 abuts against the fixing member 925, which not only realizes the limitation of the water inlet valve 923 along the axial direction of the central portion 9231, but also improves the position stability of the water inlet valve 923.

It should be particularly noted that the side surfaces of the fixing member 925 and the fixing portion 9232 facing each other are bevels to increase the contact area between the fixing member 925 and the fixing portion 9232 and improve the abutment effect of the fixing member 925 on the fixing portion 9232.

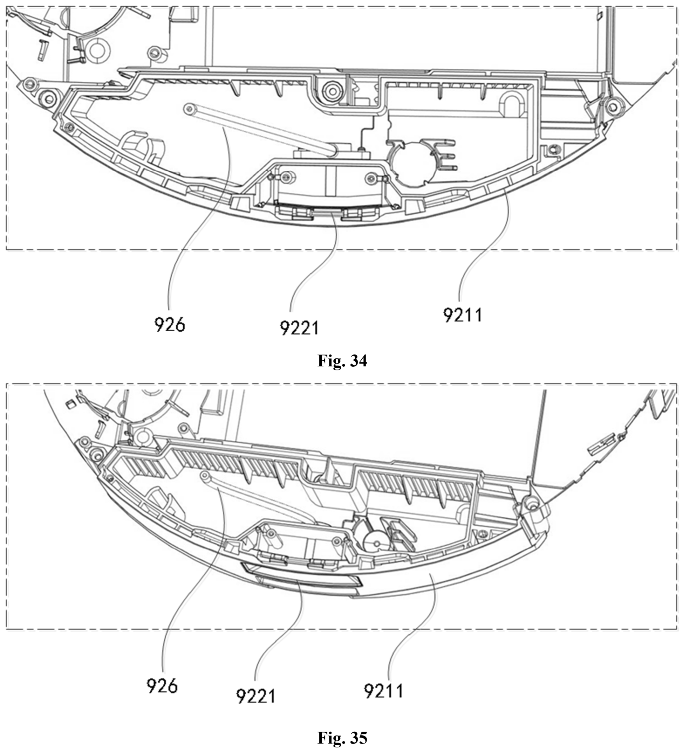

In an embodiment, as shown in FIG. 34 to FIG. 35, the water tank of the cleaning device further includes a liquid inlet pipe 926, which is disposed in the tank body 921. A first end of the liquid inlet pipe 926 is communicated with the third flow channel 9251 of the fixing member 925, and a second end of the liquid inlet pipe 926 is communicated with the inner cavity of the tank body 921. The fixing member 925 acts as a pipeline joint, and the cleaning liquid flowing out of the third flow channel 9251 of the fixing member 925 is delivered to the inner cavity of the tank body 921 through the liquid inlet pipe 926.

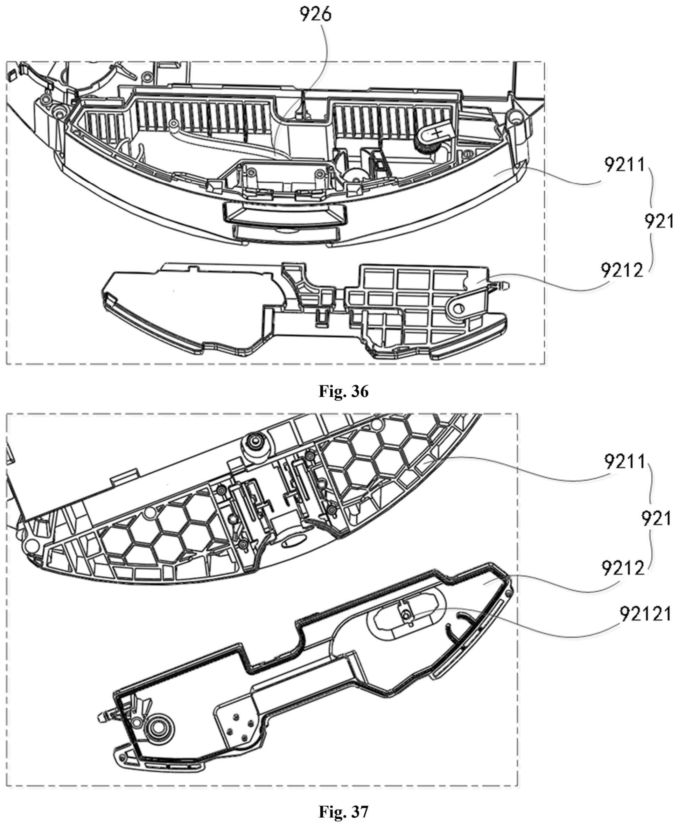

In an embodiment, as shown in FIG. 36 to FIG. 37, the tank body 921 includes a bottom shell 9211 and an upper cover 9212. The bottom shell 9211 is provided with an opening end, and the upper cover 9212 is covered on the opening end of the bottom shell 9211. The second end of the liquid inlet pipe 926 is connected to the upper cover 9212.

The liquid inlet pipe 926 may be placed in the bottom shell 9211 through the opening end of the bottom shell 9211, and the bottom shell 9211 provides an accommodation space for the liquid inlet pipe 926. The upper cover 9212 is covered on the opening end of the bottom shell 9211, such that the bottom shell 9211 and the upper cover 9212 are closed into an integral structure. The first end of the liquid inlet pipe 926 is communicated with the third flow channel 9251 of the fixing member 925, and the second end of the liquid inlet pipe 926 is connected to the upper cover 9212, which is equivalent to that the liquid inlet pipe 926 extends the water inlet port to the top of the tank body 921. When the cleaning base station injects liquid into the tank body 921, the cleaning liquid can fall from the position of the upper cover 9212 into the bottom shell 9211.

In an embodiment, the upper cover 9212 further includes a joint 92121, which is disposed on a side of the upper cover 9212 facing the bottom shell 9211. The joint 92121 is communicated with the liquid inlet pipe 926 and is provided with a communication hole which is communicated with the inner cavity of the tank body 921.

After the cleaning liquid in the liquid inlet pipe 926 is transported to the joint 92121, the communication hole of the joint 92121 acts as a shower head, such that the cleaning liquid is sprinkled from the top of the tank body 921 into the tank body 921. The communication hole of the joint 92121 is used to ensure the supply of the cleaning liquid in the tank body 921. In addition, under normal circumstances, there is a certain height difference between the liquid level of the cleaning liquid in the bottom shell 9211 and the upper cover 9212, such that when the cleaning base station stops supplying cleaning liquid to the water tank, the cleaning liquid in the water tank will not enter the liquid inlet pipe 926 through the communication hole of the joint 92121, which prevents the water flow from being sucked back and further reduces the risk of liquid leakage.

This embodiment further provides a cleaning device, including the above-mentioned water tank of the cleaning device.

The cleaning device further provided in this embodiment includes a water tank of the cleaning device for accommodating cleaning liquid. The support structure 924 of the water tank of the cleaning device can resist the deformation force of the water inlet valve 923 to reduce the risk of liquid leakage of the water tank of the cleaning device. The water tank of the cleaning device can stably and reliably provide the required cleaning liquid for components such as wet cleaning structures, thereby improving the user experience.

Since the liquid inlet pipe 922 of the water tank of the cleaning device provides cleaning liquid to the tank body 921 through the external cleaning base station, a small amount of cleaning liquid will remain in the water tank of the cleaning device after the water tank of the cleaning device is replenished and separated from the cleaning base station, resulting in a poor user experience.

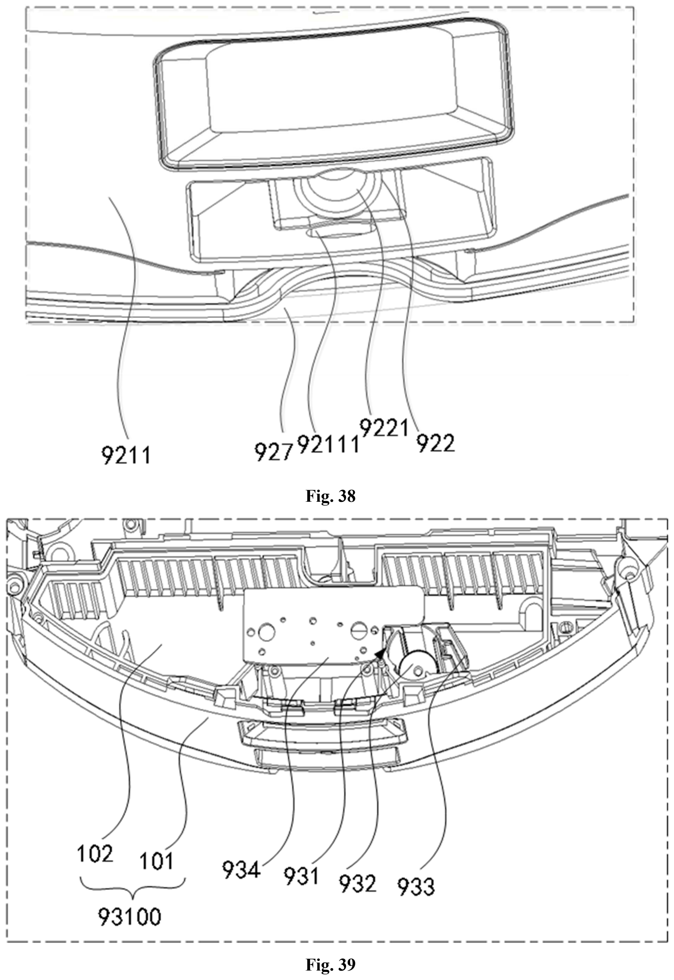

In order to solve this problem, as shown in FIG. 38, the tank body 921 of the water tank of the cleaning device is provided with a water outlet hole 92111. The water outlet hole 92111 is arranged corresponding to the liquid inlet 9221 and is used to discharge the cleaning liquid flowing out from the liquid inlet 9221.

Providing the water outlet hole 92111 on the tank body 921 corresponding to the liquid inlet 9221 is equivalent to providing the water outlet hole 92111 in a residual water area of the water tank of the cleaning device. After the cleaning device is replenished and separated from the cleaning base station, the residual cleaning liquid will flow out through the water outlet hole 92111 if a small amount of cleaning liquid remains in the water tank of the cleaning device, thereby preventing the residual cleaning liquid from occurring and improving the dryness of the residual water area.

In an embodiment, the cleaning device further includes a wet cleaning structure 927, and the tank body 921 is used to provide water to the wet cleaning structure 927.

The wet cleaning structure 927 may be a mop structure or a rolling brush structure, and the mop structure or the rolling brush structure has a water-absorbing material, such as cotton material. The mop structure can be driven to reciprocate or rotate, or the rolling brush structure can be driven to roll, such that the floor to be cleaned can be subjected to wet cleaning. The tank body 921 provides water to the wet cleaning structure 927, such that the wet cleaning structure 927 can realize the function of mopping the floor.

In an embodiment, the water outlet hole 92111 is located above the wet cleaning structure 927, such that the cleaning liquid flowing out of the liquid inlet 9221 flows into the wet cleaning structure 927 through the water outlet hole 92111.

Since the water outlet hole 92111 is located above the wet cleaning structure 927, the residual cleaning liquid flows into the wet cleaning structure 927 located below through the water outlet hole 92111. In this way, while avoiding the waste of cleaning liquid, the cleaning liquid can be effectively reused to provide the wet cleaning structure 927 with the required cleaning liquid, which can solve the problem of liquid retention caused by liquid replenishment in the cleaning device, thereby greatly improving the application experience of the cleaning device, and reducing the repair rate of the cleaning device.

Cleaning devices such as cleaning robots generally have functions such as sweeping, vacuuming and mopping. The water in the water tank is provided to the mop to achieve the mopping function. Since the amount of water in the water tank is limited, the water in the water tank may be exhausted during the mopping process. If the cleaning device is mopping without water, the mopping effect may be poor and it may be difficult to achieve the intended cleaning effect. In addition, the structure of the cleaning robot itself may be damaged, resulting in a deterioration in the mopping function of the cleaning robot.

In order to solve this problem, as shown in FIG. 39, this embodiment provides a liquid level detection structure of a water tank of a cleaning device, which is applicable to a cleaning device, and the cleaning device specifically refers to a cleaning device such as a cleaning robot or a mopping machine, or other types of cleaning devices, such as a floor washing machine. The liquid level detection structure of the water tank of the cleaning device includes a floating assembly 932 and a sensing element 934, wherein the floating assembly 932 is disposed in a water tank 93100 and is configured to move with the change of the liquid level in the water tank 93100, and the sensing element 934 is disposed on the water tank 93100 and is configured to generate a sensing signal in response to the relative movement of the floating assembly 932. The sensing element 934 can be disposed on the upper cover of the water tank 93100, or on the bottom surface of the water tank 93100. The water tank 93100 is used to accommodate a cleaning liquid, and the cleaning liquid may be water, or water added with a detergent, or other liquids with cleaning ability.

In the liquid level detection structure of the water tank of the cleaning device according to this embodiment, the floating assembly 932 can float in the water tank 93100 and can move with the change of the liquid level in the water tank 93100, such that the floating assembly 932 is close to or away from the sensing element 934. The sensing element 934 generates a sensing signal in response to the relative movement of the floating assembly 932, such that the water tank 93100 can detect the liquid level. The floating assembly 932 and the sensing element 934 are arranged up and down relative to each other, and the sensing element 934 can be arranged on the water tank cover directly above the floating assembly 932, and can also be arranged on the bottom surface of the water tank 93100 directly below the floating assembly 932.

Based on the intensity of the sensing signal generated by the sensing element 934, the distance between the floating assembly 932 and the sensing element 934 can be determined, and thus the height of the liquid level in the water tank 93100 can be determined.

It can be understood that the sensing signal generated by the sensing element 934 is a linear signal that gradually increases or decreases with the change in the distance from the floating assembly 932, rather than a signal with only two states of open and closed. Since the signal strength of the sensing signal is in a one-to-one linear relationship with respect to the distance between the sensing element 934 and the floating assembly 932, the corresponding distance can be obtained according to the signal strength, thereby obtaining the accurate value of the liquid level.

Under the action of the buoyancy of the liquid in the water tank 93100, the floating assembly 932 can move up and down along a first direction, and the first direction may be the height direction of the water tank 93100. When the shape of the floating assembly 932 is similar to a cylindrical structure, the first direction may specifically be the axial direction of the floating assembly 932.

In an embodiment, as shown in FIG. 39, the liquid level detection structure of the water tank of the cleaning device further includes a limiting structure 931. The limiting structure 931 is disposed in the water tank 93100, the floating assembly 932 is disposed in the limiting structure 931, and the limiting structure 931 is used to limit the floating assembly 932.

By arranging the floating assembly 932 in the limiting structure 931, the limiting structure 931 plays a role of limiting the floating assembly 932 to enclose the floating assembly 932, so as to avoid a large range of floating deviation of the floating assembly 932 in the horizontal direction during the floating process, and limit the movement range of the floating assembly 932 to improve the accuracy of liquid level detection.

It can be understood that, when the limiting structure 931 is arranged in the water tank 93100 and fixedly connected to the bottom surface of the water tank 93100, there may be a situation where the connection strength is insufficient. During long-term use, the limiting structure 931 may tilt. When the limiting structure 931 tilts toward the floating assembly 932, there is a risk that the limiting structure 931 jams the floating assembly 932, affecting the accuracy of detecting the liquid level. At this time, the water tank 93100 may actually be in a full liquid state, but it is detected by using the sensing element 934 and the floating assembly 932 that the water tank 93100 is not full, and continuous injection of the cleaning liquid into the water tank 93100 occur, resulting in the overflow and waste of the cleaning liquid in the water tank 93100. It is also possible that the water tank 93100 is actually in a state of lack of liquid, but it is detected by using the sensing element 934 and the floating assembly 932 that the water tank 93100 is in a state of sufficient cleaning liquid, and the water tank 93100 cannot be added with the cleaning liquid in time, resulting in the cleaning liquid in the water tank 93100 being completely exhausted during the sweeping and mopping process. If the cleaning device sweeps and mops without cleaning liquid, the structure of the cleaning device itself may be damaged, the probability of fault repair and production cost will be increased, and the sweeping and mopping function of the cleaning device will be deteriorated, affecting the user experience. When the limiting structure 931 tilts in a direction away from the floating assembly 932, the limiting ability of the limiting structure 931 will be reduced, which may cause the floating assembly 932 to not move up and down in the first direction, but to deviate in the horizontal direction, thereby increasing the distance between the floating assembly 932 and the sensing element 934 and causing inaccurate liquid level measurement.

In order to solve this problem, as shown in FIG. 39 to FIG. 40, according to this embodiment, a reinforcing structure 933 is provided on a side of the limiting structure 931 away from the floating assembly 932.

The reinforcement structure 933 is provided on a side of the limiting structure 931 away from the floating assembly 932. That is, the floating assembly 932 is provided on the inner side of the limiting structure 931, and the reinforcement structure 933 is provided on the outer side of the limiting structure 931. In this way, the reinforcement structure 933 will not interfere with the floating assembly 932, and the reinforcement structure 933 plays a role of reinforcing the structure of the limiting structure 931, thereby improving the structural stability of the limiting structure 931, avoiding tilting of the limiting structure 931, reducing the risk of the floating assembly 932 being jammed by the limiting structure 931, and avoiding the occurrence of inaccurate liquid level measurement.

The reinforcing structure 933 may be connected to the outer side of the limiting structure 931 and the bottom surface of the water tank 93100, which is equivalent to increasing the connection area between the limiting structure 931 and the bottom wall 102 of the water tank 93100, thereby improving the fixing effect of the limiting structure 931 relative to the bottom surface of the water tank 93100 and reducing the risk of tilting of the limiting structure 931.

It should be noted that the shapes and structures of the limiting structure 931 and the floating assembly 932 are adapted with each other. The floating assembly 932 may be shaped as a circular column, and the limiting structure 931 may be shaped to be similar to a cylindrical structure. Alternatively, the floating assembly 932 may be shaped as a cuboid, and the limiting structure 931 may be shaped in cross-section to be similar to a rectangular structure. The shape structures of the floating assembly 932 and the limiting structure 931 are not limited in this embodiment, and may be adjusted according to actual production conditions, as long as the shape structures of the limiting structure 931 and the floating assembly 932 are adapted with each other. That is, as long as the limiting structure 931 has the effect of following the shape of the floating assembly 932, their shape sand structures are within the protection scope of this embodiment.

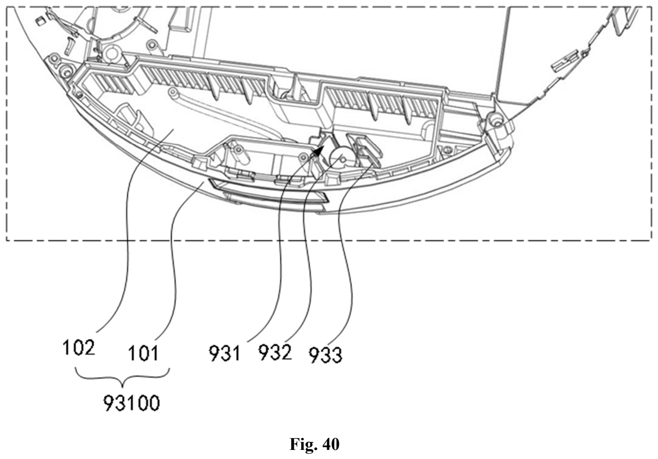

In an embodiment, as shown in FIGS. 40-41, the limiting structure 931 includes multiple enclosure members 9311, which are disposed around the floating assembly 932 to limit the floating assembly 932, and at least one of the multiple enclosure members 9311 is provided with the reinforcing structure 933.

The limiting structure 931 is configured in a split structure, and the multiple enclosure members 9311 of the limiting structure 931 are arranged around the floating assembly 932 to limit the floating assembly 932, which is equivalent to that the multiple independently arranged enclosure members 9311 enclose the floating assembly 932. In this way, by using only the multiple independently arranged enclosure members 9311, the floating assembly 932 can be limited. Under the joint action of the multiple enclosure members 9311, movement of the floating assembly 932 in a large range can be reduced. By using the reinforcement structure 933, the structural strength of at least one enclosure member 9311 can be increased.

In an embodiment, as shown in FIGS. 40-41, the limiting structure 931 further includes a portion of the side wall of the water tank, and the portion of the side wall of the water tank and the multiple enclosure members 9311 are disposed together around the floating assembly 932 to limit the floating assembly 932.

The portion of the side wall of the water tank is specifically a partial structure of the side wall 101 in the water tank 93100, and the limiting structure 931 includes not only the enclosure members 9311, but also the partial structure of the side wall 101. The multiple enclosure members 9311 and the portion of the side wall of the water tank together constitute the limiting structure 931. Since the side wall of the water tank has a good connection effect with the bottom surface of the water tank 93100 and is not easily deformed, using the partial structure of the side wall of the water tank as a part of the limiting structure 931 can reduce the risk of tilting of the limiting structure 931.

It can be understood that the position of the limiting structure 931 may be freely and flexibly set, and the limiting structure 931 may be set near the edge of the water tank 93100 or in the middle of the water tank 93100. When the limiting structure 931 is set near the edge of the water tank 93100, the multiple enclosure members 9311 and the portion of the side wall of the water tank may be arranged at intervals, that is, the enclosure members 9311 and the side wall 101 of the water tank 93100 do not contact each other. At this time, the multiple enclosure members 9311 arranged at intervals and the portion of the side wall of the water tank approximately form an annular structure, and the floating assembly 932 is located inside the annular structure, so as to realize the limiting and enclosure by the limiting structure 931 on the floating assembly 932. At least one of the enclosure members 9311 may also be connected to the portion of the side wall of the water tank, and the side wall 101 of the water tank 93100 plays a role of supporting and fixing the enclosure members 9311, thereby improving the stability of the limiting structure 931. When the limiting structure 931 is disposed in the middle of the water tank 93100, the multiple enclosure members 9311 may be arranged around the floating assembly 932 without forming the limiting structure by resorting to the side wall of the water tank.

In an embodiment, as shown in FIG. 40, a portion of the side wall of the water tank corresponding to the floating assembly 932 is recessed in a direction away from the floating assembly 932 to form a recessed portion 9313, and the recessed portion 9313 and the multiple enclosure members 9311 form the limiting structure 931.

The recessed portion 9313 plays a role of partially accommodating the floating assembly 932 and also plays a role of limiting the floating assembly 932. The recessed portion 9313 and the multiple enclosure members 9311 form the limiting structure 931, which is equivalent to using part of the side wall 101 of the water tank 93100 as a limiting part, thereby saving part of the raw production materials of the limiting structure 931 and saving production costs.

It can be understood that the water tank 93100 is shaped approximately in an arc structure to avoid the situation where an angular structure scratches the user. When the floating assembly 932 is shaped as a circular column, a portion of the arc structure of the side wall 101 of the water tank 93100 may be used to directly limit the floating assembly 932, thereby obtaining raw materials locally and having a relatively low production cost.

In an embodiment, as shown in FIGS. 40 to 41, a guide cavity 142 is formed in the limiting structure 931, and the floating assembly 932 is arranged in the guide cavity 142. A water inlet port 9312 is provided between two adjacent enclosure members 9311 or between the enclosure member 9311 and a portion of the water tank side wall, and the guide cavity 142 is communicated with the inner cavity of the water tank 93100 through the water inlet port 9312.

It can be understood that if the limiting structure 931 has a closed structure, the floating assembly 932 only represents the liquid level in the limiting structure 931, instead of the liquid level of the water tank 93100. For the limiting structure 931 of the split structure, two adjacent enclosure members 9311 are spaced apart and do not contact each other, so there is a gap between the two adjacent enclosure members 9311, and the gap may be used as the water inlet port 9312. The guide cavity 142 is communicated with the inner cavity of the water tank 93100 through the water inlet port 9312, such that the cleaning liquid in the water tank 93100 enters the guide cavity 142 through the water inlet port 9312. That is, the limiting structure 931 does not have a closed structure, and the liquid level detected by the floating assembly 932 is not only the liquid level in the guide cavity 142, but also equivalent to the liquid level of the water tank 93100.

It can be understood that in some other embodiments, the limiting structure 931 may also be an integral structure. The specific form of the limiting structure 931 is not limited in this embodiment and may be adjusted according to actual production conditions. If the limiting structure 931 is an integral structure, since the bottom of the limiting structure 931 is the part where the limiting structure 931 and the water tank 93100 are in contact, the water inlet port 9312 may be provided at the bottom of the limiting structure 931, so as to also achieve that the cleaning liquid in the water tank 93100 flows into the guide cavity 142 of the limiting structure 931 through the water inlet port 9312. At this time, the liquid level in the guide cavity 142 is the same as the liquid level in the water tank 93100.

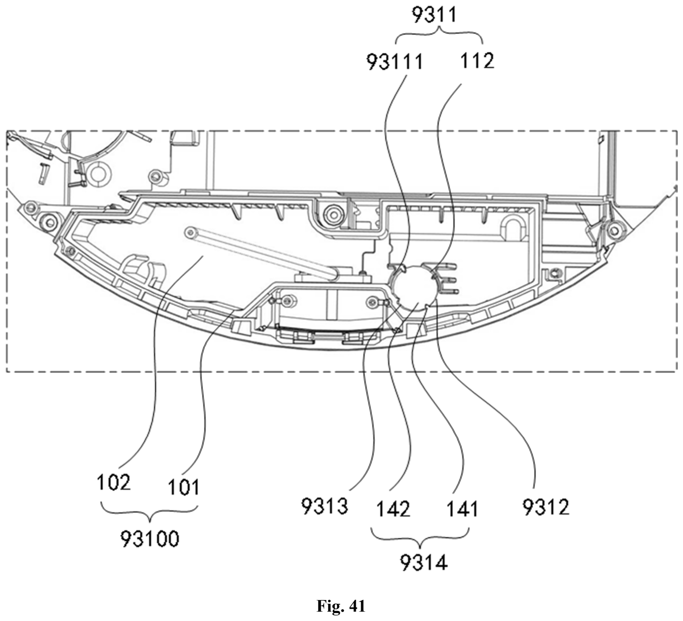

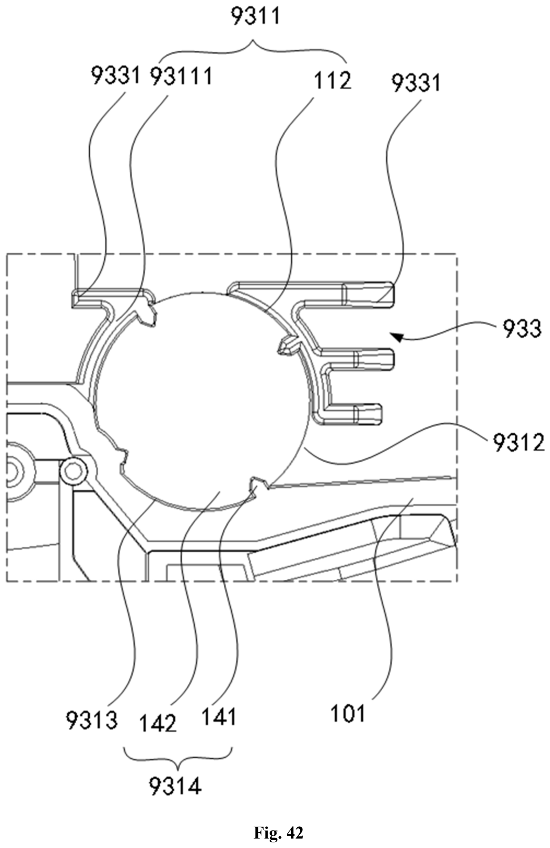

In an embodiment, as shown in FIGS. 41 to 42, the reinforcing structure 933 includes at least one reinforcing rib 9331, that is, multiple reinforcing ribs 9331 are disposed on the limiting structure 931, so as to enhance the structural strength around the limiting structure 931.

In an embodiment, the number of enclosure members 9311 provided is specifically two, and the two enclosure members 9311 are respectively arranged on both sides of the recessed portion 9313. The structures of the two enclosure members 9311 may be the same or different, and the arc lengths of the two enclosure members 9311 with respect to the center of the floating assembly 932 may be the same or different. The number of enclosure members 9311 is not limited in this embodiment, and may be adjusted according to actual production conditions.

In an embodiment, the enclosure members 9311 include a first enclosure member 93111 and a second enclosure member 112. The first enclosure member 93111 is connected to the side wall 101 of the water tank 93100, and the second enclosure member 112 is spaced apart from the side wall 101 of the water tank 93100.

When the first enclosure member 93111 is connected to the side wall 101 of the water tank 93100, the bottom of the first enclosure member 93111 and a side portion of the first enclosure member 93111 close to the side wall 101 of the water tank 93100 are both connected to the water tank 93100, thereby improving the fixing effect of the first enclosure member 93111. Since the second enclosure member 112 is spaced apart from the side wall 101 of the water tank 93100, only the bottom of the second enclosure member 112 is connected to the water tank 93100. At this time, the first side of the first enclosure member 93111 is connected to the side wall 101 of the water tank 93100, a water inlet port 9312 is provided between the second side of the first enclosure member 93111 and the second enclosure member 112, a water inlet port 9312 is provided between the side of the second enclosure member 112 away from the first enclosure member 93111 and the side wall 101 of the water tank 93100, and the guide cavity 142 of the limiting structure 931 is communicated with the water tank 93100 by means of the two water inlet ports 9312, such that the liquid levels in the inner cavity of the water tank 93100 and the guide cavity 142 remain consistent.

In an embodiment, as shown in FIGS. 41 to 42, the number of reinforcing ribs 9331 arranged corresponding to the first enclosure member 93111 is less than the number of reinforcing ribs 9331 arranged corresponding to the second enclosure member 112.

It can be understood that since the first enclosure member 93111 is connected to the side wall 101 of the water tank 93100, and the second enclosure member 112 is not connected to the side wall 101 of the water tank 93100, the connection strength between the second enclosure member 112 and the water tank 93100 is less than the connection strength between the first enclosure member 93111 and the water tank 93100. The connection strength between the second enclosure member 112 and the water tank 93100 may be enhanced by setting the number of reinforcing ribs 9331 corresponding to the second enclosure member 112 to be greater than the number of reinforcing ribs 9331 corresponding to the first enclosure member 93111, such that the first enclosure member 93111 and the second enclosure member 112 have similar upright states relative to the water tank 93100, so as to improve the positional stability of the first enclosure member 93111 and the second enclosure member 112 and reduce the jamming of the floating assembly 932 due to tilting of the limiting structure 931.

In an embodiment, as shown in FIGS. 41 to 42, multiple reinforcing ribs 9331 are arranged in parallel at intervals or crosswise with each other.

The multiple reinforcing ribs 9331 may be arranged in parallel at intervals, which is aesthetically pleasing and neat, such that the liquid level detection structure of the entire water tank of the cleaning device occupies less space and is convenient for arranging other components in the water tank 93100. The multiple reinforcing ribs 9331 may be arranged crosswise to form a reinforcing rib net to resist impact forces in different directions.

It can be understood that the multiple reinforcing ribs 9331 may also be arranged in pairs, two reinforcing ribs 9331 of each pair facing each other and bending in opposite directions, so as to achieve mutual complement.

In an embodiment, the limiting structure 931, the reinforcing structure 933 and the water tank 93100 are formed as an integrated structure. The integrated structure enables reduction of the number of parts assembling steps and reduction of production costs.

In an embodiment, as shown in FIGS. 41 to 42, a guide structure 9314 is provided on a side of the limiting structure 931 facing the floating assembly 932 and is used to guide the floating assembly 932.

By providing the guide structure 9314 on one side of the limiting structure 931 facing the floating assembly 932, the guide structure 9314 plays a role of guiding and positioning the floating assembly 932. Under the action of the guide structure 9314, the floating assembly 932 is arranged in a floatable manner in the guide cavity 142 of the limiting structure 931 and can move up and down along the first direction.

Specifically, the guide structure 9314 includes multiple claws 141, and the guide cavity 142 for limiting the floating assembly 932 is formed between the multiple claws 141. The claws 141 are arranged parallel to each other and extend along the first direction to ensure that the floating assembly 932 moves linearly along the first direction in the guide cavity 142. The multiple claws 141 are evenly arranged on a circumference at equal intervals, such that the multiple claws 141 can evenly surround the outside surface of the floating assembly 932, thereby improving the balance of the force on the claws 141 and the side surface of the floating assembly 932, and reducing the jamming and tilting of the floating assembly 932. The claws 141 can also reduce the contact area between the floating assembly 932 and the limiting structure 931, such that the surface contact between the floating assembly 932 and the limiting structure 931 is changed into the line contact between the floating assembly 932 and the claws 141, which reduces the contact area to the maximum extent and the sliding resistance, while achieving the guiding effect, thereby reducing the probability of jamming of the floating assembly 932, and improving the accuracy of liquid level detection.

The number of the claws 141 is at least three, which is not specifically limited and can be determined according to the shape of the floating assembly 932. According to the principle that three points define a plane, it is ensured that the floating assembly 932 will not be separated from the limiting structure 931. If the floating assembly 932 has a structure of circular column, three claws 141 may be used to limit the floating assembly 932 to move linearly along the first direction, and four claws 141 may also be used to obtain a more stable effect.

In an embodiment, the sensing element 934 may be disposed at a position corresponding to the guide cavity 142 of the limiting structure 931, thereby ensuring that the sensing signal obtained by the sensing element 934 reflects the real liquid level information.

It should be pointed out that the type of sensing signal generated by the sensing element 934 according to this embodiment is not limited, and the sensing signal may be derived from a magnetic field signal. Specifically, the sensing element 934 according to this embodiment is implemented as a Hall sensor, and the Hall sensor may be arranged on the top of the water tank 93100 and directly above the guide cavity 142.

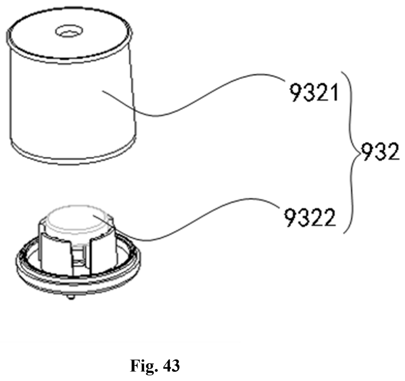

As shown in FIG. 43, the floating assembly 932 includes a floating shell 9321 and a permanent magnet 9322. The floating shell 9321 can float with the liquid level, the permanent magnet 9322 is arranged in the floating shell 9321, and the floating shell 9321 plays a role of protecting the permanent magnet 9322. The permanent magnet 9322 is of a material with a constant magnetic field. The Hall sensor is an element whose output level is linearly related to the external magnetic field within a certain range.

When the liquid level in the water tank 93100 drops, the floating shell 9321 drops along with the liquid level, and the permanent magnet 9322 fixed in the floating shell 9321 gradually moves away from the Hall sensor, such that the magnetic field strength is continuously weakened, and the electrical signal generated by the Hall sensor is also continuously weakened. If the electrical signal generated by the Hall sensor reaches the minimum limit, it means that the permanent magnet 9322 is almost close to the bottom of the water tank 93100, so it is determined that the water tank 93100 is in a state of lack of cleaning liquid, and at this time, it is necessary to provide cleaning liquid to the water tank 93100 in time.

When the floating assembly 932 floats up with the rise of the liquid level in the water tank 93100, the permanent magnet 9322 gradually approaches the Hall sensor, such that the magnetic field strength is continuously enhanced, and the electrical signal generated by the Hall sensor is also continuously enhanced. If the electrical signal generated by the Hall sensor reaches the maximum limit, it means that the permanent magnet 9322 is almost close to the top of the water tank 93100, so it is determined that the water tank 93100 is in a state of full of cleaning liquid, and at this time, there is no need to supply cleaning liquid to the water tank 93100.

It can be understood that if the Hall sensor is arranged below the permanent magnet 9322, the only difference is that when the electrical signal generated by the Hall sensor is enhanced, it means that the liquid level of the water tank 93100 is reduced; and when the electrical signal generated by the Hall sensor is reduced, it means that the liquid level of the water tank 93100 is increased. The principle is similar, so it will not be described in detail.

In an embodiment, the liquid level detection structure of the water tank of the cleaning device further includes a control panel, which is electrically connected to the Hall sensor, and is configured to obtain the corresponding liquid level value according to the change in the strength of the electrical signal, thereby realizing quantitative detection of the liquid level. It can be understood that the scheme of using the Hall sensor in this embodiment is not a limitation to the present disclosure, and the ways used by those skilled in the art to adopt other types of signal sensing elements for sensing signals such as light waves, sound waves, etc. are all within the scope of protection of the present disclosure.

This embodiment further provides a cleaning device, which includes the water tank 93100 and the above-mentioned liquid level detection structure of the water tank of the cleaning device, wherein the liquid level detection structure of the water tank of the cleaning device is disposed in the water tank 93100 and is used to detect the liquid level of the water tank 93100. The cleaning device specifically refers to a cleaning device such as a cleaning robot or a mopping machine, or other types of cleaning devices, such as a floor washing machine.

The cleaning device according to this embodiment can utilize the liquid level detection structure of the water tank of the cleaning device to detect the information on the liquid level of the water tank 93100, so as to perform cleaning and mopping actions more reasonably, thereby making the cleaning device more intelligent and improving the user experience.

In an embodiment, the cleaning device further includes a display module and a prompt module, which are electrically connected to the control panel, respectively. The display module is used to display water volume information, and the prompt module is used to send prompt information to the user. The control panel receives the sensing signal sent by the sensing element 934, calculates the corresponding water level information according to the change in the strength of the sensing signal, and sends the water level information to the display module for the user to view.

When the water level is detected to be low, a cleaning behavior may be performed intelligently, such as estimating the remaining cleaning time based on the remaining water volume and shortening the cleaning travel, or controlling the cleaning device to stop cleaning, or controlling the cleaning device to return to the cleaning base station for water replenishment, etc. At the same time, the prompt module is controlled to send a prompt message to the user.

It can be understood that the prompt module may also include a voice device, which is used to issue a prompt voice to prompt the user of the liquid level change, such as issuing a voice prompt such as "the liquid level is reduced, please add more".

With the cooperation of the control panel, the sensing element 934 and the floating assembly 932, the cleaning device can quantitatively detect the liquid level of the water tank 93100, and intelligently plan the cleaning and mopping paths according to the remaining water volume, thereby improving the cleaning and mopping effects. At the same time, the prompt module can be used to timely prompt the user to add water, thereby avoiding the cleaning device from idling due to lack of cleaning liquid and extending the service life of the cleaning device.

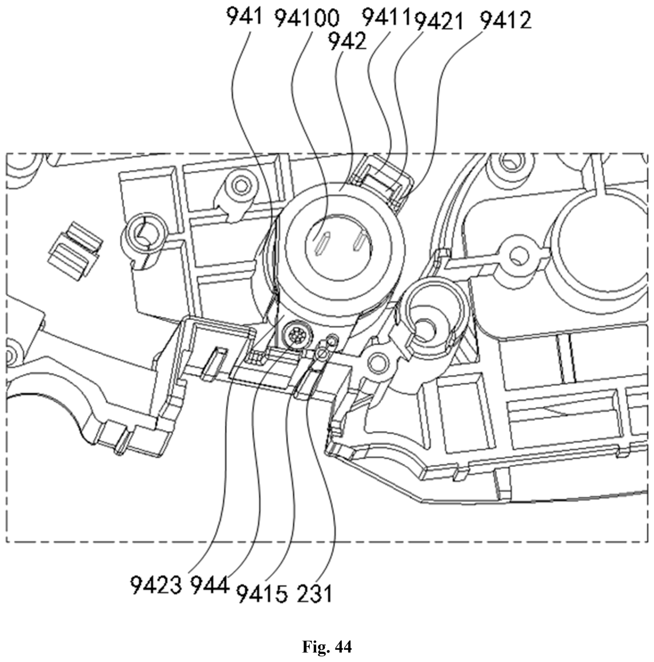

This embodiment provides a cleaning device, which may be a cleaning robot or a mopping machine, or other types of cleaning devices, such as a floor washing machine, etc. As shown in FIG. 44, the cleaning device includes a sensor 94100 and a sensor installation structure used to mount the sensor 94100.

The cleaning device according to this embodiment can realize the installation of the sensor 94100 by using the sensor installation structure, thereby improving the installation convenience of the sensor 94100. The sensor 94100 is used to detect the floor to be cleaned, such that the cleaning device can adjust the direction in time, thereby ensuring that the cleaning device can operate smoothly during sweeping or mopping.

The sensor 94100 is specifically a carpet sensor used to identify the carpet in some embodiments of the present disclosure. The carpet sensor can be used to identify whether there is a carpet, so as to provide the required parameters for the cleaning device.

It can be understood that in some other embodiments, the sensor 94100 may also be an infrared sensor, a water pressure sensor, an electricity quantity sensor, a dirty degree sensor, etc., and its installation structure and control implementation are similar to those of the carpet sensor, and will not be described in detail.

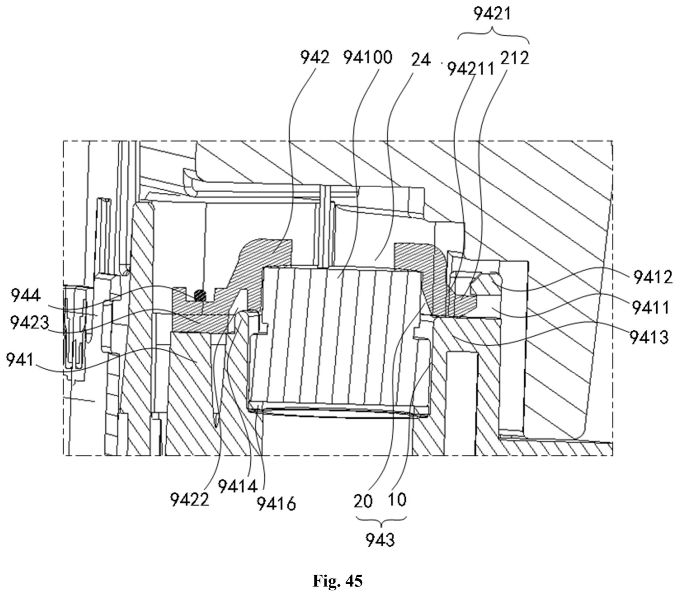

This embodiment further provides a sensor installation structure. As shown in FIGS. 44 and 45, the sensor installation structure includes a main body 941 and a fixing cover 942, the fixing cover 942 is arranged on the main body 941, and the accommodation cavity 943 is arranged between the fixing cover 942 and the main body 941 for accommodating the sensor 94100.

In the sensor installation structure according to this embodiment, the main body 941 serves to support the sensor 94100 and the fixing cover 942, and the accommodation cavity 943 is provided between the main body 941 and the fixing cover 942. The accommodation cavity 943 not only provides an accommodation space for the sensor 94100, but also serves to protect the sensor 94100.

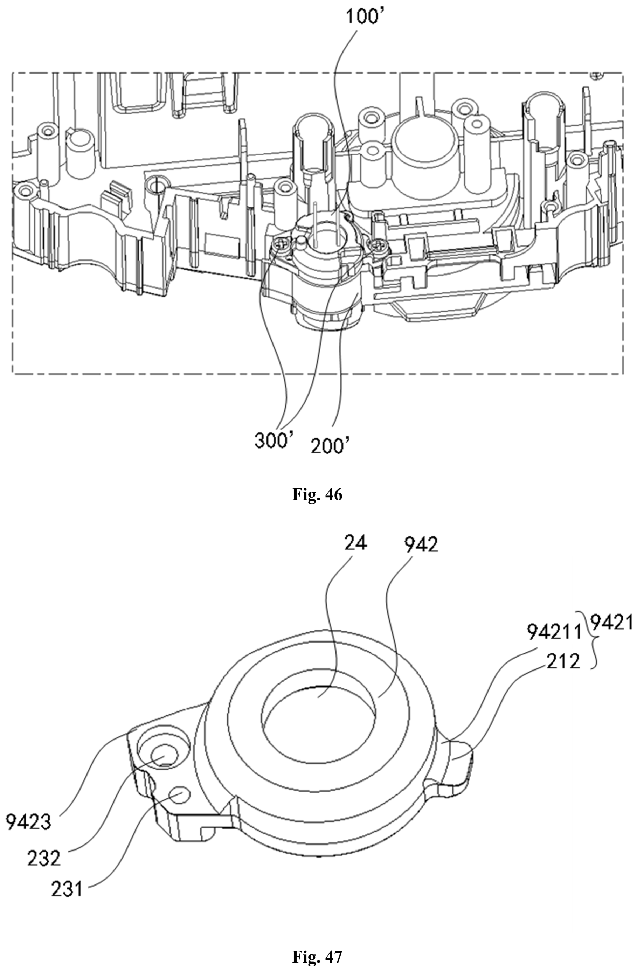

In the related art, both ends of the sensor fixing cover 100' are fixed to the main shell 200' by at least two screws 300' respectively (as shown in FIG. 46). During the installation and removal of the sensor, the two screws 300' need to be installed and removed successively, resulting in a relatively long assembly time and maintenance time of the whole machine, thereby increasing production and maintenance costs.

In order to solve this problem, in the sensor installation structure according to this embodiment, as shown in FIGS. 44 and 45, the fixing cover 942 has a first end and a second end opposite to each other. One of the main body 941 and the first end of the fixing cover 942 is provided with a fixing portion, and the other of the main body 941 and the first end of the fixing cover 942 is provided with a fixing matching portion matched with the fixing portion. The second end of the fixing cover 942 is provided with an installation portion 9423, and the installation portion 9423 is connected to the main body 941 via a fastener.

In the sensor installation structure according to this embodiment, one of the first end of the fixing cover 942 and the main body 941 is provided with the fixing portion, and the other of the first end of the fixing cover 942 and the main body 941 is provided with the fixing matching portion matched with the fixing portion. Under the mutual cooperation of the fixing portion and the fixing matching portion, the fixing between the first end of the fixing cover 942 and the main body 941 is achieved. By providing the installation portion 9423 at the second end of the fixing cover 942, the installation portion 9423 increases the contact area between the fixing cover 942 and the main body 941 while providing an installation position for the second end of the fixing cover 942, and improves the connection stability between the fixing cover 942 and the main body 941. The installation portion 9423 is connected to the main body 941 through a fastener to achieve the fixing between the second end of the fixing cover 942 and the main body 941, such that both ends of the fixing cover 942 can be fixed to the main body 941. The fixing cover 942 can be fixed to the main body 941 with only one fastener, which saves another fastener and saves the assembly time and maintenance time of the whole machine, reduces the assembly and maintenance costs, and improves the production efficiency.

The installation portion 9423 is specifically a bump protruding along the edge of the second end of the fixing cover 942, and the bump has a shape similar to a lug structure.

It should be noted that the fastener is specifically a bolt 944, which has a simple structure, is easy to use, and has a relatively low production cost. Specifically, the first connection hole 232 is provided in the installation portion 9423, and a second connection hole is provided in the main body 941 corresponding to the first connection hole 232. The bolt 944 is respectively penetrated through the first connection hole 232 and the second connection hole to fix the fixing cover 942 on the main body 941. At the same time, compared with the manner of directly penetrating the fixing cover 942 with the bolt 944 in the related art, the bolt 944 is prevented from directly gapping the fixing cover 942, thereby extending the service life of the fixing cover 942.

It can be understood that the fasteners according to this embodiment include but are not limited to bolts 944. For example, fixing pins and other fixing members may also be selected. The specific structure of the fastener according to this embodiment is not limited, and any ways of achieving the fixing between the installation portion 9423 and the main body 941 are encompassed within the protection scope of this embodiment.

Specifically, as shown in FIGS. 44 and 45, the main body 941 includes a machine body and a bottom shell. The bottom shell is arranged on the bottom surface of the machine body, and the machine body plays a role of overall support. The bottom shell has a hollow structure, and a first cavity 10 is arranged in the bottom shell for accommodating the lower half of the sensor 94100. The bottom shell has an opening end, and the sensor 94100 is placed in the first cavity 10 through the opening end of the bottom shell.

It can be understood that the sensor 94100 may be shaped in a circular column structure or a square structure, etc. The present embodiment does not limit the shape structure of the bottom shell, the fixing cover 942 and the sensor 94100, and as long as the bottom shell and the fixing cover 942 are respectively compatible with the shape of the sensor 94100, they are all within the protection scope of the present embodiment.

If the shape of the sensor 94100 is approximately a circular column structure, then the first cavity 10 is also approximately of a circular column structure. A first axial hole and a second axial hole are arranged in the first cavity 10 along its axial direction. The inner diameter of the first axial hole is slightly larger than the outer diameter of the sensor 94100. The first axial hole and the outer diameter of the sensor 94100 match with each other. The diameter of the first axial hole is larger than the diameter of the second axial hole to form a stepped hole structure. The step 9416 of the stepped hole can support the bottom edge of the sensor 94100, thereby playing a role of supporting and limiting the sensor 94100.

It should be noted that the fixing cover 942 may have a sheet structure, in which case the first cavity 10 is the accommodation cavity 943. The fixing cover 942 may also have a three-dimensional structure, and a side of the fixing cover 942 facing the bottom shell is provided with the second cavity 20 for accommodating the upper half of the sensor 94100. The second cavity 20 and the first cavity 10 are arranged opposite to each other and are communicated with each other, and the first cavity 10 and the second cavity 20 are combined to form the accommodation cavity 943. In this way, when the bottom shell and the fixing cover 942 are fixed, the contact area between the fixing cover 942 and the sensor 94100 is relatively large, and the fixing cover 942 plays a role of press-fitting the sensor 94100, thereby improving the position stability of the sensor 94100.

It should be noted that the structure formed by the fixing cover 942 and the main body 941 is not a closed structure. Specifically, as shown in FIG. 45, a through hole 24 is provided in the fixing cover 942, and the through hole 24 may be a circular through hole. A wiring bundle is provided on the top of the sensor 94100, and the wiring bundle is electrically connected to a main board after passing through the through hole 24. The bottom of the sensor 94100 is arranged toward the ground, and the bottom of the sensor 94100 emits a sensing signal. The sensing signal passes through the second axial hole of the first cavity 10 and is sent to the carpet. Then, the sensor 94100 transmits the received sensing signal to the main board, thereby realizing the carpet detection and identification process.

In an embodiment, as shown in FIGS. 44-45, the fixing portion is an extension arm 9421, the fixing matching portion is an insertion hole 9411, and the extension arm 9421 is at least partially disposed in the insertion hole 9411.

In other words, a side of one of the fixing cover 942 and the main body 941 opposite to the other of the fixing cover 942 and the main body 941 is provided with the extension arm 9421. For example, the extension arm 9421 is formed by extending along an edge of the fixing cover 942 or an edge of the main body 941. The other of the fixing cover 942 and the main body 941 is arranged with the insertion hole 9411, and the insertion hole 9411 provides an insertion space for the extension arm 9421, such that the extension arm 9421 is at least partially inserted into the insertion hole 9411, thereby achieving fixing between the extension arm 9421 and the insertion hole 9411.

It can be understood that the insertion hole 9411 may have a through hole structure, or a blind hole structure. That is, the insertion hole 9411 may be a slot, and any ways of achieving the insertion between the extension arm 9421 and the insertion hole 9411 fall within the protection scope of this embodiment.

In an embodiment, the extension arm 9421 is snap-fitted to the insertion hole 9411.

By the snap-fitting between the extension arm 9421 and the insertion hole 9411, the fixing effect between the extension arm 9421 and the insertion hole 9411 is improved. At this time, the first end of the fixing cover 942 and the main body 941 are fixed by snap-fitting, and the second end of the fixing cover 942 and the main body 941 are fixed by bolts 944.

During installation, the second end of the fixing cover 942 may be slightly higher than the first end of the fixing cover 942. For example, the second end of the fixing cover 942 is first lifted, the first end of the fixing cover 942 is brought close to the main body 941, and the extension arm 9421 is partially inserted into the insertion hole 9411 to achieve precise alignment between the extension arm 9421 and the insertion hole 9411, which plays a role of initial pre-positioning. Then, the second end of the fixing cover 942 is gradually dropped and caused to contact the main body 941, until the extension arm 9421 is snap-fitted into the insertion hole 9411. Finally, the second end of the fixing cover 942 is connected to the main body 941 by the bolt 944 to achieve fixing between the fixing cover 942 and the main body 941.

Alternatively, the second end of the fixing cover 942 does not need to be higher than the first end of the fixing cover 942, and the first end of the fixing cover 942 is directly moved in parallel to the main body 941, until the extension arm 9421 is inserted into the insertion hole 9411. When the fixing cover 942 is installed, the present embodiment does not limit the relative height of the first end and the second end of the fixing cover 942, and any ways of achieving the alignment between the extension arm 9421 and the insertion hole 9411 fall within the protection scope of the present embodiment.

It should be particularly noted that the mutual cooperation between the fixing portion and the fixing matching portion includes but is not limited to snap-fitting, and may also include a rotational connection, or fixing by other connecting members.

Specifically, as shown in FIG. 44 to FIG. 45, the extension arm 9421 includes a snap-fitting portion 212 and a transition portion 94211. The first end of the transition portion 94211 is connected to the edge of the fixing cover 942, and the second end of the transition portion 94211 is connected to the snap-fitting portion 212. The snap-fitting portion 212 is snap-fitted to the insertion hole 9411, and the transition portion 94211 plays a role of intermediate transition. If the accommodation cavity 943 has a circular column structure, the position where the fixing cover 942 and the transition portion 94211 are connected is approximately along the axial direction of the accommodation cavity 943, and the depth direction of the insertion hole 9411 is approximately along the radial direction of the accommodation cavity 943. At this time, the transition portion 94211 plays a role of intermediate connection and also plays a role of direction conversion, such that the extension arm 9421 actually plays the role of a clasper.

It can be understood that the connection portions between the inner side and the outer side of the transition part 94211 and the fixing cover 942 may have a rounded structures to reduce the possibility of hard scratches.

In an embodiment, as shown in FIGS. 47 to 48, a limiting portion 9412 is disposed on the outer side of the insertion hole 9411 to limit the extension arm 9421, such that the extension arm 9421 is inserted in the insertion hole 9411.

It should be particularly noted that the extension arm 9421 is disposed on a side of the first end of the fixing cover 942 facing the main body 941, or the extension arm 9421 is disposed on a side of the main body 941 facing the first end of the fixing cover 942. For example, the extension arm 9421 protrudes from one of the fixing cover 942 and the main body 941, such that the extension arm 9421 can approach the other of the fixing cover 942 and the main body 941. The limiting portion 9412 is disposed on the other of the fixing cover 942 and the main body 941. The limiting portion 9412 is disposed on the outside of the insertion hole 9411. When the extension arm 9421 is inserted into the insertion hole 9411, the limiting portion 9412 plays a role of limiting the extension arm 9421, such that the extension arm 9421 can be accurately aligned with the insertion hole 9411, thereby improving the convenience of inserting the extension arm 9421 into the insertion hole 9411.

Specifically, the insertion hole 9411 may be directly arranged in the limiting portion 9412, and the limiting portion 9412 provides an arrangement position for the insertion hole 9411. After the limiting portion 9412 preliminarily positions the extension arm 9421, the extension arm 9421 can be directly connected to the insertion hole 9411 arranged in the limiting portion 9412, thereby improving the accuracy and convenience of insertion.

In an embodiment, the limiting portion 9412 has a U-shaped structure, the opening end of the U-shaped structure is arranged toward the extension arm 9421, and the side of the U-shaped structure away from the opening end is provided with the insertion hole 9411.

The opening end of the U-shaped structure is arranged toward the extension arm 9421 to provide an entrance for the extension arm 9421 to extend into the limiting portion 9412. The two side arms of the U-shaped structure limit the two sides of the extension arm 9421 to improve the facing effect. The side of the U-shaped structure away from the opening end is used to limit the end of the extension arm 9421 to prevent the extension arm 9421 from moving a large distance beyond the limiting portion 9412. The insertion hole 9411 is arranged on the side of the U-shaped structure away from the opening end. For example, the opening end and the insertion hole 9411 are arranged in a manner of facing each other, such that the extension arm 9421 can directly dock with the insertion hole 9411 after entering through the opening end.

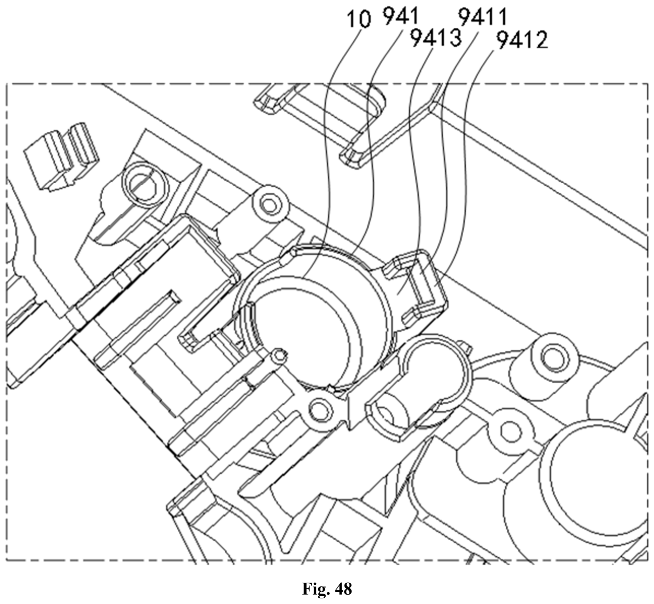

In an embodiment, as shown in FIG. 45 and FIG. 48, the limiting portion 9412 is disposed on a bearing portion 9413 used to bear at least a portion of the extension arm 9421.

Since the extension arm 9421 protrudes from one of the fixing cover 942 and the main body 941, the bearing portion 9413 is provided at the other of the first end of the fixing cover 942 and the main body 941, and the limiting portion 9412 is provided on the bearing portion 9413. The bearing portion 9413 plays a role of supporting and bearing the limiting portion 9412. The bearing portion 9413 can bear at least part of the extension arm 9421. The bearing portion 9413 also plays a role of supporting and bearing the extension arm 9421, and plays a role of bracketing the extension arm 9421, so as to prevent the extension arm 9421 from being disengaged from the insertion hole 9411 due to its large mass.

In an embodiment, as shown in FIGS. 45 and 48, the upper and lower sides of the extension arm 9421 abut against the bearing portion 9413 and the inner wall of the insertion hole 9411, respectively. Alternatively, the upper side of the extension arm 9421 abuts against the inner wall of the insertion hole 9411.

Specifically, the upper side of the snap-fitting portion 212 in the extension arm 9421 abuts against the inner wall of the insertion hole 9411, and the lower side of the snap-fitting portion 212 abuts against the top surface of the bearing portion 9413. At this time, the bearing portion 9413 and the insertion hole 9411 jointly fix the extension arm 9421.

When installing the fixing cover 942, a side of the extension arm 9421 gradually close to the limiting portion 9412 may be pressed down, such that the first end of the fixing cover 942 with the extension arm 9421 is lower than the second end of the fixing cover 942 without the extension arm 9421. As the extension arm 9421 is gradually inserted into the insertion hole 9411, the second end of the fixing cover 942 is pressed down. When the lower side of the extension arm 9421 abuts against the bearing portion 9413, the upper side of the extension arm 9421 just abuts against the inner wall of the insertion hole 9411, which is easy to operate and convenient for installation and disassembly.

It can be understood that if the thickness of the extension arm 9421 is relatively large, the upper and lower sides of the extension arm 9421 can respectively abut against the bearing portion 9413 and the inner wall of the insertion hole 9411; and if the thickness of the extension arm 9421 is relatively small, as long as the upper side of the extension arm 9421 abuts against the inner wall of the insertion hole 9411, the fixing of the extension arm 9421 can be achieved.

It can be understood that the first end of the fixing cover 942 is inserted into the insertion hole 9411 through the extension arm 9421 and is snap-fitted with the insertion hole 9411, and the second end of the fixing cover 942 is fixed to the main body 941 by means of the bolt 944. Compared with the prior art, another screw is saved, the production cost is reduced, and installation and disassembly are more convenient.

In an embodiment, as shown in FIG. 45, one of the main body 941 and the fixing cover 942 is provided with a limiting protrusion 9414, and the other of the main body 941 and the fixing cover 942 is provided with a limiting groove 9422. The limiting groove 9422 is used to limit the position of the limiting protrusion 9414.

Specifically, the limiting protrusion 9414 is provided on the side of the main body 941 facing the fixing cover 942, and the limiting groove 9422 is provided on the fixing cover 942 corresponding to the limiting protrusion 9414. The limiting groove 9422 is used to limit the limiting protrusion 9414. With the mutual cooperation between the limiting protrusion 9414 and the limiting groove 9422, the position limiting between the main body 941 and the fixing cover 942 is achieved, and the initial pre-positioning is enabled when the fixing cover 942 is installed to the main body 941.

It should be noted that the limiting protrusion 9414 may have a column structure, and the cross section of the limiting protrusion 9414 may also have a ring structure or an arc structure. This embodiment does not limit the shape structure of the limiting protrusion 9414, as long as the limiting protrusion 9414 and the limiting groove 9422 can be mutually limited, it is within the protection scope of this embodiment.

In an embodiment, one of the installation portion 9423 and the main body 941 is provided with a positioning column 9415 (as shown in FIG. 44), and the other of the installation portion 9423 and the main body 941 is provided with a positioning hole 231 (as shown in FIG. 47), and the positioning column 9415 is inserted in the positioning hole 231.

Specifically, the positioning column 9415 is provided at a position of the main body 941 corresponding to the installation portion 9423, and the positioning hole 231 is provided at the installation portion 9423 of the fixing cover 942 corresponding to the positioning column 9415. The positioning column 9415 is inserted in the positioning hole 231 to achieve initial positioning between the second end of the fixing cover 942 and the main body 941, thereby improving the effect of fixing the installation portion 9423 and the main body 941.

The cleaning device according to this embodiment uses the sensor installation structure to install the sensor 94100, thereby improving the installation convenience of the sensor 94100 in the cleaning device. The sensor 94100 is used to detect the floor to be cleaned, such that the cleaning device can perform the corresponding cleaning operation according to the detection result of the sensor, and the cleaning device can smoothly carry out the process of sweeping or mopping. One of the first end of the fixing cover 942 and the main body 941 is provided with the fixing portion, and the other of the first end of the fixing cover 942 and the main body 941 is provided with the fixing matching portion matched with the fixing portion. Under the mutual cooperation of the fixing portion and the fixing matching portion, fixing between the first end of the fixing cover 942 and the main body 941 is achieved. Since the second end of the fixing cover 942 is connected to the main body 941 by a fastener, fixing between the second end of the fixing cover 942 and the main body 941 is achieved, such that both ends of the fixing cover 942 can be fixed to the main body 941, and the fixing effect is good. The fixing cover 942 only needs one fastener to be fixed to the main body 941, which saves another screw, saves the assembly time and maintenance time of the whole machine, reduces the assembly and maintenance costs, and improves production efficiency.

In an embodiment, the cleaning device further includes a display module, a prompt module and a control module. The control module is electrically connected to the display module, the prompt module and the carpet sensor, respectively. The display module is used to display information; the prompt module is used to send prompt information to the user; and the main board of the control module is used to receive the sensing signal emitted by the carpet sensor, calculate the corresponding floor information based on the acquired sensing signal, and send the floor information to the display module for the user to view. When the detected floor information indicates the presence of a carpet, the cleaning device is controlled to disable the sweeping or mopping function, and the prompt module is controlled to send prompt information to the user, or the sweeping and mopping behaviors are performed more reasonably, so as to make the cleaning device more intelligent and improve the user experience.

The embodiments of the present disclosure relate to the following aspects.

According to one aspect of the present disclosure, a cleaning device is provided, including:
a water storage tank configured to store cleaning liquid;
an overflow structure disposed on the water storage tank;
an enclosing structure at least partially disposed around the overflow structure and configured to retain and guide the cleaning liquid flowing out of the overflow structure; and
a wet cleaning structure, the water storage tank being configured to provide the cleaning liquid for the wet cleaning structure.

In some embodiments of the present disclosure, a portion of the water storage tank disposed inside the enclosing structure is provided with a guide slope, which is inclined in a direction away from the overflow structure and is configured to guide the cleaning liquid flowing out of the overflow structure.

In some embodiments of the present disclosure, the cleaning device further includes:
a guide pipe disposed in the water storage tank;
wherein the enclosing structure is a closed ring structure, and the enclosing structure is sleeved around the overflow structure and the guide pipe, such that the cleaning liquid flowing out of the overflow structure flows out through the guide pipe.

In some embodiments of the present disclosure, the cleaning device further includes a cover plate, an opening is provided on a side of the enclosing structure away from the water storage tank, and the cover plate covers the enclosing structure and blocks the opening.

In some embodiments of the present disclosure, the enclosing structure and the water storage tank are of an integrally formed structure.

In some embodiments of the present disclosure, an accommodating space is formed by the cover plate, the water storage tank and the enclosing structure, and the accommodating space is configured to accommodate the cleaning liquid flowing out of the overflow structure and prevent the cleaning liquid from overflowing.

In some embodiments of the present disclosure, one of the enclosing structure and the cover plate is provided with a mounting column, and the other of the enclosing structure and the cover plate is provided with a mounting hole, in which the mounting column passes through the mounting hole.

In some embodiments of the present disclosure, the cover plate is bonded to the enclosing structure by a bonding member.

In some embodiments of the present disclosure, the enclosing structure is provided with an open end, such that the cleaning liquid flowing out from the overflow structure flows out through the open end of the enclosing structure.

In some embodiments of the present disclosure, the enclosing structure is made of sealing foam.

In some embodiments of the present disclosure, the cleaning device further includes a protective cover, the protective cover is disposed on the top of the water storage tank, and the enclosing structure is sandwiched between the protective cover and the water storage tank.

In some embodiments of the present disclosure, the water storage tank is provided with a limiting structure, and the limiting structure is configured to limit the position of the enclosing structure.

In some embodiments of the present disclosure, a side of the enclosing structure adjacent to the overflow structure is at least partially attached to the overflow structure, and a side of the enclosing structure away from the overflow structure is at least partially attached to the limiting structure.

In some embodiments of the present disclosure, the water storage tank is provided with a water trough, a first end of the water trough is configured to receive the cleaning liquid flowing out from the open end of the enclosing structure, and a second end of the water trough is configured to discharge the cleaning liquid.

In some embodiments of the present disclosure, a bevel is provided at the bottom of the water trough, and the bevel is inclined in a direction away from the opening of the enclosing structure, such that the cleaning liquid flowing out from the opening end of the enclosing structure is drained to the wet cleaning structure through the bevel.

In some embodiments of the present disclosure, the bevel is provided with reinforcing ribs.

In some embodiments of the present disclosure, the cleaning device further includes:
a main body, the water storage tank being disposed in the main body;
wherein the main body is provided with a communicating hole, and the cleaning liquid is discharged from the water trough through the communicating hole.

In some embodiments of the present disclosure, the enclosing structure is configured to guide the cleaning liquid flowing out of the overflow structure to the wet cleaning structure.

In some embodiments of the present disclosure, the water storage tank includes:
a bottom shell provided with an open end; and
an upper cover covering the open end of the bottom shell, the overflow structure and the enclosing structure being disposed on the upper cover.

The present disclosure provides a liquid storage tank for a cleaning device and a cleaning device.

According to one aspect of the present disclosure, a liquid storage tank for a cleaning device is provided, including:
a tank body configured to store cleaning liquid; and
a liquid discharging structure disposed in the tank body and including a liquid discharging bevel structure and a liquid discharging pipeline, a first end of the liquid discharging pipeline being connected to a bottom end of the liquid discharging bevel structure, and a second end of the liquid discharging pipeline being provided with a liquid discharging port;
wherein the cleaning liquid in the tank body is configured to be guided to the first end of the liquid discharging pipeline through the liquid discharging bevel structure, and discharged to the outside of the tank body through the liquid discharging port.

In some embodiments of the present disclosure, the height of the liquid discharging bevel structure away from the first end of the liquid discharging pipeline is h1, and the height of the liquid discharging bevel structure close to the second end of the liquid discharging pipeline is h2;
where h1 >h2.

In some embodiments of the present disclosure, h1-h2>1 mm.

In some embodiments of the present disclosure, a liquid discharging trough is further provided at the bottom end of the liquid discharging bevel structure, the first end of the liquid discharging pipeline is connected to the bottom end of the liquid discharging bevel structure through the liquid discharging trough, and the liquid discharging pipeline is communicated with the liquid discharging trough.

In some embodiments of the present disclosure, the height of bottom of the liquid discharging trough is lower than the height of the bottom end of the liquid discharging bevel structure, and the cleaning liquid in the tank body is configured to be guided into the liquid discharging trough through the liquid discharging bevel structure.

In some embodiments of the present disclosure, a liquid discharging joint is provided at the bottom of the liquid discharging trough and is connected to the first end of the liquid discharging pipeline, and the liquid discharging joint is provided with a communicating hole communicated with the inner cavity of the tank body.

In some embodiments of the present disclosure, the tank body includes:
a bottom shell provided with an open end; and
an upper cover covering the opening end of the bottom shell;
wherein the liquid discharging bevel structure is disposed on a bottom wall of the bottom shell, and the cleaning liquid in the tank body is configured to be discharged through the liquid discharging pipeline connected to the bottom wall of the bottom shell when the tank body is in an upright state.

In some embodiments of the present disclosure, the tank body includes:
a bottom shell provided with a water flow channel and an open end; and
an upper cover covering the opening end of the bottom shell;
wherein the liquid discharging bevel structure is disposed on an inner wall of the upper cover, the second end of the liquid discharging pipeline is communicated with the water flow channel, and the cleaning liquid in the tank body is configured to be discharged through the liquid discharging pipeline and the water flow channel when the tank body is in an inverted state.

In some embodiments of the present disclosure, the tank body includes:
a bottom shell provided with a water flow channel and an open end;
an upper cover covering the opening end of the bottom shell;
wherein a bottom wall of the bottom shell and an inner wall of the upper cover are both provided with the liquid discharging bevel structure, and the cleaning liquid in the tank body is configured to be discharged through the liquid discharging pipeline connected to the bottom wall of the bottom shell when the tank body is in an upright state, and the cleaning liquid in the tank body is configured to be discharged through the liquid discharging pipeline connected to the inner wall of the upper cover and through the water flow channel when the tank body is in an inverted state.

According to one aspect of the present disclosure, a cleaning device is provided, including the above-mentioned liquid storage tank for a cleaning device.

In some embodiments of the present disclosure, the cleaning device further includes:
a wet cleaning structure, the liquid storage tank being configured to supply cleaning liquid for the wet cleaning structure.

In some embodiments of the present disclosure, the cleaning device further includes:
a delivery pipeline, a first end of the delivery pipeline being communicated with the liquid discharging port of the liquid discharging pipeline, and a second end of the delivery pipeline being connected to the wet cleaning structure.

According to one aspect of the present disclosure, a cleaning device is provided, including:
a main body;
a liquid storage tank configured to store cleaning liquid and including a bottom shell, the bottom shell and the main body being of an integrally formed structure; and
a wet cleaning structure, the liquid storage tank being configured to supply cleaning liquid for the wet cleaning structure.

In some embodiments of the present disclosure, the bottom shell includes a shell and a protective structure, and the protective structure is disposed in the shell to prevent impact and deformation of the liquid storage tank.

In some embodiments of the present disclosure, the protective structure includes a rib plate, a bottom of the rib plate is connected to a bottom wall of the shell, and a side wall of the rib plate is connected to a side wall of the shell.

In some embodiments of the present disclosure, the rib plate and the shell are of an integrally formed structure.

In some embodiments of the present disclosure, the rib plate includes a first rib plate and a second rib plate, and the first rib plate and the second rib plate are bent toward sides opposite to each other.

In some embodiments of the present disclosure, the liquid storage tank further includes:
an upper cover, the bottom shell being provided with an opening end, and the upper cover covering the opening end of the bottom shell.

In some embodiments of the present disclosure, the bottom shell and the upper cover are connected by an ultrasonic process.

In some embodiments of the present disclosure, one of the upper cover and the bottom shell is provided with an ultrasonic protrusion, and the other of the upper cover and the bottom shell is provided with an ultrasonic surface, and the ultrasonic protrusion is connected to the ultrasonic surface such that the bottom shell and the upper cover are connected through an ultrasonic process.

In some embodiments of the present disclosure, the ultrasonic protrusion is of a closed ring structure, and the ultrasonic protrusion is disposed along the circumference of the upper cover; and
the ultrasonic surface is of a closed ring structure, and the ultrasonic surface is disposed along the circumference of the bottom shell.

In some embodiments of the present disclosure, a cross-section of the ultrasonic protrusion is at least one of a triangle, an arc, and a polygon.

In some embodiments of the present disclosure, the upper cover is made of a transparent material.

In some embodiments of the present disclosure, the cleaning device further includes:
a liquid inlet pipe disposed in the liquid storage tank, the liquid storage tank being provided with a water inlet, a first end of the liquid inlet pipe being communicated with the water inlet, and a second end of the liquid inlet pipe being configured to transport the cleaning liquid to an inner cavity of the liquid storage tank.

In some embodiments of the present disclosure, the cleaning device further includes:
a liquid outlet pipe assembly disposed in the liquid storage tank, a first end of the liquid outlet pipe assembly being communicated with the inner cavity of the liquid storage tank, and a second end of the liquid outlet pipe assembly being communicated with the wet cleaning structure and configured to transport the cleaning liquid from the liquid storage tank to the wet cleaning structure.

In some embodiments of the present disclosure, the liquid outlet pipe assembly includes:
a liquid outlet pipe disposed in the liquid storage tank and communicated with the inner cavity of the liquid storage tank; and
a liquid outlet joint, a first end of the liquid outlet joint being communicated with the liquid outlet pipe, and a second end of the liquid outlet joint being communicated with the wet cleaning structure.

In some embodiments of the present disclosure, the cleaning device further includes:
an overflow structure disposed on the liquid storage tank and configured to overflow the cleaning liquid in the liquid storage tank.

According to one aspect of the present disclosure, a cleaning device water tank is provided, including:
a tank body;
a liquid inlet pipe disposed on the tank body, a liquid inlet being disposed at a first end of the liquid inlet pipe;
a water inlet valve disposed inside a second end of the liquid inlet pipe, the liquid inlet pipe being capable of communicating with the inner cavity of the tank body through the water inlet valve; and
a supporting structure disposed in the liquid inlet pipe and configured to support one side of the water inlet valve.

In some embodiments of the present disclosure, the water inlet valve is a one-way valve, which allows the cleaning liquid flowing in from the liquid inlet to flow into the inner cavity of the tank body in a one-way manner.

In some embodiments of the present disclosure, the water inlet valve includes:
a central portion provided with a valve port, the valve port being configured to be selectively opened and closed;
a fixing portion disposed around the central portion; and
an annular portion disposed between the central portion and the fixing portion;
wherein the supporting structure supports a side of the annular portion facing the liquid inlet.

In some embodiments of the present disclosure, along a radial direction of the central portion, the inner wall of the liquid inlet pipe and the supporting structure are clamped on two sides of the fixing portion respectively.

In some embodiments of the present disclosure, a first flow channel is provided in the liquid inlet pipe, a first end of the first flow channel is provided with the liquid inlet, and a second end of the first flow channel is provided with the water inlet valve and the supporting structure;
wherein the supporting structure is disposed between the water inlet valve and the first flow channel.

In some embodiments of the present disclosure, the supporting structure is a support ring, and along an axial direction of the water inlet valve, a first end of the support ring is disposed opposite to the first flow channel and is communicated therewith, and a second end of the support ring abuts against the annular portion.

In some embodiments of the present disclosure, the supporting structure is at least one support protrusion, the support protrusion abuts against the annular portion, and two adjacent support protrusions are spaced apart at the second end of the first flow channel.

In some embodiments of the present disclosure, the cleaning device water tank further includes:
a fixing member, the liquid inlet pipe being communicated with the inner cavity of the tank body through a third flow channel in the fixing member;
wherein the fixing member is connected to the tank body and is disposed on a side of the water inlet valve away from the supporting structure, and is configured to fix the water inlet valve.

In some embodiments of the present disclosure, the cleaning device water tank further includes:
a liquid inlet pipe disposed in the tank body, a first end of the liquid inlet pipe being communicated with the third flow channel of the fixing member, and a second end of the liquid inlet pipe being communicated with the inner cavity of the tank body.

In some embodiments of the present disclosure, the tank body includes:
a bottom shell provided with an open end; and
an upper cover covering the opening end of the bottom shell;
wherein the second end of the liquid inlet pipe is connected to the upper cover.

In some embodiments of the present disclosure, the upper cover further includes:
a joint disposed on a side of the upper cover facing the bottom shell, the joint being communicated with the liquid inlet pipe and provided with a communication hole, and the communication hole being communicated with the inner cavity of the tank body.

According to one aspect of the present disclosure, an embodiment of the present disclosure provides a cleaning device, including the above-mentioned cleaning device water tank.

In some embodiments of the present disclosure, the tank body of the cleaning device water tank is provided with a water outlet hole, which is disposed corresponding to the liquid inlet and configured to discharge the cleaning liquid flowing out of the liquid inlet.

In some embodiments of the present disclosure, the cleaning device further includes:
a wet cleaning structure, the tank body being configured to provide water to the wet cleaning structure;
wherein the water outlet hole is located above the wet cleaning structure, such that the cleaning liquid flowing out of the liquid inlet flows to the wet cleaning structure through the water outlet hole.

According to one aspect of the present disclosure, a liquid level detection structure of a cleaning device water tank is provided, including:
a limiting structure disposed in the water tank;
a floating assembly disposed in the limiting structure and configured to move as a liquid level in the water tank changes, the limiting structure being configured to limit the floating assembly; and
a sensing member disposed on the water tank and configured to generate a sensing signal in response to the movement of the floating assembly;
wherein a reinforcement structure is disposed on a side of the limiting structure away from the floating assembly.

In some embodiments of the present disclosure, the limiting structure includes a plurality of enclosure members disposed around the floating assembly to limit the floating assembly, and at least one of the plurality of enclosure members is provided with the reinforcement structure.

In some embodiments of the present disclosure, the limiting structure further includes a portion of a water tank side wall, and the portion of the water tank side wall and the plurality of enclosure members are disposed around the floating assembly to limit the floating assembly.

In some embodiments of the present disclosure, a water inlet is provided between two adjacent enclosure members or between the enclosure member and the portion of the water tank side wall, a guide cavity is formed in the limiting structure, the floating assembly is provided in the guide cavity, and the guide cavity is communicated with the inner cavity of the water tank through the water inlet.

In some embodiments of the present disclosure, a recessed portion is formed on the portion of the water tank side wall, and the recessed portion and the plurality of enclosure members form the limiting structure.

In some embodiments of the present disclosure, the reinforcement structure includes at least one reinforcement rib.

In some embodiments of the present disclosure, the enclosure member includes:
a first enclosure member connected to a side wall of the water tank; and
a second enclosure member spaced apart from the side wall of the water tank;
wherein the number of the reinforcing ribs corresponding to the first enclosure member is less than the number of the reinforcing ribs corresponding to the second enclosure member.

In some embodiments of the present disclosure, the reinforcing ribs are disposed in parallel and at intervals or cross-disposed with each other.

In some embodiments of the present disclosure, the reinforcement structure is connected to a bottom surface of the water tank.

In some embodiments of the present disclosure, the limiting structure, the reinforcement structure and the water tank are of an integrally formed structure.

In some embodiments of the present disclosure, a guide structure is provided on a side of the limiting structure facing the floating assembly, and the guide structure is configured to guide the floating assembly.

According to one aspect of the present disclosure, an embodiment of the present disclosure also provides a cleaning device, including a water tank and the above-mentioned liquid level detection structure of a cleaning device water tank, in which the liquid level detection structure of a cleaning device water tank is disposed in the water tank for detecting the liquid level of the water tank.

Other embodiments of the present disclosure after considering the specification and practicing the inventions disclosed herein. The present disclosure is intended to cover any variations, uses, or adaptations of the present disclosure that follow the general principles of the present disclosure and include common knowledge or customary techniques in the art that are not disclosed in the present disclosure. The specification and example embodiments are intended to be exemplary only, and the true scope and spirit of the present disclosure are indicated by the appended claims.

It should be understood that the present disclosure is not limited to the precise structures that have been described above and shown in the drawings, and that various modifications and changes may be made without departing from its scope. The scope of protection of the present disclosure is limited only by the appended claims.

## Claims

1. A sensor mounting structure, comprising:
a main body (941); and
a fixing cover (942), an accommodation cavity (943) being provided between the fixing cover (942) and the main body (941) and configured to accommodate a sensor (94100);
wherein the fixing cover (942) has a first end and a second end opposite to each other, one of the first end and the main body (941) is provided with a fixing portion, and the other of the first end and the main body (941) is provided with a fixing matching portion matching with the fixing portion, and the second end of the fixing cover (942) is provided with a mounting portion (9423), and the mounting portion (9423) is connected to the main body (941) via a fastener.

2. The sensor mounting structure according to claim 1, wherein the fixing portion is an extension arm (9421), the fixing matching portion is an insertion hole (9411), and the extension arm (9421) is at least partially inserted into the insertion hole (9411).

3. The sensor mounting structure according to claim 2, wherein the extension arm (9421) is snap-connected to the insertion hole (9411).

4. The sensor mounting structure according to claim 2, wherein a limiting portion (9412) is provided on an outer side of the insertion hole (9411) and configured to limit the extension arm (9421) such that the extension arm (9421) is inserted into the insertion hole (9411).

5. The sensor mounting structure according to claim 4, wherein the limiting portion (9412) is of a U-shaped structure, and an open end of the U-shaped structure is disposed toward the extension arm (9421).

6. The sensor mounting structure according to claim 4, wherein the limiting portion (9412) is disposed on a bearing portion (9413), and the bearing portion (9413) is configured to bear at least a portion of the extension arm (9421).

7. The sensor mounting structure according to claim 6, wherein upper and lower sides of the extension arm (9421) respectively abut against an inner wall of the insertion hole (9411) and the bearing portion (9413); or
the upper side of the extension arm (9421) abuts against the inner wall of the insertion hole (9411).

8. The sensor mounting structure according to any one of claims 1 to 7, wherein one of the main body (941) and the fixing cover (942) is provided with a limiting protrusion (9414), and the other of the main body (941) and the fixing cover (942) is provided with a limiting groove (9422), and the limiting groove (9422) is configured to limit the limiting protrusion (9414).

9. The sensor mounting structure according to any one of claims 1 to 7, wherein the fastener is a bolt.

10. The sensor mounting structure according to any one of claims 1 to 7, wherein one of the mounting portion (9423) and the main body (941) is provided with a positioning column (9415), and the other of the mounting portion (9423) and the main body (941) is provided with a positioning hole (231), and the positioning column (9415) is inserted into the positioning hole (231).

11. A cleaning device, comprising a sensor (94100) and a sensor mounting structure according to any one of claims 1 to 10, the sensor mounting structure being configured to mount the sensor (94100).

12. The cleaning device according to claim 11, wherein the sensor (94100) is a carpet sensor.

13. The cleaning device according to claim 11, further comprising:
a water storage tank (901) configured to store cleaning liquid;
an overflow structure (902) disposed on the water storage tank (901);
an enclosing structure (903) at least partially disposed around the overflow structure (902) and configured to retain and guide the cleaning liquid flowing out of the overflow structure (902); and
a wet cleaning structure (904), the water storage tank (901) being configured to provide the cleaning liquid to the wet cleaning structure (904).

14. The cleaning device according to claim 11, comprising a liquid storage tank for the cleaning device, the liquid storage tank comprising:
a tank body (911) configured to store cleaning liquid;
a liquid discharging structure (912) disposed in the tank body (911) and comprising a liquid discharging bevel structure (9121) and a liquid discharging pipeline (9122), a first end of the liquid discharging pipeline (9122) being connected to a bottom end of the liquid discharging bevel structure (9121), and a second end of the liquid discharging pipeline (9122) being provided with a liquid discharging port;
wherein the cleaning liquid in the tank body (911) is configured to be guided to the first end of the liquid discharging pipeline (9122) through the liquid discharging bevel structure (9121), and discharged to outside of the tank body (911) through the liquid discharging port.

15. The cleaning device according to claim 11, comprising a cleaning device water tank, the cleaning device water tank comprising:
a tank body (921);
a liquid inlet pipe (922) disposed on the tank body (921), a liquid inlet (9221) being disposed at a first end of the liquid inlet pipe (922);
a water inlet valve (923) disposed inside a second end of the liquid inlet pipe (922), the liquid inlet pipe (922) being capable of communicating with an inner cavity of the tank body (921) through the water inlet valve (923); and
a supporting structure (924) disposed in the liquid inlet pipe (922) and configured to support one side of the water inlet valve (923).

16. The cleaning device according to claim 11, comprising a liquid level detection structure for the cleaning device water tank, the liquid level detection structure comprising:
a limiting structure (931) disposed in the water tank (93100);
a floating assembly (932) disposed in the limiting structure (931) and configured to move as a liquid level in the water tank (93100) changes, the limiting structure (931) being configured to limit the floating assembly (932); and
a sensing member (934) disposed on the water tank (93100) and configured to generate a sensing signal in response to the movement of the floating assembly (932);
wherein a reinforcement structure (933) is provided on a side of the limiting structure (931) away from the floating assembly (932).

17. The cleaning device according to claim 11, further comprising:
a main body (1);
a liquid storage tank (2) configured to store cleaning liquid and comprising a bottom shell (21), the bottom shell (21) and the main body (1) being of an integrally formed structure; and
a wet cleaning structure (4), the liquid storage tank (2) being configured to provide the cleaning liquid to the wet cleaning structure (4).
